# EUROPEAN PATENT APPLICATION

(11) **EP 4 397 352 A1**
(43) Date of publication of application: **10.07.2024**
(21) Application number: 22864185.8
(22) Date of filing: 05.08.2022
(51) Int. Cl.: A61M 25/092, A61B 1/01

(54) **MEDICAL DEVICE**

(30) Priority: 31.08.2021 JP 2021141376
(71) Applicant: CANON KABUSHIKI KAISHA, Tokyo 146-8501 (JP)
(72) Inventor: IWASAWA, Ryo, Tokyo 146-8501 (JP); NIIKAWA, Yusuke, Tokyo 146-8501 (JP); NOGUCHI, Tomio, Tokyo 146-8501 (JP)
(74) Representative: Canon Europe Limited
(86) International application number: PCT/JP2022/030177
(87) International publication number: WO 2023/032599

(57) **Abstract**

A medical apparatus includes a base unit including a drive source therein, and a bendable unit that is to be detachably attached to the base unit. The bendable unit includes a connection unit including a first member connected to the drive source and a second member connected to a linear member. In a case where pulling force at a first threshold value or less or compression force at a second threshold value or less acts between the first member and the second member, the connection unit maintains a state in which the first member and the second member are connected, and transmits drive force from the drive source to the linear member, and in a case where pulling force exceeding the first threshold value or compression force exceeding the second threshold value acts between the first member and the second member, the connection unit separates the first member and the second member from each other and cuts off transmission of the drive force.

## Description

### Technical Field

The present invention relates to a bendable medical apparatus.

### Background Art

A medical apparatus, such as an endoscope or a catheter, includes a bending portion at a part of an insertion portion to be inserted into a human body. The bending portion is configured to be bendable and deformable by a linear member connected to a drive source. Japanese Patent Application Laid-Open No. 2013-248116 discusses a configuration in which a drive wire connected to a drive source and a control wire connected to a bending portion of an insertion portion are connected via a breaker unit with cutoff strength lower than that of the control wire.

According to the document, in response to tensile force at a threshold value or more acting on the control wire, the breaker unit is cut off, which prevents a leading end of the insertion portion from strongly pressing an object.

### Citation List

### Patent Literature

PTL 1: Japanese Patent Laid-Open No. 2013-248116

In the configuration discussed in Japanese Patent Application Laid-Open No. 2013-248116, however, each time the connection of the breaker unit is cut off, a user is required to reach into the breaker unit and replace a member, such as a cable, that is included in the breaker unit.

The present invention is directed to bringing a medical apparatus into a usable state again by a simple method after connection between a drive source and a linear member has been cut off.

### Summary of Invention

According to an aspect of the present invention, a medical apparatus includes a base unit including a drive source therein, and a bendable unit that is to be detachably attached to the base unit and includes a bending portion and a bending drive unit configured to receive drive force from the drive source and bend the bending portion, the bending drive unit including a linear member connected to the bending portion, wherein the bendable unit includes a connection unit including a first member connected to the drive source, and a second member connected to the linear member, wherein, in a case where pulling force at a first threshold value or less or compression force at a second threshold value or less acts between the first member and the second member, the connection unit maintains a state in which the first member and the second member are connected, and transmits the drive force from the drive source to the linear member, and wherein, in a case where pulling force exceeding the first threshold value or compression force exceeding the second threshold value acts between the first member and the second member, the connection unit separates the first member and the second member from each other, and cuts off transmission of the drive force from the drive source to the linear member.

### Advantageous Effects of Invention

According to the present invention, a medical apparatus can be brought into a usable state again by a simple method after connection between a drive source and a linear member has been cut off.

### Brief Description of Drawings

[Fig. 1] Fig. 1 is an overall view of a medical system.
[Fig. 2] Fig. 2 is a perspective view illustrating a medical apparatus and a support base.
[Fig. 3A] Fig. 3A is an explanatory diagram illustrating a catheter.
[Fig. 3B] Fig. 3B is an explanatory diagram illustrating the catheter.
[Fig. 4A] Fig. 4Ais an explanatory diagram illustrating a catheter unit.
[Fig. 4B] Fig. 4B is an explanatory diagram illustrating the catheter unit.
[Fig. 5A] Fig. 5A is an explanatory diagram illustrating a base unit and a wire drive unit.
[Fig. 5B] Fig. 5B is an explanatory diagram illustrating the base unit and the wire drive unit.
[Fig. 5C] Fig. 5C is an explanatory diagram illustrating the base unit and the wire drive unit.
[Fig. 6A] Fig. 6A is an explanatory diagram illustrating the wire drive unit, a coupling device, and a bending drive unit.
[Fig. 6B] Fig. 6B is an explanatory diagram illustrating the wire drive unit, the coupling device, and the bending drive unit.
[Fig. 6C] Fig. 6C is an explanatory diagram illustrating the wire drive unit, the coupling device, and the bending drive unit.
[Fig. 7A] Fig. 7A is an explanatory diagram illustrating attachment of the catheter unit.
[Fig. 7B] Fig. 7B is an explanatory diagram illustrating attachment of the catheter unit.
[Fig. 8A] Fig. 8A is a diagram illustrating coupling between the catheter unit and the base unit.
[Fig. 8B] Fig. 8B is a diagram illustrating coupling between the catheter unit and the base unit.
[Fig. 9] Fig. 9 is an exploded view illustrating coupling between the catheter unit and the base unit.
[Fig. 10] Fig. 10 is a diagram illustrating fixing of a drive wire by using a coupling unit.
[Fig. 11] Fig. 11 is a diagram illustrating fixing of the drive wire by using the coupling unit.
[Fig. 12] Fig. 12 is a diagram illustrating fixing of the drive wire by using the coupling unit.
[Fig. 13] Fig. 13 is a diagram illustrating fixing of the drive wire by using the coupling unit.
[Fig. 14] Fig. 14 is a diagram illustrating fixing of the drive wire by using the coupling unit.
[Fig. 15A] Fig. 15A is an explanatory diagram illustrating the catheter unit and the base unit.
[Fig. 15B] Fig. 15B is an explanatory diagram illustrating the catheter unit and the base unit.
[Fig. 15C] Fig. 15C is an explanatory diagram illustrating the catheter unit and the base unit.
[Fig. 16A] Fig. 16A is a diagram illustrating an operation that is performed by an operation unit.
[Fig. 16B] Fig. 16B is a diagram illustrating an operation that is performed by the operation unit.
[Fig. 16C] Fig. 16C is a diagram illustrating an operation that is performed by the operation unit.
[Fig. 17A] Fig. 17A is a cross-sectional view illustrating an operation that is performed by the operation unit.
[Fig. 17B] Fig. 17B is a cross-sectional view illustrating an operation that is performed by the operation unit.
[Fig. 17C] Fig. 17C is a cross-sectional view illustrating an operation that is performed by the operation unit.
[Fig. 18A] Fig. 18A is a diagram illustrating a connection unit according to a first exemplary embodiment.
[Fig. 18B] Fig. 18B is a diagram illustrating the connection unit according to the first exemplary embodiment.
[Fig. 18C] Fig. 18C is a diagram illustrating the connection unit according to the first exemplary embodiment.
[Fig. 19A] Fig. 19A is a diagram illustrating an operation that is performed by the connection unit according to the first exemplary embodiment.
[Fig. 19B] Fig. 19B is a diagram illustrating an operation that is performed by the connection unit according to the first exemplary embodiment.
[Fig. 19C] Fig. 19C is a diagram illustrating an operation that is performed by the connection unit according to the first exemplary embodiment.
[Fig. 20A] Fig. 20A is a diagram illustrating an operation that is performed by the connection unit according to the first exemplary embodiment.
[Fig. 20B] Fig. 20B is a diagram illustrating an operation that is performed by the connection unit according to the first exemplary embodiment.
[Fig. 20C] Fig. 20C is a diagram illustrating an operation that is performed by the connection unit according to the first exemplary embodiment.
[Fig. 21A] Fig. 21A is a diagram illustrating a modified example of a connection unit according to a second exemplary embodiment.
[Fig. 21B] Fig. 21B is a diagram illustrating a modified example of a connection unit according to the second exemplary embodiment.
[Fig. 21C] Fig. 21C is a diagram illustrating a modified example of a connection unit according to the second exemplary embodiment.
[Fig. 21D] Fig. 21D is a diagram illustrating a modified example of a connection unit according to the second exemplary embodiment.
[Fig. 21E] Fig. 21E is a diagram illustrating a modified example of a connection unit according to the second exemplary embodiment.
[Fig. 21F] Fig. 21F is a diagram illustrating a modified example of a connection unit according to the second exemplary embodiment.
[Fig. 21G] Fig. 21G is a diagram illustrating a modified example of a connection unit according to the second exemplary embodiment.
[Fig. 22A] Fig. 22A is a diagram illustrating a connection unit according to the second exemplary embodiment.
[Fig. 22B] Fig. 22B is a diagram illustrating the connection unit according to the second exemplary embodiment.
[Fig. 22C] Fig. 22C is a diagram illustrating the connection unit according to the second exemplary embodiment.
[Fig. 23A] Fig. 23A is a diagram illustrating an operation that is performed by the connection unit according to the second exemplary embodiment.
[Fig. 23B] Fig. 23B is a diagram illustrating an operation that is performed by the connection unit according to the second exemplary embodiment.
[Fig. 23C] Fig. 23C is a diagram illustrating an operation that is performed by the connection unit according to the second exemplary embodiment.
[Fig. 24A] Fig. 24A is a diagram illustrating a connection unit according to a third exemplary embodiment.
[Fig. 24B] Fig. 24B is a diagram illustrating a connection unit according to the third exemplary embodiment.
[Fig. 24C] Fig. 24C is a diagram illustrating a connection unit according to the third exemplary embodiment.
[Fig. 25A] Fig. 25A is a diagram illustrating an operation that is performed by the connection unit according to the third exemplary embodiment.
[Fig. 25B] Fig. 25B is a diagram illustrating an operation that is performed by the connection unit according to the third exemplary embodiment.
[Fig. 25C] Fig. 25C is a diagram illustrating an operation that is performed by the connection unit according to the third exemplary embodiment.
[Fig. 26A] Fig. 26A is a diagram illustrating a connection unit according to a fourth exemplary embodiment.
[Fig. 26B] Fig. 26B is a diagram illustrating the connection unit according to the fourth exemplary embodiment.
[Fig. 26C] Fig. 26C is a diagram illustrating the connection unit according to the fourth exemplary embodiment.
[Fig. 27A] Fig. 27A is a diagram illustrating an operation that is performed by the connection unit according to the fourth exemplary embodiment.
[Fig. 27B] Fig. 27B is a diagram illustrating an operation that is performed by the connection unit according to the fourth exemplary embodiment.
[Fig. 27C] Fig. 27C is a diagram illustrating an operation that is performed by the connection unit according to the fourth exemplary embodiment.
[Fig. 28A] Fig. 28A is a diagram illustrating a connection unit according to a modified example.
[Fig. 28B] Fig. 28B is a diagram illustrating the connection unit according to the modified example.
[Fig. 28C] Fig. 28C is a diagram illustrating the connection unit according to the modified example.
[Fig. 29] Fig. 29 is a diagram illustrating the connection unit according to the modified example.
[Fig. 30A] Fig. 30A is a diagram illustrating an operation that is performed by a connection unit according to the modified example.
[Fig. 30B] Fig. 30B is a diagram illustrating an operation that is performed by a connection unit according to the modified example.
[Fig. 30C] Fig. 30C is a diagram illustrating an operation that is performed by a connection unit according to the modified example.
[Fig. 31A] Fig. 31A is a diagram illustrating a connection unit according to a fifth exemplary embodiment.
[Fig. 31B] Fig. 31B is a diagram illustrating the connection unit according to the fifth exemplary embodiment.
[Fig. 31C] Fig. 31C is a diagram illustrating the connection unit according to the fifth exemplary embodiment.
[Fig. 32A] Fig. 32A is a diagram illustrating an assembling method of the connection unit according to the fifth exemplary embodiment.
[Fig. 32B] Fig. 32B is a diagram illustrating the assembling method of the connection unit according to the fifth exemplary embodiment.
[Fig. 32C] Fig. 32C is a diagram illustrating the assembling method of the connection unit according to the fifth exemplary embodiment.
[Fig. 32D] Fig. 32D is a diagram illustrating the assembling method of the connection unit according to the fifth exemplary embodiment.
[Fig. 32E] Fig. 32E is a diagram illustrating the assembling method of the connection unit according to the fifth exemplary embodiment.
[Fig. 32F] Fig. 32F is a diagram illustrating the assembling method of the connection unit according to the fifth exemplary embodiment.
[Fig. 33] Fig. 33 is a diagram illustrating an operation that is performed by the connection unit according to the fifth exemplary embodiment.

### Description of Embodiments

Hereinafter, exemplary embodiments according to the present disclosure will be described with reference to the drawings.

### [First Exemplary Embodiment]

### <Medical System and Medical Apparatus>

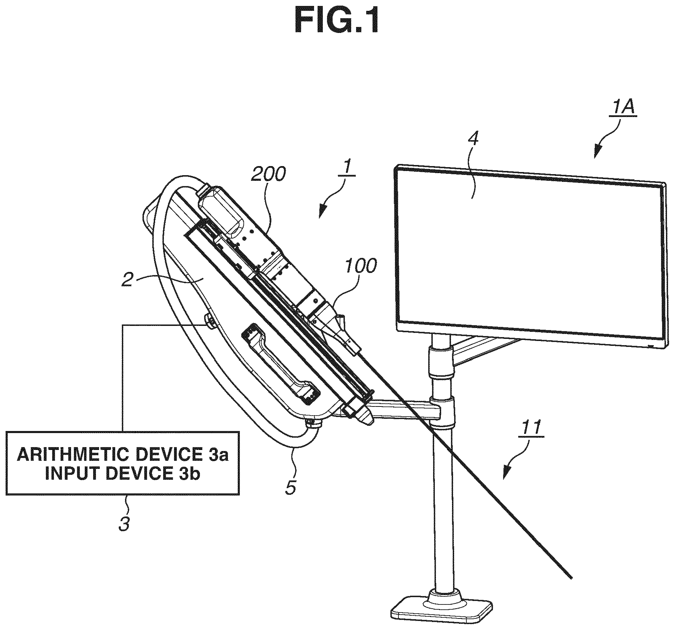

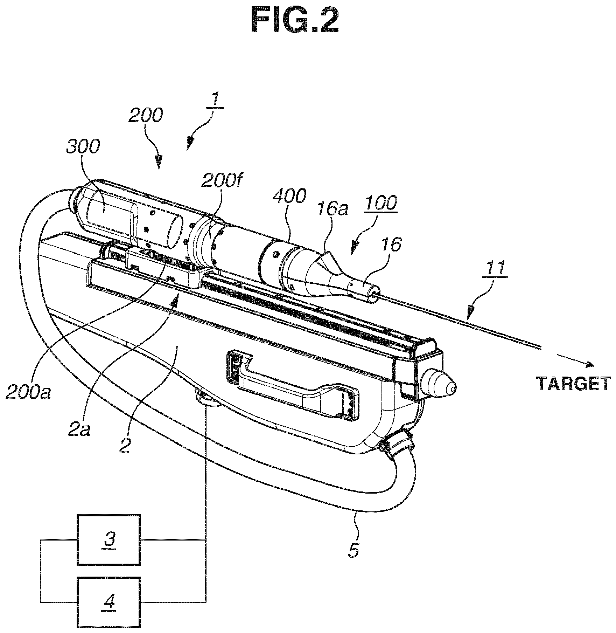

A medical system 1A and a medical apparatus 1 according to a first exemplary embodiment will be described with reference to Figs. 1 and 2. Fig. 1 is an overall view of the medical system 1A. Fig. 2 is a perspective view illustrating the medical apparatus 1 and a support base 2.

The medical system 1A includes the medical apparatus 1, the support base 2 for mounting the medical apparatus 1, and a control unit (control device) 3 that controls the medical apparatus 1. In the present exemplary embodiment, the medical system 1A includes a monitor 4 serving as a display device.

The medical apparatus 1 includes a catheter unit (bendable unit) 100 including a catheter 11 serving as a bendable member, and a base unit (drive unit, mounting unit) 200. The catheter unit 100 is configured to be detachably attached to the base unit 200.

In the present exemplary embodiment, a user of the medical system 1A and the medical apparatus 1 inserts the catheter 11 into the inside of a target to perform operations, such as observation of the inside of the target, collection of various samples from the inside of the target, and treatment on the inside of the target. As an exemplary embodiment, the user can insert the catheter 11 into the inside of a patient serving as a target. Specifically, the user inserts the catheter 11 into a bronchus via a mouth cavity or a nasal cavity of the patient to perform operations, such as observation, collection, and ablation of a lung tissue.

The catheter 11 can be used as a guide (sheath) that guides medical appliances for the above-described operations. Examples of the medical appliances (tools) include endoscopes, forceps, and ablation devices. In addition, the bendable member itself may have functions as the above-described medical appliances. In this case, the shape of the bendable member is not limited to a tubular shape, and the bendable member may have a columnar shape, for example.

In the present exemplary embodiment, the control unit 3 includes an arithmetic device 3a and an input device 3b. The input device 3b receives commands and inputs for operations of the catheter 11. The arithmetic device 3a includes a storage and a random access memory that store programs and various types of data for control of the catheter 11, and a central processing unit for executing the programs. The control unit 3 may also include an output unit that outputs a signal to display an image on the monitor 4.

As illustrated in Fig. 2, in the present exemplary embodiment, the medical apparatus 1 is electrically connected to the control unit 3 via a cable 5 that connects the base unit 200 of the medical apparatus 1 and the support base 2, and the support base 2. The medical apparatus 1 and the control unit 3 may be directly connected to each other via a cable. The medical apparatus 1 and the control unit 3 may be wirelessly connected to each other.

The medical apparatus 1 is detachably attached to the support base 2 via the base unit 200. More specifically, in the medical apparatus 1, a mounting unit (connection unit) 200a of the base unit 200 is detachably attached to a movement stage (reception portion) 2a of the support base 2. Even in a state in which the mounting unit 200a of the medical apparatus 1 is detached from the movement stage 2a, the connection between the medical apparatus 1 and the control unit 3 is maintained in such a manner that the medical apparatus 1 can be controlled by the control unit 3. In the present exemplary embodiment, even in a state in which the mounting unit 200a of the medical apparatus 1 is detached from the movement stage 2a, the medical apparatus 1 and the support base 2 are connected by the cable 5.

In a state in which the medical apparatus 1 is detached from the support base 2 (in a state in which the medical apparatus 1 is detached from the movement stage 2a), the user can manually move the medical apparatus 1 and insert the catheter 11 into the inside of the target.

In a state in which the catheter 11 is inserted into the target and the medical apparatus 1 is attached to the support base 2, the user can use the medical apparatus 1. Specifically, movement of the movement stage 2a in a state in which the medical apparatus 1 is attached to the movement stage 2 moves the medical apparatus 1. Then, an operation of moving the movement stage 2a in a direction in which the catheter 11 is inserted into the target, and an operation of moving the movement stage 2a in a direction in which the catheter 11 is withdrawn from the target are performed. The movement of the movement stage 2a is controlled by the control unit 3.

The mounting unit 200a of the base unit 200 includes a release switch (not illustrated) and a detachment switch (not illustrated). In a state in which the mounting unit 200a is attached to the movement stage 2a, the user can manually move the medical apparatus 1 in a guide direction of the movement stage 2a while continuously pressing the release switch. In other words, the movement stage 2a includes a guide configuration for guiding the movement of the medical apparatus 1. In response to the user releasing the release switch, the medical apparatus 1 is fixed to the movement stage 2a. On the other hand, in response to the user pressing the detachment switch in a state in which the mounting unit 200a is attached to the movement stage 2a, the medical apparatus 1 is detached from the movement stage 2a.

One switch may have the function of the release switch and the function of the detachment switch. In a case of the release switch having a mechanism for switching between a pressed state and a non-pressed state of the release switch, the user is freed from continuously pressing the release switch during a manual slide movement of the medical apparatus 1.

In a state in which the mounting unit 200a is attached to the movement stage 2a and the release switch and the detachment switch are not pressed, the medical apparatus 1 is fixed to the movement stage 2a and moved by the movement stage 2a that is driven by a motor (not illustrated).

The medical apparatus 1 includes a wire drive unit (linear member drive unit, line drive unit, main body drive unit) 300 for driving the catheter 11. In the present exemplary embodiment, the medical apparatus 1 is a robot catheter apparatus that drives the catheter 11 with the wire drive unit 300 controlled by the control unit 3.

The control unit 3 can control the wire drive unit 300 and perform an operation of bending the catheter 11. In the present exemplary embodiment, the wire drive unit 300 is incorporated into the base unit 200. More specifically, the base unit 200 includes a base casing 200f accommodating the wire drive unit 300. That is, the base unit 200 includes the wire drive unit 300. The wire drive unit 300 and the base unit 200 can be collectively referred to as a catheter drive apparatus (base apparatus, main body).

In a length direction of the catheter 11, an end portion at which a leading end of the catheter 11 to be inserted into a target is disposed will be referred to as a distal end. In the extending direction of the catheter 11, an opposite side of the distal end will be referred to as a proximal end.

The catheter unit 100 includes a proximal end cover 16 that covers the proximal end side of the catheter 11. The proximal end cover 16 includes a tool hole 16a. A medical appliance can be inserted into the catheter 11 via the tool hole 16a.

As described above, in the present exemplary embodiment, the catheter 11 has a function as a guide device for guiding the medical appliance to a desired position in a target.

For example, in a state in which an endoscope is inserted into the catheter 11, the catheter 11 is inserted up to a target position in a target. In this process, at least any one of a manual operation of the user, the movement of the movement stage 2a, and the driving of the catheter 11 using the wire drive unit 300 is used. After the catheter 11 has reached the target position, the endoscope is withdrawn from the catheter 11 via the tool hole 16a. Then, a medical appliance is inserted from the tool hole 16a, and operations, such as the collection of various samples from the inside of the target, and the treatment on the inside of the target are performed.

As described below, the catheter unit 100 is detachably attached to the catheter drive apparatus (base apparatus, main body). More specifically, the catheter unit 100 is detachably attached to the base unit 200. After using the medical apparatus 1, the user detaches the catheter unit 100 from the base unit 200 and attaches a new catheter unit 100 to the base unit 200, to use the medical apparatus 1 again. That is, the catheter unit 100 can be used as a disposable unit. The disposable means that the catheter unit 100 once used in a treatment is to be discarded after the use. With this configuration, reuse of the catheter unit 100 is prevented and the medical apparatus 1 can be always kept in a clean state.

As illustrated in Fig. 2, the medical apparatus 1 includes an operation unit 400. In the present exemplary embodiment, the operation unit 400 is included in the catheter unit 100. The operation unit 400 is operated by the user when the catheter unit 100 is fixed to the base unit 200 and the catheter unit 100 is detached from the base unit 200.

Connecting an endoscope to be inserted into the catheter 11 to the monitor 4enables an image captured by the endoscope to be displayed on the monitor 4. Connecting the monitor 4 to the control unit 3 enables the state of the medical apparatus 1 and information regarding control of the medical apparatus 1 to be displayed on the monitor 4. For example, the position of the catheter 11 in a target and information regarding the navigation of the catheter 11 in the target can be displayed on the monitor 4. The monitor 4, the control unit 3, and the endoscope may be connected via a cable or may be wirelessly connected. Alternatively, the monitor 4 and the control unit 3 may be connected via the support base 2.

### <Catheter>

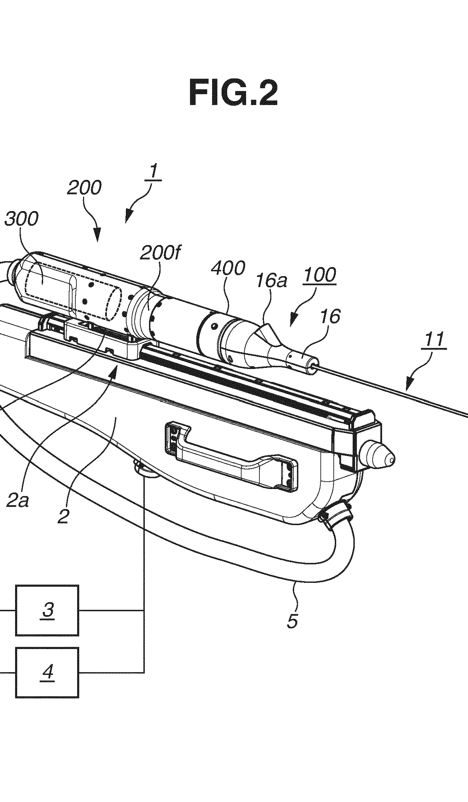

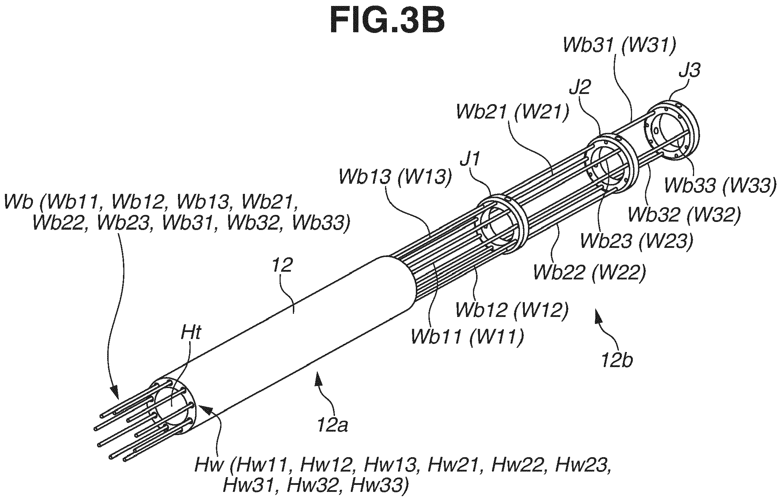

The catheter 11 serving as a bendable member will be described with reference to Figs. 3A and 3B. Figs. 3A and 3B are explanatory diagrams illustrating the catheter 11. Fig. 3A is a diagram illustrating the overall view of the catheter 11. Fig. 3B is an enlarged view of the catheter 11.

The catheter 11 includes a bending portion (bending member, catheter main body) 12 and a bending drive unit (catheter drive unit) 13 configured to bend the bending portion 12. The bending drive unit 13 is configured to bend the bending portion 12 with drive force from the wire drive unit 300 via a coupling device 21 to be described below.

The catheter 11 has a length in an insertion direction of the catheter 11 with respect to a target. The length direction (lengthwise direction) of the catheter 11 is the same as a length direction (lengthwise direction) of the bending portion 12, and a length direction (lengthwise direction) of first to ninth drive wires (W11 to W33) to be described below.

The bending drive unit 13 includes a plurality of drive wires (drive lines, linear members, linear actuators) connected to the bending portion 12. Specifically, the bending drive unit 13 includes the first drive wire W11, the second drive wire W12, the third drive wire W13, the fourth drive wire W21, the fifth drive wire W22, the sixth drive wire W23, the seventh drive wire W31, the eighth drive wire W32, and the ninth drive wire W33.

The first to ninth drive wires (W11 to W33) each include a held portion (held shaft, rod) Wa. Specifically, the first drive wire W11 includes a first held portion Wa11. The second drive wire W12 includes a second held portion Wa12. The third drive wire W13 includes a third held portion Wa13. The fourth drive wire W21 includes a fourth held portion Wa21. The fifth drive wire W22 includes a fifth held portion Wa22. The sixth drive wire W23 includes a sixth held portion Wa23. The seventh drive wire W31 includes a seventh held portion Wa31. The eighth drive wire W32 includes an eighth held portion Wa32. The ninth drive wire W33 includes a ninth held portion Wa33.

In the present exemplary embodiment, the first to ninth held portions (Wa11 to Wa33) have the same shape.

The first to ninth drive wires (W11 to W33) each include a wire member (wire member, line member, linear member) Wb having flexibility. The wire member Wb refers to a member that enables an object connected via the wire member Wb to be pushed or pulled, and has a certain level of rigidity. At the same time, the wire member Wb is a member deformable from a linear shape, which causes the bending portion 12 to be bent. The first drive wire W11 includes a first wire member Wb11. The second drive wire W12 includes a second wire member Wb12. The third drive wire W13 includes a third wire member Wb13. The fourth drive wire W21 includes a fourth wire member Wb21. The fifth drive wire W22 includes a fifth wire member Wb22. The sixth drive wire W23 includes a sixth wire member Wb23. The seventh drive wire W31 includes a seventh wire member Wb31. The eighth drive wire W32 includes an eighth wire member Wb32. The ninth drive wire W33 includes a ninth wire member Wb33.

Furthermore, in the present exemplary embodiment, a connection unit Wc that transmits drive force from a drive source to a wire member and cuts off transmission of drive force from the drive source to the linear member in a case where load at a threshold value or more acts is disposed for each set of a drive source M and a drive wire W. Specifically, the first wire member Wb11 is connected to a first drive source M11 via a first connection unit Wc11. The second wire member Wb12 is connected to a second drive source M12 via a second connection unit Wc12. The third wire member Wb13 is connected to a third drive source M13 via a third connection unit Wc13. The fourth wire member Wb21 is connected to a fourth drive source M21 via a fourth connection unit Wc21. The fifth wire member Wb22 is connected to a fifth drive source M22 via a fifth connection unit Wc22. The sixth wire member Wb23 is connected to a sixth drive source M23 via a sixth connection unit Wc23. The seventh wire member Wb31 is connected to a seventh drive source M31 via a seventh connection unit Wc31. The eighth wire member Wb32 is connected to an eighth drive source M32 via an eighth connection unit Wc11. The ninth wire member Wb33 is connected to a ninth drive source M33 via a ninth connection unit W c 11.

Here, in each of the following exemplary embodiments, a threshold value is not limited to a preset fixed value (constant value). While a threshold value hardly changes depending on an apparatus specification or a manufacturing condition in some cases, a threshold value dynamically changes in other cases. That is, although a target value set in designing a product exists, a threshold value at which the connection between the drive source M and the wire member Wb is actually cut off can vary to a certain degree for each apparatus due to the influence of a tolerance variation or the like. Because these threshold values sometimes vary to a certain degree due to the influence of a surrounding environment (temperature, humidity, etc.), the threshold values can vary depending on an operation timing even on the same apparatus. Thus, the medical apparatus 1 described in each of the above-described exemplary embodiments is only required to be designed or manufactured in such a manner that threshold values fall within predefined ranges in view of these influences, and threshold values include dynamically changing threshold values. The threshold values may be rephrased as limit values.

The connection unit Wc functions as a breakaway mechanism or a separation mechanism that cuts off connection (coupling, drive transmission) between a drive source and a wire member in a case where load exceeding a predetermined threshold value acts on a drive wire due to malfunction of the drive source or external force on the catheter 11. The detailed configuration and function of the connection unit Wc will be described below.

In the present exemplary embodiment, the first to third wire members (Wb11 to Wb13) have the same shape. The fourth to sixth wire members (Wb21 to Wb23) have the same shape. The seventh to ninth wire members (Wb31 to Wb33) have the same shape. In the present exemplary embodiment, the first to ninth wire members (Wb11 to Wb33) have the same shape except for their lengths.

The first to ninth held portions (Wa11 to Wa33) are attached to proximal ends of the first to ninth wire members (Wb11 to Wb33) via the first to ninth connection units (Wc11 to Wc33). The first to ninth drive wires (W11 to W33) are inserted into and fixed to the bending portion 12 via a wire guide 17.

In the present exemplary embodiment, the material of each of the first to ninth wire members (Wb11 to Wb33) is metal. The material of each of the first to ninth wire members (Wb11 to Wb33) may be resin. The material of each of the first to ninth wire members (Wb11 to Wb33) may contain metal and resin.

An arbitrary one of the first to ninth drive wires (W11 to W33) can be called as the drive wire W. In the present exemplary embodiment, the first to ninth drive wires (W11 to W33) have the same shape except for the lengths of the first to ninth wire members (Wb11 to Wb33).

In the present exemplary embodiment, the bending portion 12 is a tubular member that has flexibility and includes a pathway Ht for inserting a medical appliance.

A plurality of wire holes each for letting a different one of the first to ninth drive wires (W 11 to W33) through are formed in the wall of the bending portion 12. Specifically, a first wire hole Hw11, a second wire hole Hw12, and a third wire hole Hw13 are formed in the wall of the bending portion 12. Furthermore, a fourth wire hole Hw21, a fifth wire hole Hw22, and a sixth wire hole Hw23 are formed in the wall of the bending portion 12. Furthermore, a seventh wire hole Hw31, an eighth wire hole Hw32, and a ninth wire hole Hw33 are formed in the wall of the bending portion 12. The first to ninth wire holes Hw (Hw11 to Hw33) respectively correspond to the first to ninth drive wires (W11 to W33). Numbers postfixed to the reference sign Hw indicate the numbers of corresponding drive wires. For example, the first drive wire W11 is inserted into the first wire hole Hw11.

An arbitrary one of the first to ninth wire holes (Hw11 to Hw33) can be called as the wire hole Hw. In the present exemplary embodiment, the first to ninth wire holes (Hw11 to Hw33) have the same shape.

The bending portion 12 includes an intermediate region 12a and a bending region 12b. The bending region 12b is disposed at the distal end of the bending portion 12. The bending region 12b includes a first guide ring J1, a second guide ring J2, and a third guide ring J3. The bending region 12b refers to a region where the first guide ring J1, the second guide ring J2, and the third guide ring J3 are moved by the bending drive unit 13 so that the extent and the direction of bending of the bending portion 12 can be controlled. In Fig. 3B, the illustration of a part of the bending portion 12 covering the first to third guide rings (J1 to J3) is omitted.

In the present exemplary embodiment, the bending portion 12 includes a plurality of auxiliary rings (not illustrated). In the bending region 12b, the first guide ring J1, the second guide ring J2, and the third guide ring J3 are fixed to the wall surface of the bending portion 12. In the present exemplary embodiment, the plurality of auxiliary rings is arranged between the first guide ring J1 and the second guide ring J2, and between the second guide ring J2 and the third guide ring J3.

A medical appliance is guided up to the leading end of the catheter 11 by the pathway Ht, the first to third guide rings (J1 to J3), and the plurality of auxiliary rings.

The first to ninth drive wires (W11 to W33) are fixed to the first to third guide rings (J1 to J3) via the intermediate region 12a. Specifically, the first drive wire W11, the second drive wire W12, and the third drive wire W13 are fixed to the first guide ring J1. The fourth drive wire W21, the fifth drive wire W22, and the sixth drive wire W23 pass through the first guide ring J1 and the plurality of auxiliary rings, and are fixed to the second guide ring J2. The seventh drive wire W31, the eighth drive wire W32, and the ninth drive wire W33 pass through the first guide ring J1, the second guide ring J2, and the plurality of auxiliary rings, and are fixed to the third guide ring J3.

By driving the bending drive unit 13 using the wire drive unit 300, the medical apparatus 1 can bend the bending portion 12 toward a direction intersecting with the length direction of the catheter 11. Specifically, by moving the first to ninth drive wires (W11 to W33) in a length direction of the bending portion 12, the bending region 12b of the bending portion 12 can be bent via the first to third guide rings (J1 to J3) in a direction intersecting with the length direction.

The user utilizes at least any one of the movements of the medical apparatus 1 that is caused manually or using the movement stage 2a, and the bending of the bending portion 12, to insert the catheter 11 to a targeted portion in a target.

In the present exemplary embodiment, the first to ninth drive wires (W11 to W33) are used to move the first to third guide rings (J1 to J3) to bend the bending portion 12, but the configuration is not limited to this configuration. Any one or two of the first to third guide rings (J1 to J3) and drive wires fixed thereto may be omitted.

For example, the catheter 11 may have a configuration in which the seventh to ninth drives wires (W31 to W33) and the third guide ring J3 are disposed, and the first to sixth drive wires (W11 to W23) and the first and second guide rings (J1 and J2) are omitted. Alternatively, the catheter 11 may have a configuration in which the fourth to ninth drive wires (W21 to W33) and the second and third guide rings (J2 and J3) are disposed, and the first to third drive wires (W11 to W13) and the first guide ring J1 are omitted.

In addition, the catheter 11 may have a configuration in which one guide ring is driven using two drive wires. Also in this case, the number of guide rings may be one, or may be larger than one.

### <Catheter Unit>

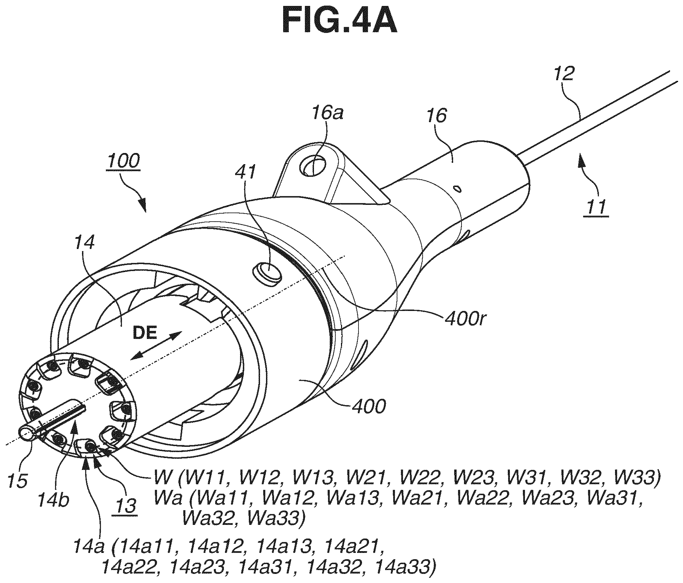

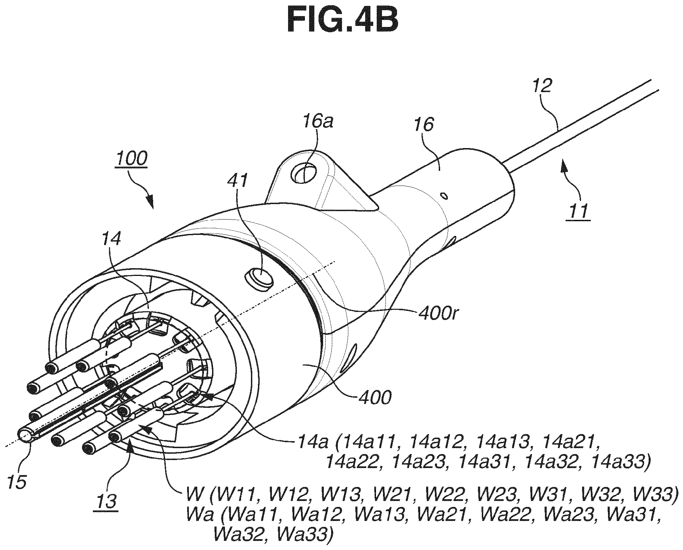

The catheter unit 100 will be described with reference to Figs. 4A and 4B. Figs. 4A and 4B are explanatory diagrams illustrating the catheter unit 100. Fig. 4A is an explanatory diagram illustrating the catheter unit 100 in a state in which a wire cover 14 to be described below is at a cover position. Fig. 4B is an explanatory diagram illustrating the catheter unit 100 in a state in which the wire cover 14 to be described below is at an exposure position.

The catheter unit 100 includes the catheter 11 including the bending portion 12 and the bending drive unit 13, and the proximal end cover 16 supporting the proximal end of the catheter 11. The catheter unit 100 includes the cover (wire cover) 14 for covering and protecting the first to ninth drive wires (W11 to W33) serving as a plurality of drive wires.

The catheter unit 100 is attachable and removable with respect to the base unit 200 in an attaching/detaching direction DE. An attaching direction of the catheter unit 100 with respect to the base unit 200, and a detaching direction of the catheter unit 100 from the base unit 200 are parallel to the attaching/detaching direction DE.

The proximal end cover (frame member, bending portion casing, catheter casing) 16 is a cover that covers a part of the catheter 11. The proximal end cover 16 includes the tool hole 16a for inserting a medical appliance into the pathway Ht of the bending portion 12.

The wire cover 14 has a plurality of exposure holes (wire cover holes, cover holes) each for letting a different one of the first to ninth drive wires (W11 to W33) pass through. The wire cover 14 includes a first exposure hole 14a11, a second exposure hole 14a12, a third exposure hole 14a13, a fourth exposure hole 14a21, a fifth exposure hole 14a22, a sixth exposure hole 14a23, a seventh exposure hole 14a31, an eighth exposure hole 14a32, and a ninth exposure hole 14a33. The first to ninth exposure holes (14a11 to 14a33) respectively correspond to the first to ninth drive wires (W11 to W33). Numbers postfixed to the reference numeral 14a indicate the numbers of corresponding drive wires. For example, the first drive wire W11 is inserted into the first exposure hole 14a11.

An arbitrary one of the first to ninth exposure holes (14a11 to 14a33) can be called as the exposure hole 14a. In the present exemplary embodiment, the first to ninth exposure holes (14a11 to 14a33) have the same shape.

The wire cover 14 can be moved to the cover position (refer to Fig. 4A) at which the wire cover 14 covers the first to ninth drive wires (W11 to W33), and a cover retracted position (refer to Fig. 4B) at which the wire cover 14 is retracted from the cover position. The cover retracted position can also be called the exposure position at which the first to ninth drive wires (W11 to W33) are exposed.

Before the catheter unit 100 is attached to the base unit 200, the wire cover 14 is at the cover position. In response to the catheter unit 100 being attached to the base unit 200, the wire cover 14 moves from the cover position to the exposure position in the attaching/detaching direction DE.

In the present exemplary embodiment, after movement of the wire cover 14 from the cover position to the exposure position, the wire cover 14 is kept at the exposure position. Thus, after attachment of the catheter unit 100 to the base unit 200, the wire cover 14 is kept at the exposure position even in a case where the catheter unit 100 is detached from the base unit 200.

Alternatively, the wire cover 14 may be configured to return to the cover position after moving from the cover position to the exposure position. For example, the catheter unit 100 may include an urging member that urges the wire cover 14 from the exposure position toward the cover position. In this case, in a case where the catheter unit 100 is detached from the base unit 200 after attachment of the catheter unit 100 to the base unit 200, the wire cover 14 is moved from the exposure position to the cover position.

When the wire cover 14 is at the exposure position, the first to ninth held portions (Wa11 to Wa33) of the first to ninth drive wires (W11 to W33) are exposed. Consequently, coupling between the bending drive unit 13 and the coupling device 21 to be described below is enabled. When the wire cover 14 is at the exposure position, the first to ninth held portions (Wa11 to Wa33) of the first to ninth drive wires (W11 to W33) protrude from the first to ninth exposure holes (14a11 to 14a33). More specifically, the first to ninth held portions (Wa11 to Wa33) protrude from the first to ninth exposure holes (14a11 to 14a33) toward an attaching direction Da to be described below.

As illustrated in Fig. 4B, the first to ninth drive wires (W11 to W33) are arranged along a circle (virtual circle) having a predetermined radius.

In the present exemplary embodiment, the catheter unit 100 includes a key shaft (key, catheter side key) 15. In the present exemplary embodiment, the key shaft 15 has a length in the attaching/detaching direction DE. The wire cover 14 has a shaft hole 14b through which the key shaft 15 passes. The key shaft 15 can be engage with a key acceptance portion 22 to be described below. Engagement of the key shaft 15 with the key acceptance portion 22 restricts, in a circumferential direction of the circle (virtual circle) on which the first to ninth drive wires (W11 to W33) are arranged, movement of the catheter unit 100 with respect to the base unit 200 within a predetermined range.

In the present exemplary embodiment, when viewed in the attaching/detaching direction DE, the first to ninth drive wires (W11 to W33) are arranged at positions centered on the key shaft 15 in such a manner as to surround the key shaft 15. In other words, the key shaft 15 is disposed on the inside of the circle (virtual circle) on which the first to ninth drive wires (W11 to W33) are arranged. With this configuration, the key shaft 15 and the first to ninth drive wires (W11 to W33) can be disposed in a saved space.

In the present exemplary embodiment, the catheter unit 100 includes the operation unit 400. The operation unit 400 is configured to be movable (rotatable) with respect to the proximal end cover 16 and the bending drive unit 13. In the operation unit 400, the operation unit 400 can rotate around a rotational axis 400r. The rotational axis 400r of the operation unit 400 extends in the attaching/detaching direction DE.

The operation unit 400 is configured to be movable (rotatable) with respect to the base unit 200 in a state in which the catheter unit 100 is attached to the base unit 200. More specifically, the operation unit 400 is configured to be movable (rotatable) with respect to the base casing 200f, the wire drive unit 300, and the coupling device 21 to be described below.

### <Base Unit>

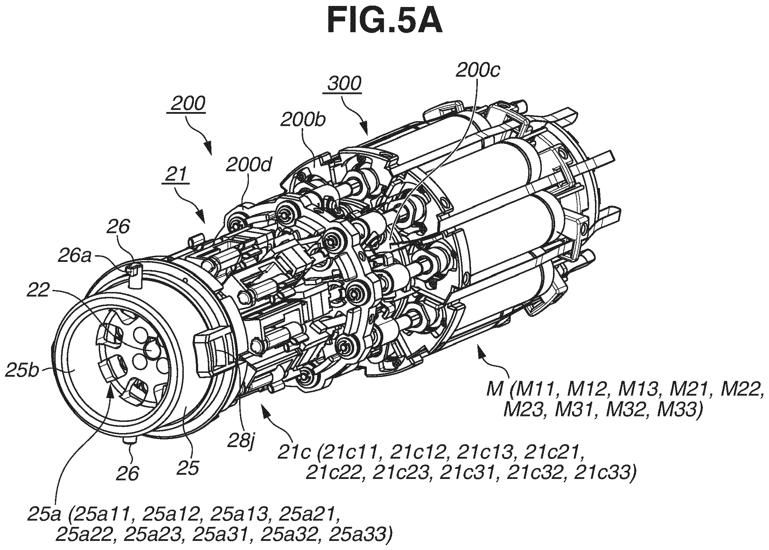

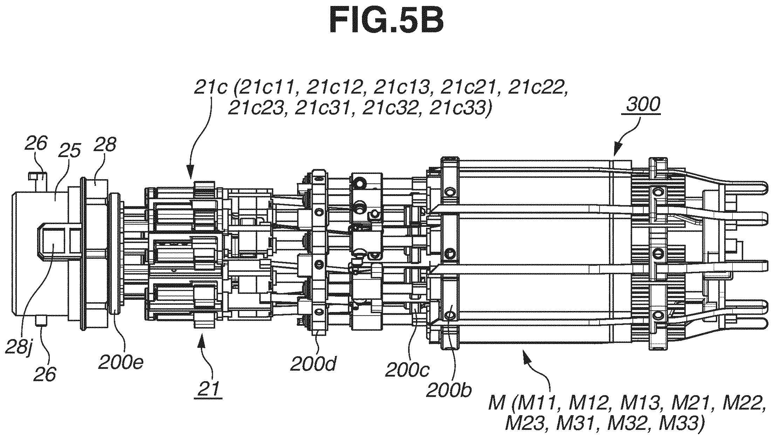

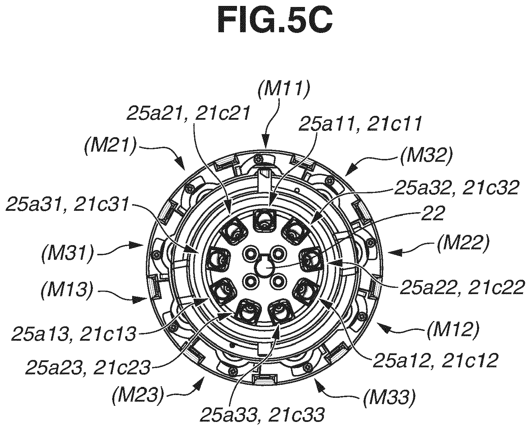

The base unit 200 and the wire drive unit 300 will be described with reference to Figs. 5A, 5B, and 5C. Figs. 5A, 5B, and 5C are explanatory diagrams illustrating the base unit 200 and the wire drive unit 300. Fig. 5A is a perspective view illustrating an internal structure of the base unit 200. Fig. 5B is a side view illustrating the internal structure of the base unit 200. Fig. 5C is a diagram illustrating the base unit 200 viewed in the attaching/detaching direction DE.

As described above, the medical apparatus 1 includes the base unit 200 and the wire drive unit 300. In the present exemplary embodiment, the wire drive unit 300 is accommodated in the base casing 200f and included inside the base unit 200. In other words, the base unit 200 includes the wire drive unit 300.

The wire drive unit 300 includes a plurality of drive sources (motors, actuators). In the present exemplary embodiment, the wire drive unit 300 includes the first drive source M11, the second drive source M12, the third drive source M13, the fourth drive source M21, the fifth drive source M22, the sixth drive source M23, the seventh drive source M31, the eighth drive source M32, and the ninth drive source M33.

An arbitrary one of the first to ninth drive sources (M11 to M33) can be called as the drive source M. In the present exemplary embodiment, the first to ninth drive sources (M11 to M33) have the same configuration.

The base unit 200 includes the coupling device 21. The coupling device 21 is accommodated in the base casing 200f. The coupling device 21 is connected to the wire drive unit 300. The coupling device 21 includes a plurality of coupling units. In the present exemplary embodiment, the coupling device 21 includes a first coupling unit 21c11, a second coupling unit 21c12, a third coupling unit 21c13, a fourth coupling unit 21c21, a fifth coupling unit 21c22, a sixth coupling unit 21c23, a seventh coupling unit 21c31, an eighth coupling unit 21c32, and a ninth coupling unit 21c33.

An arbitrary one of the first to ninth coupling units (21c11 to 21c33) can be called as the coupling unit 21c. In the present exemplary embodiment, the first to ninth coupling units (21c11 to 21c33) have the same configuration.

The plurality of coupling units is respectively connected to the plurality of drive sources and driven by the plurality of drive sources. Specifically, the first coupling unit 21c11 is connected to the first drive source M11 and driven by the first drive source M11. The second coupling unit 21c12 is connected to the second drive source M12 and driven by the second drive source M12. The third coupling unit 21c13 is connected to the third drive source M13 and driven by the third drive source M13. The fourth coupling unit 21c21 is connected to the fourth drive source M21 and driven by the fourth drive source M21. The fifth coupling unit 21c22 is connected to the fifth drive source M22 and driven by the fifth drive source M22. The sixth coupling unit 21c23 is connected to the sixth drive source M23 and driven by the sixth drive source M23. The seventh coupling unit 21c31 is connected to the seventh drive source M31 and driven by the seventh drive source M31. The eighth coupling unit 21c32 is connected to the eighth drive source M32 and driven by the eighth drive source M32. The ninth coupling unit 21c33 is connected to the ninth drive source M33 and driven by the ninth drive source M33.

As described below, the bending drive unit 13 including the first to ninth drive wires (W11 to W33) is coupled to the coupling device 21. The bending drive unit 13 receives drive force of the wire drive unit 300 via the coupling device 21 and bends the bending portion 12.

The drive wire W is coupled to the coupling unit 21c via the held portion Wa. The plurality of drive wires is respectively coupled to the plurality of coupling units. Specifically, the first held portion Wa11 of the first drive wire W 11 is coupled to the first coupling unit 21c11. The second held portion Wa12 of the second drive wire W12 is coupled to the second coupling unit 21c12. The third held portion Wa13 of the third drive wire W13 is coupled to the third coupling unit 21c13. The fourth held portion Wa21 of the fourth drive wire W21 is coupled to the fourth coupling unit 21c21. The fifth held portion Wa22 of the fifth drive wire W22 is coupled to the fifth coupling unit 21c22. The sixth held portion Wa23 of the sixth drive wire W23 is coupled to the sixth coupling unit 21c23. The seventh held portion Wa31 of the seventh drive wire W31 is coupled to the seventh coupling unit 21c31. The eighth held portion Wa32 of the eighth drive wire W32 is coupled to the eighth coupling unit 21c32. The ninth held portion Wa33 of the ninth drive wire W33 is coupled to the ninth coupling unit 21c33.

The base unit 200 includes a base frame 25. The base frame 25 has a plurality of insertion holes for respectively letting the first to ninth drive wires (W11 to W33) pass through. The base frame 25 includes a first insertion hole 25a11, a second insertion hole 25a12, a third insertion hole 25a13, a fourth insertion hole 25a21, a fifth insertion hole 25a22, a sixth insertion hole 25a23, a seventh insertion hole 25a31, an eighth insertion hole 25a32, and a ninth insertion hole 25a33. The first to ninth insertion holes (25a11 to 25a33) respectively correspond to the first to ninth drive wires (W11 to W33). Numbers postfixed to the reference numeral 25a indicate the numbers of corresponding drive wires. For example, the first drive wire W11 is inserted into the first insertion hole 25a11.

An arbitrary one of the first to ninth insertion holes (25a11 to 25a33) can be called as the insertion hole 25a. In the present exemplary embodiment, the first to ninth insertion holes (25a11 to 25a33) have the same shape.

The base frame 25 includes an attachment opening 25b into which the wire cover 14 is to be inserted. The first to ninth insertion holes (25a11 to 25a33) are formed on a bottom portion of the attachment opening 25b.

Furthermore, the base unit 200 includes a motor frame 200b, a first bearing frame 200c, a second bearing frame 200d, and a third bearing frame 200e. The motor frame 200b, the first bearing frame 200c, the second bearing frame 200d, and the third bearing frame 200e are coupled to each other.

The base frame 25 includes the key acceptance portion (key hole, base side key, main body side key) 22 that accepts the key shaft 15. Engagement of the key shaft 15 with the key acceptance portion 22 prevents the catheter unit 100 from being attached to the base unit 200 at a wrong phase.

Engagement of the key shaft 15 with the key acceptance portion 22 restricts, in the circumferential direction of the circle (virtual circle) on which the first to ninth drive wires (W11 to W33) are arranged, the movement of the catheter unit 100 with respect to the base unit 200 within a predetermined range.

Consequently, the first to ninth drive wires (W11 to W33) engage with the respective first to ninth insertion holes (25a11 to 25a33) and the respective first to ninth coupling units (21c11 to 21c33). In other words, the drive wire W is prevented from engaging with an insertion hole 25a different from a corresponding insertion hole 25a, and a coupling unit 21c different from a corresponding coupling unit 21c.

By engaging the key shaft 15 and the key acceptance portion 22 with each other, the user can correctly couple the first to ninth drive wires (W11 to W33) to the respective first to ninth coupling units (21c11 to 21c33). Accordingly, the user can easily attach the catheter unit 100 to the base unit 200.

In the present exemplary embodiment, the key shaft 15 includes a protruding portion protruding in a direction intersecting with the attaching/detaching direction DE, and the key acceptance portion 22 includes a recess portion into which the protruding portion is to be inserted. In the circumferential direction, a position at which the protruding portion and the recess portion engage with each other is a position at which the drive wire W engages with a corresponding insertion hole 25a and a corresponding coupling unit 21c.

The key shaft 15 can be disposed in either one of the base unit 200 and the catheter unit 100, and the key acceptance portion 22 can be disposed in the other one. For example, the key shaft 15 may be disposed on the base unit 200, and the key acceptance portion 22 may be disposed on the catheter unit 100.

The base unit 200 includes a joint 28 including a j oint engagement portion 28j. The base frame 25 includes a lock shaft 26 including a lock protrusion 26a. Functions of these components will be described below.

### <Coupling between Motor and Drive Wire>

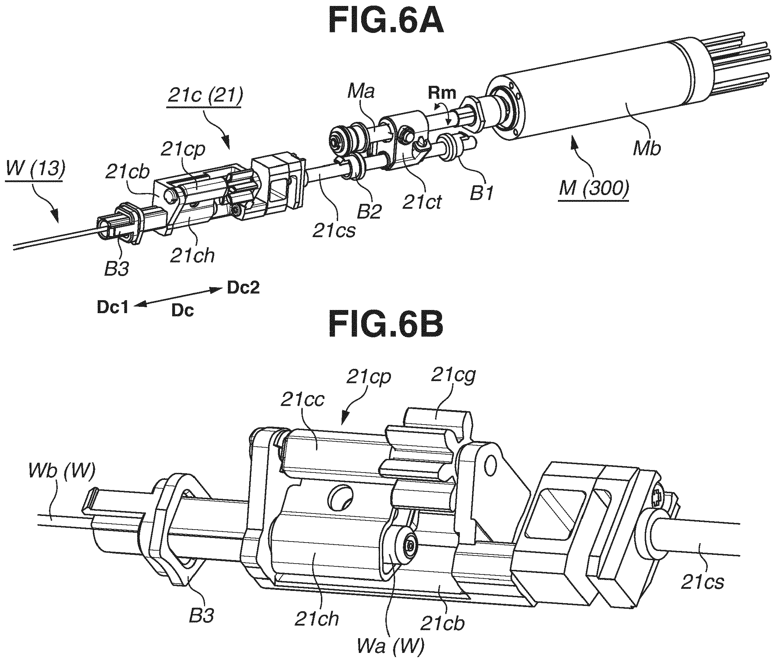

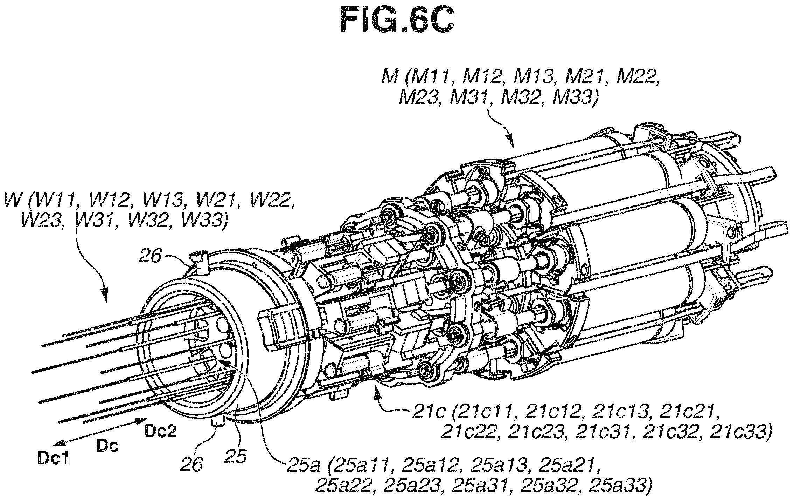

The coupling between the wire drive unit 300, the coupling device 21, and the bending drive unit 13 will be described with reference to Figs. 6A, 6B, and 6C. Figs. 6A, 6B, and 6C are explanatory diagrams illustrating the wire drive unit 300, the coupling device 21, and the bending drive unit 13. Fig. 6A is a perspective view illustrating the drive source M, the coupling unit 21c, and the drive wire W. Fig. 6B is an enlarged view illustrating the coupling unit 21c and the drive wire W. Fig. 6C is a perspective view illustrating the coupling between the wire drive unit 300, the coupling device 21, and the bending drive unit 13.

In the present exemplary embodiment, the first to ninth drive wires (W11 to W33) and the first to ninth coupling units (21c11 to 21c33) are respectively coupled to each other in the same configuration. In addition, the first to ninth coupling units (21c11 to 21c33) and the first to ninth drive sources (M11 to M33) are respectively connected to each other in the same configuration. Thus, in the following description, a configuration in which one drive wire W, one coupling unit 21c, and one drive source M are connected to each other will be described.

As illustrated in Fig. 6A, the drive source M includes an output shaft Ma, and a motor main body Mb that rotates the output shaft Ma in a rotational direction Rm. A spiral groove is formed on a surface of the output shaft Ma. The output shaft Ma has a so-called screw shape. The motor main body Mb is fixed to the motor frame 200b.

The coupling unit 21c includes a tractor 21ct connected to the output shaft Ma, and a tractor support shaft 21cs that supports the tractor 21ct. The tractor support shaft 21cs is connected to a coupling base 21cb.

The coupling unit 21c includes a plate spring 21ch serving as a holding unit for holding the held portion Wa of the drive wire W. The drive wire W engages with the coupling unit 21c through the insertion hole 25a. More specifically, the held portion Wa engages with the plate spring 21ch. As described below, the plate spring 21ch can be brought into a state (fixing state) in which the plate spring 21ch holds the held portion Wa to fix the held portion Wa, and a state (release state) in which the plate spring 21ch releases the held portion Wa.

The coupling unit 21c includes a pressing member 21cp. The pressing member 21cp includes a gear portion 21cg that engages with an internal gear 29 to be described below, and a cam 21cc serving as a pressing unit for pressing the plate spring 21ch.

As described below, the cam 21cc can be moved with respect to the plate spring 21ch. Movement of the cam 21cc switches the state of the plate spring 21ch between the fixing state and the release state.

The coupling unit 21c is supported by a first bearing B1, a second bearing B2, and a third bearing B3. The first bearing B 1 is supported by the first bearing frame 200c of the base unit 200. The second bearing B2 is supported by the second bearing frame 200d of the base unit 200. The third bearing B3 is supported by the third bearing frame 200e of the base unit 200. With this configuration, in rotation of the output shaft Ma in the rotational direction Rm, rotation of the coupling unit 21c around the output shaft Ma is restricted. The first bearing B 1, the second bearing B2, and the third bearing B3 are disposed for each of the first to ninth coupling units (21c11 to 21c33).

Since the rotation of the coupling unit 21c around the output shaft Ma is restricted, in rotation of the output shaft Ma, the spiral groove of the output shaft Ma causes the tractor 21ct to receive force in a rotational axis direction of the output shaft Ma. Consequently, the coupling unit 21c moves in the rotational axis line direction of the output shaft Ma (direction Dc). The movement of the coupling unit 21c moves the drive wire W, whereby the bending portion 12 bends. In this process, by switching the rotational direction of the drive source M, the coupling unit 21c can drive the drive wire W in both of a direction (direction Dc1) in which the drive wire W is pressed, and a direction (direction Dc2) in which the drive wire W is pulled.

That is, the output shaft Ma and the tractor 21ct form a so-called feed screw that converts rotational motion transmitted from the drive source M into linear motion. In the present exemplary embodiment, the output shaft Ma and the tractor 21ct correspond to a sliding screw but may correspond to a ball screw.

As illustrated in Fig. 6C, the catheter unit 100 is attached to the base unit 200, whereby the first to ninth drive wires (W11 to W33) and the first to ninth coupling units (21c11 to 21c33) are respectively coupled to each other.

The control unit 3 can control the first to ninth drive sources (M11 to M33) independently of each other. That is, an arbitrary drive source of the first to ninth drive sources (M11 to M33) can independently operate or stop irrespectively of whether the other drive sources are in a stopped state. In other words, the control unit 3 can control the first to ninth drive wires (W11 to W33) independently of each other. Consequently, the first to third guide rings (J1 to J3) are controlled independently of each other, and the bending region 12b of the bending portion 12 can bend in an arbitrary direction.

### <Attaching of Catheter Unit >

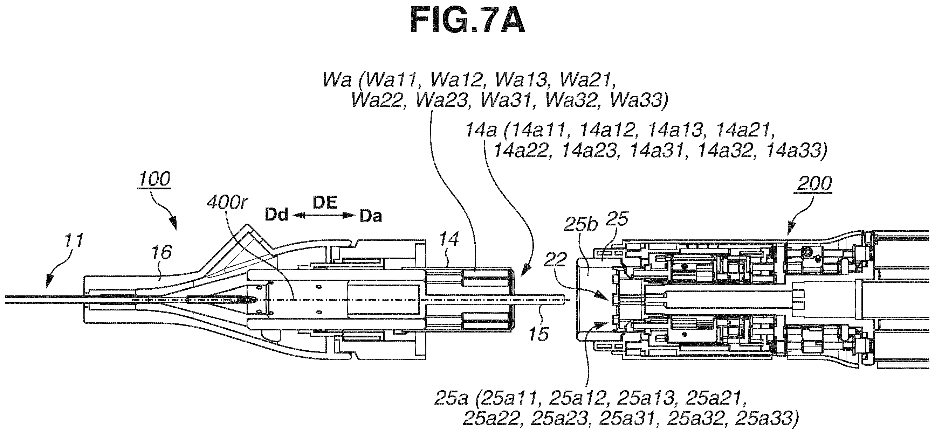

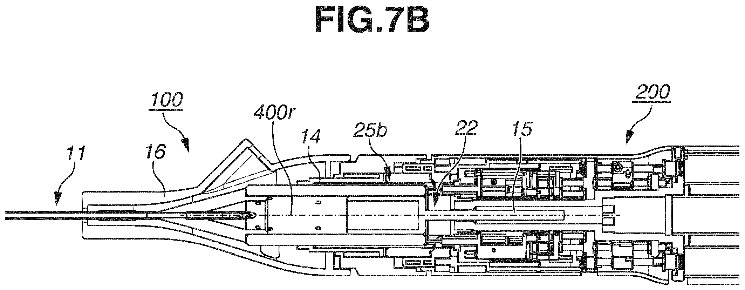

An operation of attaching the catheter unit 100 to the base unit 200 will be described with reference to Figs. 7A and 7B. Figs. 7A and 7B are explanatory diagrams illustrating the attaching of the catheter unit 100. Fig. 7A is a diagram illustrating a state in which the catheter unit 100 is to be attached to the base unit 200. Fig. 7B is a diagram illustrating a state in which the catheter unit 100 has been attached to the base unit 200.

In the present exemplary embodiment, the attaching/detaching direction DE of the catheter unit 100 is the same as the direction of the rotational axis 400r of the operation unit 400. In the attaching/detaching direction DE, a direction in which the catheter unit 100 is attached to the base unit 200 will be referred to as the attaching direction Da. In the attaching/detaching direction DE, a direction in which the catheter unit 100 is detached from the base unit 200 (the opposite direction of the attaching direction Da) will be referred to as a detaching direction Dd.

As illustrated in Fig. 7A, in a state in which the catheter unit 100 is to be attached to the base unit 200, the wire cover 14 is at the cover position. In this state, the wire cover 14 covers the first to ninth drive wires (W11 to W33) in such a manner as to prevent the first to ninth held portions (Wa11 to Wa33) from protruding from the first to ninth exposure holes (14a11 to 14a33) of the wire cover 14. With this configuration, in a state in which the catheter unit 100 is to be attached to the base unit 200, the first to ninth drive wires (W11 to W33) can be protected.

When the catheter unit 100 is attached to the base unit 200, the key shaft 15 is engaged with the key acceptance portion 22. The key shaft 15 protrudes from the wire cover 14. In the present exemplary embodiment, in a state in which the key shaft 15 has reached an inlet of the key acceptance portion 22, the wire cover 14 does not engage with the attachment opening 25b. That is, when a phase of the catheter unit 100 with respect to the base unit 200 is a phase at which the key shaft 15 and the key acceptance portion 22 cannot engage with each other, the wire cover 14 does not engage with the attachment opening 25b and the state in which the wire cover 14 is at the cover position is kept. With this configuration, even in a case where the catheter unit 100 is moved in such a manner that the key shaft 15 and the key acceptance portion 22 engage with each other, the first to ninth drive wires (W11 to W33) are protected.

Through the engagement between the key shaft 15 and the key acceptance portion 22 and the movement of the catheter unit 100 in the attaching direction Da with respect to the base unit 200, the catheter unit 100 is attached to the base unit 200. The attachment of the catheter unit 100 to the base unit 200 causes the wire cover 14 to move to the exposure position. In the present exemplary embodiment, coming into contact with the base frame 25, the wire cover 14 moves from the cover position to the exposure position (refer to Fig. 7B).

More specifically, in the attachment of the catheter unit 100, the wire cover 14 comes into contact with the base frame 25 and stops. The movement of the catheter unit 100 in the attaching direction Da in this state causes, in the catheter unit 100, the wire cover 14 to move relative to a portion other than the wire cover 14. This results in movement of the wire cover 14 from the cover position to the exposure position.

While the wire cover 14 moves from the cover position to the exposure position, the held portion Wa of the drive wire W protrudes from the exposure hole 14a of the wire cover 14 and is inserted into the insertion hole 25a. The held portion Wa engages with the plate spring 21ch of the coupling unit 21c (refer to Fig. 6B).

In a state in which the catheter unit 100 is merely attached to the base unit 200, the catheter unit 100 can be moved in the detaching direction Dd with respect to the base unit 200 and detached. As described below, the state in which the catheter unit 100 is merely attached to the base unit 200 is a state in which fixing between the drive wire W and the coupling unit 21c is released.

An operation on the operation unit 400 in a state in which the catheter unit 100 is attached to the base unit 200 prevents the catheter unit 100 from being detached from the base unit 200. Furthermore, an operation on the operation unit 400 in a state in which the catheter unit 100 is attached to the base unit 200 causes the bending drive unit 13 to be fixed to the coupling device 21, and the bending drive unit 13 is coupled to the wire drive unit 300 via the coupling device 21.

### <Fixing of Bending Drive Unit and Release of Fixing>

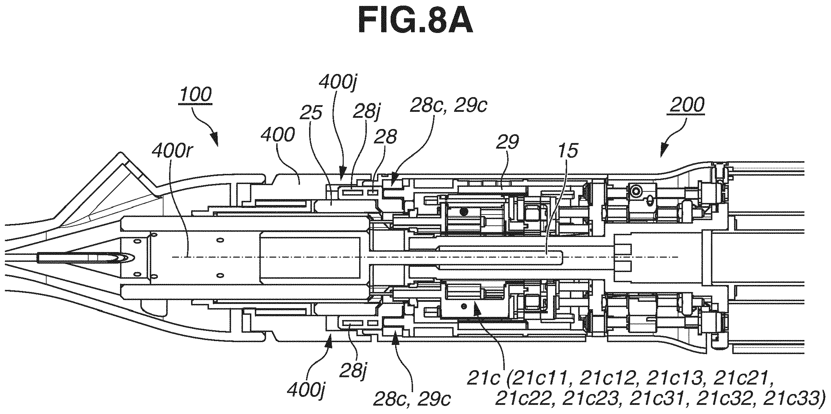

A configuration for fixing the bending drive unit 13 to the coupling device 21, and a configuration for releasing the fixing of the bending drive unit 13 in the coupling device 21 will be described with reference to Figs. 8A, 8B, 9, 10, 11, 12, 13, and 14.

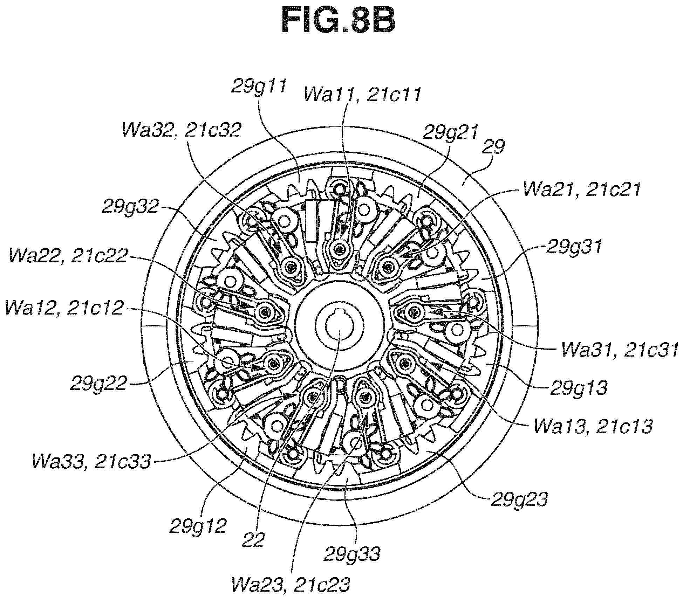

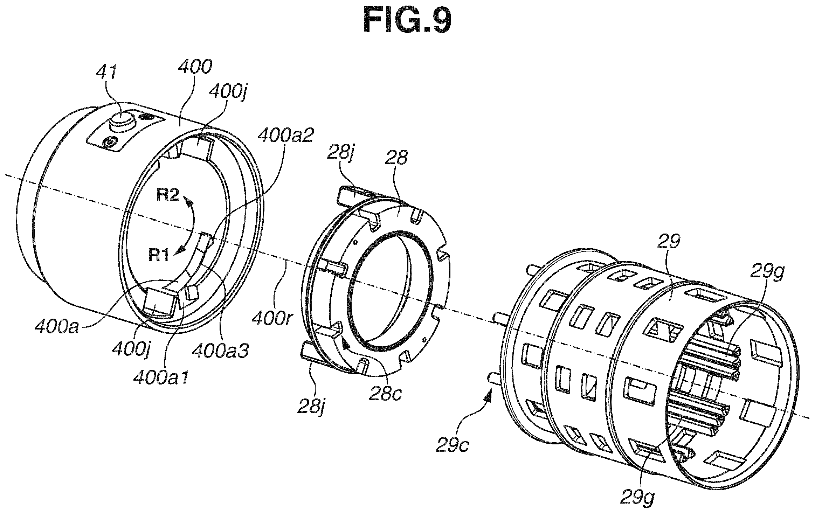

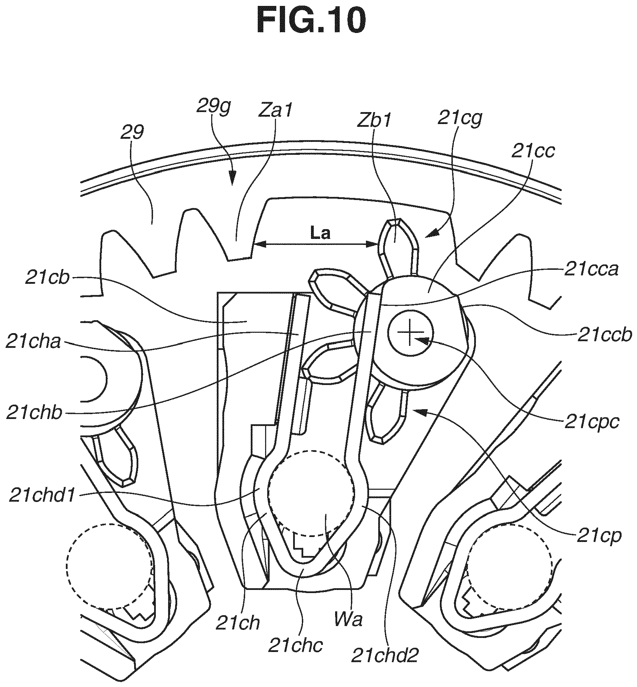

Figs. 8A and 8B are diagrams illustrating the coupling between the catheter unit 100 and the base unit 200. Fig. 8A is a cross-sectional view illustrating the catheter unit 100 and the base unit 200. Fig. 8A is a cross-sectional view illustrating the catheter unit 100 and the base unit 200 taken along the rotational axis 400r. Fig. 8B is a cross-sectional view illustrating the base unit 200. Fig. 8B is a cross-sectional view illustrating the base unit 200 taken along at a portion of the coupling unit 21c in a direction orthogonal to the rotational axis 400r. Fig. 9 is an exploded view illustrating the coupling between the catheter unit 100 and the base unit 200. Figs. 10, 11, 12, 13, and 14 are diagrams illustrating the fixing of the drive wire W with the coupling unit 21c.

As illustrated in Figs. 8A and 9, the base unit 200 includes the joint (intermediate member, second transmission member) 28, and the internal gear 29 serving as a movement gear (interlocking gear, transmission member, first transmission member) that interlocks with the operation unit 400 via the joint 28.

The joint 28 includes a plurality of transmission units 28c, and the internal gear 29 includes a plurality of transmission receiving units 29c. The plurality of transmission units 28c engage with the plurality of transmission receiving units 29c, and in a case where the joint 28 rotates, the rotation of the joint 28 is transmitted to the internal gear 29.

When the catheter unit 100 is attached to the base unit 200, an engagement portion 400j included in the operation unit 400 engages with the joint engagement portion 28j of the joint 28. In rotation of the operation unit 400, the rotation of the operation unit 400 is transmitted to the joint 28. The operation unit 400, the joint 28, and the internal gear 29 rotate in the same direction.

The internal gear 29 includes a plurality of gear tooth portions for switching the states of the first to ninth coupling units (21c11 to 21c33) between a state of fixing the respective first to ninth drive wires (W11 to W33), and a state of releasing the respective first to ninth drive wires (W11 to W33). The plurality of gear tooth portions (action portions, switching gear portions) of the internal gear 29 engage with the gear portions 21cg of the pressing members 21cp respectively included in the first to ninth coupling units (21c11 to 21c33).

Specifically, in the present exemplary embodiment, the internal gear 29 includes a first gear tooth portion 29g11, a second gear tooth portion 29g12, a third gear tooth portion 29g13, a fourth gear tooth portion 29g21, a fifth gear tooth portion 29g22, a sixth gear tooth portion 29g23, a seventh gear tooth portion 29g31, an eighth gear tooth portion 29g32, and a ninth gear tooth portion 29g33. The first to ninth gear tooth portions (29g11 to 29g33) are formed with clearance gaps therebetween.

The first gear tooth portion 29g11 engages with the gear portion 21cg of the first coupling unit 21c11. The second gear tooth portion 29g12 engages with the gear portion 21cg of the second coupling unit 21c12. The third gear tooth portion 29g13 engages with the gear portion 21cg of the third coupling unit 21c13. The fourth gear tooth portion 29g21 engages with the gear portion 21cg of the fourth coupling unit 21c21. The fifth gear tooth portion 29g22 engages with the gear portion 21cg of the fifth coupling unit 21c22. The sixth gear tooth portion 29g23 engages with the gear portion 21cg of the sixth coupling unit 21c23. The seventh gear tooth portion 29g31 engages with the gear portion 21cg of the seventh coupling unit 21c31. The eighth gear tooth portion 29g32 engages with the gear portion 21cg of the eighth coupling unit 21c32. The ninth gear tooth portion 29g33 engages with the gear portion 21cg of the ninth coupling unit 21c33.

An arbitrary one of the first to ninth gear tooth portions (29g11 to 29g33) can be called as the gear tooth portion 29g. In the present exemplary embodiment, the first to ninth gear tooth portions (29g11 to 29g33) have the same configuration.

In the present exemplary embodiment, the first to ninth drive wires (W11 to W33) and the first to ninth coupling units (21c11 to 21c33) are respectively coupled to each other in the same configuration. In addition, the first to ninth coupling units (21c11 to 21c33) and the first to ninth gear tooth portions (29g11 to 29g33) are respectively connected to each other in the same configuration. Thus, in the following description, a configuration in which one drive wire W, one coupling unit 21c, and one gear tooth portion 29g are connected will be described.

In each of the first to ninth coupling units (21c11 to 21c33), the gear portion 21cg is moved by the internal gear 29, the pressing member 21cp rotates, and the cam 21cc moves to a press position and a retracted position at which the cam 21cc is retracted from the press position.

Rotation of the operation unit 400 rotates the internal gear 29. The rotation of the internal gear 29 causes the first to ninth coupling units (21c11 to 21c33) to operate. That is, an operation of rotating one operation unit 400, causes the first to ninth coupling units (21c11 to 21c33) to be operated.

In a state in which the catheter unit 100 is attached to the base unit 200, the operation unit 400 can move to a fixing position (lock position) and a detaching position. As described below, in a state in which the catheter unit 100 is attached to the base unit 200, the operation unit 400 can move to a release position. In the circumferential direction of the operation unit 400, the release position is between the fixing position and the detaching position. In a state in which the operation unit 400 is at the detaching position, the catheter unit 100 is attached to the base unit 200.

The state in which the catheter unit 100 is attached to the base unit 200 is a state in which the fixing (lock) of the drive wire W with respect to the coupling unit 21c is released. This state is called a release state of the coupling unit 21c. A state in which the drive wire W is fixed (locked) to the coupling unit 21c is called a lock state of the coupling unit 21c.

An operation that is performed when the drive wire W is to be fixed to the coupling unit 21c will be described with reference to Figs. 10, 11, 12, 13, and 14.

In a state in which the catheter unit 100 has been attached to the base unit 200 and the operation unit 400 is to be operated, the catheter unit 100 can be detached from the base unit 200. Hereinafter, a state in which the catheter unit 100 can be detached from the base unit 200 is referred to as a detachable state.

Fig. 10 is a diagram illustrating states of the internal gear 29 and the coupling unit 21c in the detachable state. Fig. 10 is a diagram illustrating the internal gear 29 and the coupling unit 21c in a state in which the operation unit 400 is at the fixing position.

The plate spring 21ch of the coupling unit 21c includes a fixed portion 21cha fixed to the coupling base 21cb, and a pressed portion 21chb that is in contact with the cam 21cc of the pressing member 21cp. The plate spring 21ch includes a first portion 21chd1 and a second portion 21chd2. In attachment of the catheter unit 100 to the base unit 200, the held portion Wa is inserted between the first portion 21chd1 and the second portion chd2.

The cam 21cc has a holding surface 21cca and a pressing surface 21ccb. In a rotation radius direction of the pressing member 21cp, the holding surface 21cca is disposed at a position closer to a rotational center 21cpc of the pressing member 21cp than the pressing surface 21ccb.

As illustrated in Fig. 10, in the detachable state (state in which the operation unit 400 is at the detaching position), the plate spring 21ch is held at a position at which the pressed portion 21chb is in contact with the holding surface 21cca. In addition, a gear tooth Za1 of the internal gear 29 and a gear tooth Zb1 of the gear portion 21cg are stopped with a clearance La therebetween.

In the rotational direction of the operation unit 400, a direction in which the operation unit 400 moves toward the release position and the fixing position from the detaching position is called a lock direction (fixing direction), and a direction in which the operation unit 400 moves toward the release position and the detaching position from the fixing position is called a release direction. The operation unit 400 rotates from the release position in the release direction and moves to the detaching position. The operation unit 400 rotates from the release position in the lock direction and moves to the fixing position.

In a state in which the catheter unit 100 has been attached to the base unit 200 and the operation unit 400 is at the detaching position, the coupling unit 21c is in the release state, which is a state in which the fixing of the drive wire W in the coupling unit 21c has been released.

When the coupling unit 21c is in the release state, the cam 21cc is at the retracted position at which the cam 21cc has been retracted from the press position to be described below. In this state, the fixing of the held portion Wa by the plate spring 21ch is released. Force by which the first portion 21chd1 and the second portion 21chd2 tighten up the held portion Wa when the coupling unit 21c is in the release state is smaller than force by which the first portion 21chd1 and the second portion 21chd2 tighten up the held portion Wa when the coupling unit 21c is in the lock state.

When the coupling unit 21c is in the release state, in a case where the catheter unit is moved in the detaching direction Dd with respect to the base unit 200, the held portion Wa can be pulled out from between the first portion 21chd1 and the second portion 21chd2.

When the coupling unit 21c is in the release state, a state in which force by which the first portion 21chd1 and the second portion 21chd2 tighten up the held portion Wa is not generated (state in which the pressure is zero) is desirable. When the coupling unit 21c is in the release state, a clearance gap is desirably generated between at least either one of the first portion 21chd1 and the second portion 21chd2 and the held portion Wa.

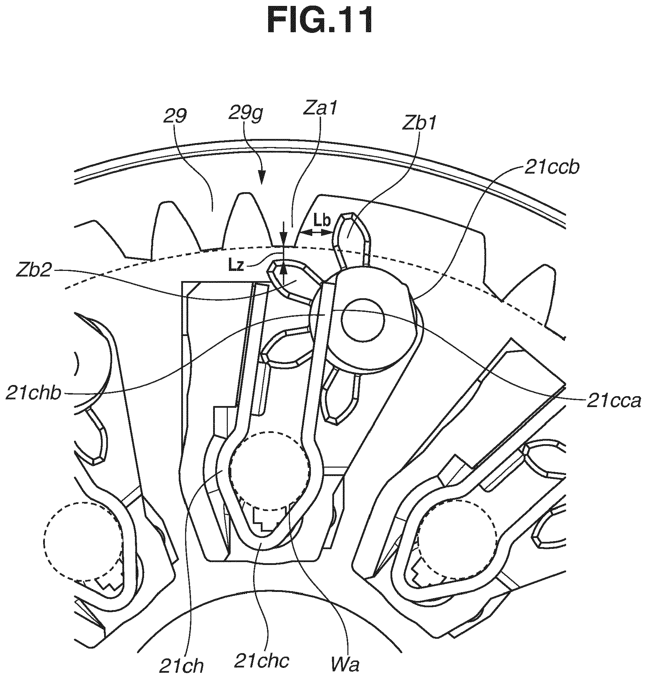

Fig. 11 is a diagram illustrating a state of the internal gear 29 and the coupling unit 21c in rotation of the operation unit 400 from the detaching position in the lock direction. Fig. 11 is a diagram illustrating the state of the internal gear 29 and the coupling unit 21c in a state in which the operation unit 400 is at the release position.

In a case where the operation unit 400 is rotated in the lock direction in a state in which the operation unit 400 is at the detaching position (Fig. 10), the internal gear 29 rotates clockwise. Then, the operation unit 400 is moved to the release position.

Since the key shaft 15 and the key acceptance portion 22 are engaged with each other even in a case where the operation unit 400 is rotated, the rotation of the entire catheter unit 100 (excluding the operation unit 400) with respect to the base unit 200 is restricted. That is, in a state in which the entire catheter unit 100 (excluding the operation unit 400) and the base unit 200 are stopped, the operation unit 400 can rotate with respect to these units.

In clockwise rotation of the internal gear 29, a clearance between the gear tooth Za1 of the internal gear 29 and the gear tooth Zb1 of the gear portion 21cg reduces from the clearance La to a clearance Lb.

A gear tooth Zb2 of the gear portion 21cg is disposed at a position with a clearance Lz from an addendum circle (dotted line) of the gear tooth portion 29g of the internal gear 29. Thus, the internal gear 29 can rotate without interfering with the gear tooth Zb2. Meanwhile, the coupling unit 21c is kept in the same state (release state) as the state illustrated in Fig. 10.

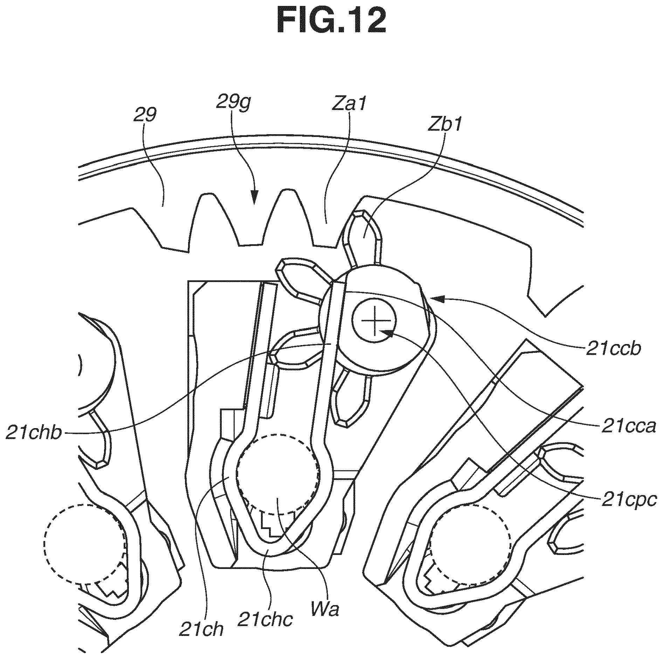

In a case where the operation unit 400 is further rotated in the lock direction from the state illustrated in Fig. 11, the internal gear 29 further rotates clockwise. Fig. 12 illustrates the state of the internal gear 29 and the coupling unit 21c in the rotation.

Fig. 12 is a diagram illustrating the state of the internal gear 29 and the coupling unit 21c in rotation of the operation unit 400 from the release position in the lock direction. As illustrated in Fig. 12, in rotation of the operation unit 400 from the release position in the lock direction, the gear tooth Za1 of the internal gear 29 and the gear tooth Zb1 of the gear portion 21cg come into contact with each other. Meanwhile, the coupling unit 21c remains in the same state as the state illustrated in Figs. 10 and 11, and kept in the release state.

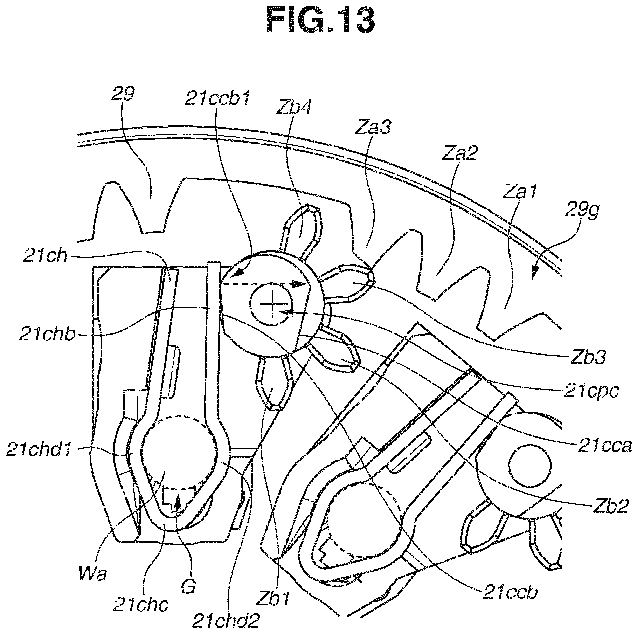

Fig. 13 is a diagram illustrating a state in which the pressing member 21cp has been rotated by the operation unit 400 rotating in the lock direction. As illustrated in Fig. 13, with further rotation of the operation unit 400 in the lock direction from the state illustrated in Fig. 12, the internal gear 29 further rotates clockwise.

In movement of the internal gear 29 from the state illustrated in Fig. 12 to the state illustrated in Fig. 13, the internal gear 29 rotates the gear portion 21cg clockwise. In the rotation of the gear portion 21cg, the holding surface 21cca is separated from the pressed portion 21chb and the pressing surface 21ccb moves closer to the pressed portion 21chb. Then, the first portion 21chd1 and the second portion 21chd2 start to hold the held portion Wa therebetween.

Then, with the pressed portion 21chb being pressed by a corner portion 21ccb1 disposed at the end portion of the pressing surface 21ccb, a gear tooth Za3 of the internal gear 29 moves up to a position at which the gear tooth Za3 is separated from a gear tooth Zb3 of the gear portion 21cg. In this state, the held portion Wa is held between the first portion 21chd1 and the second portion 21chd2.

When the gear tooth Za3 of the internal gear 29 has separated from the gear tooth Zb3 of the gear portion 21cg, transmission of drive force from the internal gear 29 to the gear portion 21cg ends. In this state, in the cam 21cc, the corner portion 21ccb1 receives reactive force from the plate spring 21ch.

In the rotation radius direction of the pressing member 21cp, reactive force of the plate spring 21ch that acts on the corner portion 21ccb1 acts on a position separated from the rotational center 21cpc of the pressing member 21cp, and the pressing member 21cp rotates clockwise. In the rotation, the pressing member 21cp rotates toward the same direction as a direction in which the pressing member 21cp is rotated by the internal gear 29 rotating clockwise.

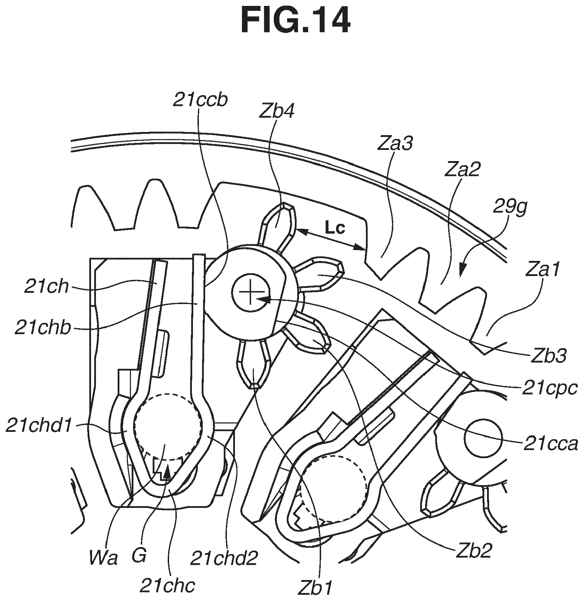

Fig. 14 is a diagram illustrating a state of the internal gear 29 and the coupling unit 21c in a state in which the operation unit 400 is at the fixing position. As illustrated in Fig. 14, the pressing member 21cp receives reactive force of the plate spring 21ch and further rotates from the state illustrated in Fig. 13.

As illustrated in Fig. 14, the pressing member 21cp stops in a state in which the pressing surface 21ccb of the cam 21cc and the pressed portion 21chb of the plate spring 21ch have surface contact with each other. That is, the pressing surface 21ccb and the surface of the pressed portion 21chb are on the same plane. In this state, the coupling unit 21c is in the lock state. When the coupling unit 21c is in the lock state, the cam 21cc of the pressing member 21cp is at the press position, and the pressing surface 21ccb presses the pressed portion 21chb.

When the coupling unit 21c is in the lock state, the held portion Wa is held between the first portion 21chd1 and the second portion 21chd2. That is, the plate spring 21ch is pressed by the cam 21cc, and the held portion Wa is tightened up by the plate spring 21ch. Consequently, the held portion Wa is fixed by the plate spring 21ch.

In the present exemplary embodiment, in the plate spring 21ch, the first portion 21chd1 and the second portion 21chd2 press the held portion Wa at positions separated from each other. Furthermore, a bending portion 21chc linking the first portion 21chd1 and the second portion 21chd2 is disposed between the first portion 21chd1 and the second portion 21chd2. The bending portion 21chc is disposed with a clearance gap G from the held portion Wa. With this configuration, the held portion Wa is stably fixed by the first portion 21chd1 and the second portion 21chd2.

Resin or metal can be used as the material of the plate spring 21ch, but metal is desirably used.

When the coupling unit 21c is in the lock state, extraction of the held portion Wa from between the first portion 21chd1 and the second portion 21chd2 is restricted.

The gear tooth Za3 of the internal gear 29 and a gear tooth Zb4 of the gear portion 21cg stop at positions with a clearance Lc therebetween. A surface at a gear tooth tip of the gear tooth Za3 is inclined in such a manner as to separate from the rotational axis line toward the downstream in the release direction, with respect to a cylindrical surface that comes into contact with surfaces at gear tooth tips of the other gear teeth Za1 and Za2 around the rotational axis line of the internal gear 29. With this configuration, in a case where the internal gear 29 is rotated in the release direction from the state in Fig. 14 as described below, the gear tooth Za3 of the internal gear 29 can reach the gear tooth Zb4 subsequent to the gear tooth Zb3 without prying the gear tooth Zb3 of the gear portion 21cg.

In releasing of fixing between the drive wire W and the coupling unit 21c, the operation unit 400 at the fixing position is rotated in the release direction. In the rotation, the internal gear 29 rotates counterclockwise from the state illustrated in Fig. 14. In the counterclockwise rotation of the internal gear 29, the gear tooth Za3 of the internal gear 29 comes into contact with the gear tooth Zb4 of the gear portion 2 leg, and the pressing member 21cp is rotated counterclockwise.

In further counterclockwise rotation of the internal gear 29, the fixing of the drive wire W by the coupling unit 21c is released. Operations to be performed by the internal gear 29 and the pressing member 21cp in this rotation are reverse of the above-described operations. That is, through reverse operations of the above-described operations that have been performed for fixing the drive wire W with the coupling unit 21c, the fixing of the drive wire W with the coupling unit 21c is released.

The above-described operations are performed by each of the first to ninth coupling units (21c11 to 21c33). More specifically, during the process of the operation unit 400 moving from the detaching position to the fixing position, by the movement (rotation) of the operation unit 400, the first to ninth coupling units (21c11 to 21c33) change from the release state to the lock state. During the process of the operation unit 400 moving from the fixing position to the detaching position, by the movement (rotation) of the operation unit 400, the first to ninth coupling units (21c11 to 21c33) change from the lock state to the release state. That is, by operating one operation unit 400, the user can switch the state of the plurality of coupling units between the release state and the lock state.

That is, there is no need of an operation unit for switching a state between the release state and the lock state, for each of the plurality of coupling units, and an operation on the operation unit by the user. This allows the user to easily attach or detach the catheter unit 100 to or from the base unit 200. Furthermore, the medical apparatus 1 can be simplified.

A state in which the first to ninth drive wires (W11 to W33) are respectively fixed by the first to ninth coupling units (21c11 to 21c33) is called a first state. A state in which the fixing of the first to ninth drive wires (W11 to W33) by the first to ninth coupling units (21c11 to 21c33) is released is called a second state.

The state is switched between the first state and the second state in conjunction with the movement of the operation unit 400. More specifically, the state is switched between the first state and the second state in conjunction with the movement of the operation unit 400 between the detaching position and the fixing position.

The internal gear 29 is configured to interlock with the operation unit 400. In the present exemplary embodiment, the joint 28 functions as a transmission member for interlocking the operation unit 400 and the internal gear 29. The internal gear 29 and the joint 28 have a function as an interlocking unit that interlocks with the operation unit 400 in such a manner that the state is switched between the first state and the second state in conjunction with the movement of the operation unit 400.

Specifically, in a state in which the catheter unit 100 is attached to the base unit 200, the internal gear 29 and the joint 28 move, in conjunction with the movement of the operation unit 400, a part (the pressed portion 21chb) of the plate spring 21ch with respect to the held portion Wa. In the movement of the pressed portion 21chb, the state of the coupling unit 21c is switched between the lock state and the release state.

A configuration in which the internal gear 29 is moved directly from the operation unit 400 may be employed. In this case, the internal gear 29 has a function as the interlocking unit.

### <Movement of Operation Unit>

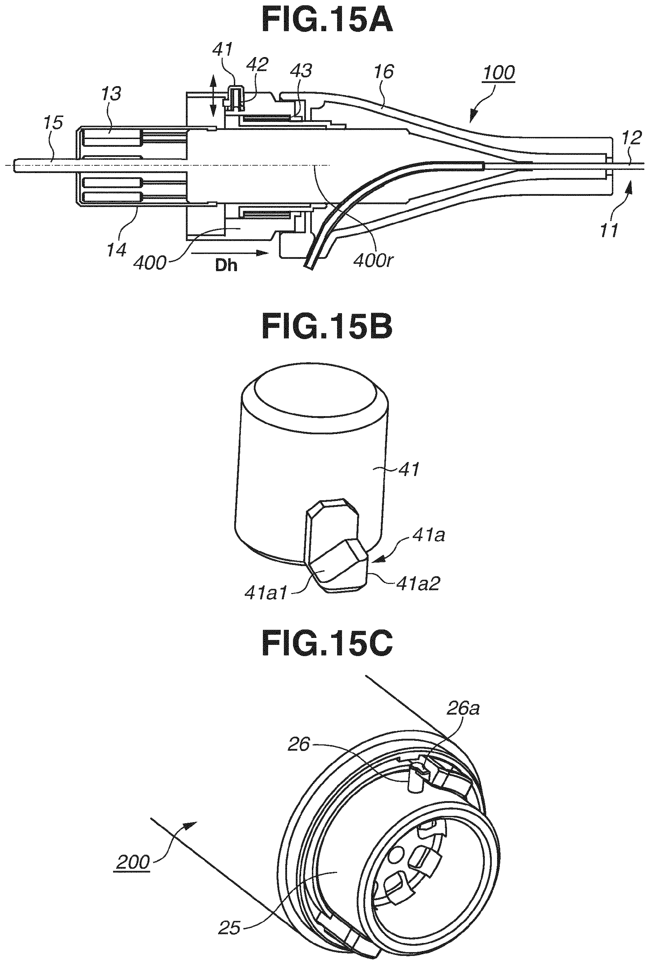

The movement of the operation unit 400 will be described with reference to Figs. 15A, 15B, 15C, 16A, 16B, 16C, 17A, 17B, and 17C.

In the present exemplary embodiment, the operation unit 400 is configured to be movable between the detaching position, the release position, and the fixing position in a state in which the catheter unit 100 is attached to the base unit 200. The release position is between the detaching position and the fixing position.

In the present exemplary embodiment, the state is switched between the first state and the second state in conjunction with the movement of the operation unit 400 between the release position and the fixing position.

In the present exemplary embodiment, movement of the operation unit 400 in a direction different from the attaching/detaching direction DE allows the operation unit 400 to move between the detaching position and the fixing position. The operation unit 400 moves between the detaching position and the fixing position by moving in a direction intersecting with (desirably, direction orthogonal to) the attaching/detaching direction DE. In the present exemplary embodiment, the operation unit 400 moves between the detaching position and the fixing position by rotating around the rotational axis 400r extending in the attaching/detaching direction DE. This results in excellent operability of the operation unit 400 by the user.

Figs. 15A, 15B, and 15C are explanatory diagrams illustrating the catheter unit 100 and the base unit 200. Fig. 15A is a cross-sectional view illustrating the catheter unit 100. Fig. 15B is a perspective view illustrating a button 41. Fig. 15C is a perspective view illustrating the base unit 200.

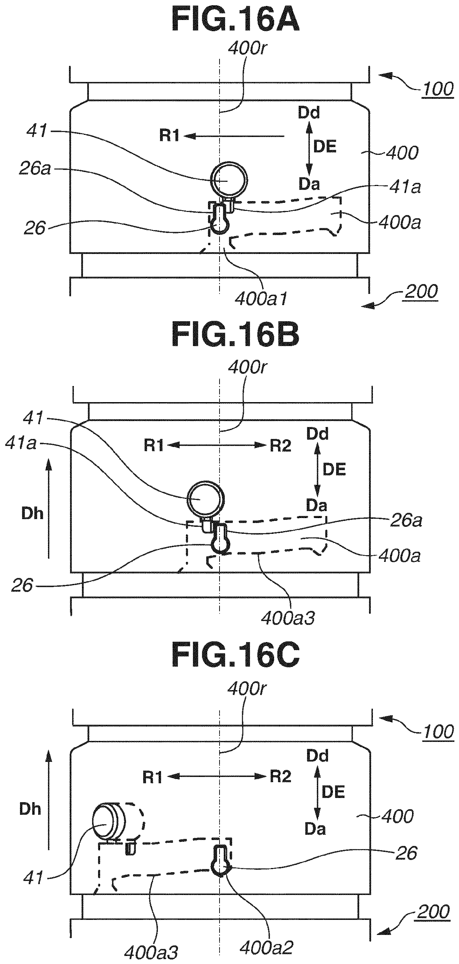

Figs. 16A, 16B, and 16C are diagrams illustrating an operation that is performed by the operation unit 400. Fig. 16A is a diagram illustrating a state in which the operation unit 400 is at the detaching position. Fig. 16B is a diagram illustrating a state in which the operation unit 400 is at the release position. Fig. 16C is a diagram illustrating a state in which the operation unit 400 is at the fixing position.

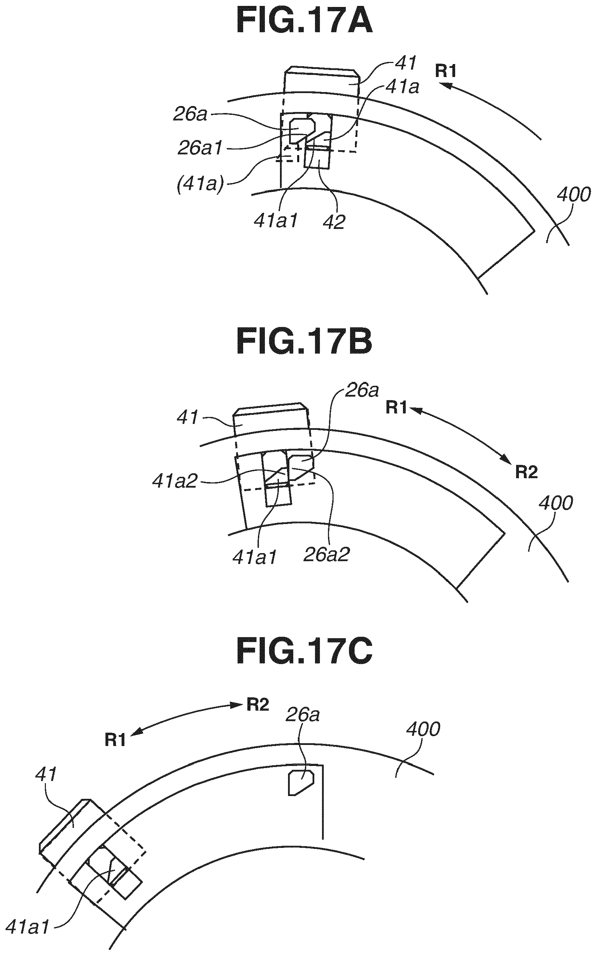

Figs. 17A, 17B, and 17C are cross-sectional views illustrating an operation that is performed by the operation unit 400. Fig. 17A is a cross-sectional view illustrating a state in which the operation unit 400 is at the detaching position. Fig. 17B is a cross-sectional view illustrating a state in which the operation unit 400 is at the release position. Fig. 17C is a cross-sectional view illustrating a state in which the operation unit 400 is at the fixing position.

When the operation unit 400 is at the fixing position, the coupling unit 21c is in the lock state, and the held portion Wa of the drive wire W is fixed to a corresponding coupling unit 21c (refer to Fig. 14).

When the operation unit 400 is at the release position, the coupling unit 21c is in the release state, and the lock between the held portion Wa of the drive wire W and the coupling unit 21c is released (refer to Fig. 11). In this state, the connection between the drive wire W and the wire drive unit 300 is cut off. Accordingly, in a case where the catheter 11 receives external force, the bending portion 12 can be freely bent without receiving resistance generated by the wire drive unit 300.

When the operation unit 400 is at the detaching position, the catheter unit 100 can be detached from the base unit 200. In a state in which the operation unit 400 is at the detaching position, the catheter unit 100 can be attached to the base unit 200. When the operation unit 400 is at the detaching position, the coupling unit 21c is in the release state, and the lock between the held portion Wa of the drive wire W and the coupling unit 21c is released (refer to Fig. 10).

As illustrated in Fig. 15A, the catheter unit 100 includes an operation unit urging spring 43 that urges the operation unit 400, the button 41 serving as a movement member, and a button spring 42 that urges the button 41.

In the present exemplary embodiment, the operation unit urging spring 43 is a compression spring. The operation unit 400 is urged by the operation unit urging spring 43 toward a direction Dh in which the operation unit 400 moves closer to the proximal end cover 16.

In the present exemplary embodiment, the button 41 and the button spring 42 are included in the operation unit 400. In the movement of the operation unit 400 to the detaching position, the release position, and the fixing position, the button 41 and the button spring 42 move together with the operation unit 400.

The button 41 is configured to be movable with respect to the operation unit 400 toward a direction intersecting with the direction of the rotational axis 400r of the operation unit 400. The button 41 is urged by the button spring 42 toward the outside of the catheter unit 100 (direction in which the button 41 moves away from the rotational axis 400r).

As described below, the movement of the operation unit 400 from the release position to the detaching position is restricted by the button 41. Movement of the button 41 with respect to the operation unit 400 enables the operation unit 400 to be moved from the release position to the detaching position.

The button 41 includes a button protrusion (restricted portion) 41a. The button protrusion 41a has an inclined surface 41a1 and a restricted surface 41a2.

The base unit 200 includes the base frame 25. The base frame 25 includes the lock shaft 26. The lock shaft 26 includes the lock protrusion (restriction portion) 26a.

In the present exemplary embodiment, a plurality of lock shafts 26 (two lock shafts 26 in the present exemplary embodiment) are disposed. The lock shafts 26 may each include the lock protrusion 26a, or some of the lock shafts 26 may each include the lock protrusion 26a.

As illustrated in Figs. 9, 16A, 16B, and 16C, a lock groove 400a that engages with the lock shaft 26 is formed inside the operation unit 400. The lock groove 400a has a length in a direction different from the attaching/detaching direction DE. In the present exemplary embodiment, the lock groove 400a has a length in the rotational direction of the operation unit 400. It can also be said that the lock groove 400a has a length in a direction intersecting with (orthogonal to) the attaching/detaching direction DE.

In a case where a plurality of lock shafts 26 are disposed, the lock grooves 400a are formed in such a manner as to respectively correspond to the plurality of lock shafts 26.

As illustrated in Fig. 16A, in attachment of the catheter unit 100 to the base unit 200, the lock shaft 26 engages with the lock groove 400a via an inlet 400a1 of the lock groove 400a.

In the attachment, the operation unit 400 is at the detaching position, and the coupling unit 21c is in the release state (refer to Fig. 10). In this state, the fixing of the first to ninth drive wires (W11 to W33) that are respectively fixed by the first to ninth coupling units (21c11 to 21c33) is released. As illustrated in Fig. 17A, the button protrusion 41a and the lock protrusion 26a face each other.

In rotation of the operation unit 400 in a lock direction R1 in a state in which the operation unit 400 is at the detaching position, the inclined surface 41a1 of the button protrusion 41a comes into contact with an inclined surface 26a1 of the lock protrusion 26a. Against urging force of the button spring 42, the button 41 moves toward the inside of the operation unit 400 (a direction in which the button 41 moves closer to the rotational axis 400r). Then, the button protrusion 41a moves over the lock protrusion 26a, and the operation unit 400 moves to the release position (refer to Fig. 17B).

In the movement, the coupling unit 21c is in the release state (refer to Fig. 11). In this state, the fixing of the first to ninth drive wires (W11 to W33) that is respectively fixed by the first to ninth coupling units (21c11 to 21c33) is released.

In the present exemplary embodiment, the operation unit 400 is allowed to be moved from the detaching position to the release position without an operation on the button 41. That is, in the movement of the operation unit 400 from the detaching position to the release position, the user needs not operate the button 41.

In rotation of the operation unit 400 in the lock direction R1 in a state in which the operation unit 400 is at the release position, the operation unit 400 moves to the fixing position. In a state in which the operation unit 400 is at the fixing position, a positioning unit 400a2 of the lock groove 400a is at a position corresponding to the lock shaft 26. The operation unit 400 is urged by the operation unit urging spring 43 toward the direction Dh in which the operation unit 400 moves closer to the proximal end cover 16. Consequently, the positioning unit 400a2 engages with the lock shaft 26.

During the process of the movement of the operation unit 400 from the release position to the fixing position, the held portion Wa of the drive wire W is fixed to the coupling unit 21c as described above.

In a state in which the operation unit is at the fixing position, the coupling unit 21c is in the lock state (refer to Fig. 14). Accordingly, the first to ninth drive wires (W11 to W33) are respectively fixed to the first to ninth coupling units (21c11 to 21c33). In this case, drive force can be transmitted from the wire drive unit 300 to the bending drive unit 13. That is, drive force is transmitted from the first to ninth drive sources (M11 to M33) to the first to ninth drive wires (W11 to W33) via the first to ninth coupling units (21c11 to 21c33), respectively.

When the operation unit 400 is at the release position, a wall 400a3 forming the lock groove 400a is on the upstream side of the lock shaft 26 in the detaching direction Dd of the catheter unit 100. When the operation unit 400 is at the fixing position the positioning unit 400a2 is on the upstream side of the lock shaft 26 in the detaching direction Dd. Consequently, when the operation unit 400 is at the release position, or when the operation unit 400 is at the fixing position, the detachment of the catheter unit 100 from the base unit 200 is restricted. Meanwhile, when the operation unit 400 is at the detaching position, the inlet 400a1 of the lock groove 400a is on the upstream side of the lock shaft 26 in the detaching direction Dd. Consequently, the catheter unit 100 is allowed to be detached from the base unit 200.

In rotation of the operation unit 400 toward a release direction R2 in a state in which the operation unit 400 is at the fixing position, the operation unit 400 is moved at the release position. During the process of the movement of the operation unit 400 from the fixing position to the release position, as described above, the held portion Wa of the drive wire W is released from the coupling unit 21c.

In a state in which the operation unit 400 is at the release position, the restricted surface 41a2 of the button protrusion 41a comes into contact with a restriction surface 26a2 of the lock protrusion 26a (refer to Fig. 17B). In this state, rotation of the operation unit 400 in the release direction R2 is restricted. In addition, detachment of the catheter unit 100 from the base unit 200 is restricted.

When the user presses the button 41 toward the inside of the operation unit 400 in a state in which the operation unit 400 is at the release position, the restricted surface 41a2 moves away from the restriction surface 26a2 and the button protrusion 41a moves over the lock protrusion 26a. This allows the operation unit 400 to be rotated in the release direction R2, and the operation unit 400 can move from the release position to the detaching position.

When the operation unit 400 is moved to the detaching position, the coupling unit 21c enters the release state. With this configuration, load acting on the drive wire W (for example, resistance to be received by the coupling unit 21c) can be reduced in detachment and attachment of the catheter unit 100 from and to the base unit 200. Thus, the user can easily attach or detached the catheter unit 100.

When the operation unit 400 is moved to the release position, detachment of the catheter unit 100 from the base unit 200 is restricted, and the coupling unit 21c enters the release state. As described above, when the coupling unit 21c is in the release state, the connection between the drive wire W and the wire drive unit 300 is cut off, and the bending portion 12 can be freely bent without receiving resistance generated by the wire drive unit 300.

By moving the operation unit 400 to the release position in a state in which the catheter 11 is inserted in a target, the user can stop driving of the catheter 11 that is performed by the wire drive unit 300. Furthermore, since detachment of the catheter unit 100 from the base unit 200 is restricted, the user can pull out the catheter 11 from the inside of the target while holding the base unit 200.

In the configuration of the present exemplary embodiment, in a case where the button 41 is not operated, the movement of the operation unit 400 from the release position to the detaching position is restricted. Thus, the user is prevented from wrongly moving the operation unit 400 up to the detaching position when the user moves the operation unit 400 from the fixing position to the release position.

In the present exemplary embodiment, the number of lock protrusions 26a is one, and the number of buttons 41 is one. Alternatively, the medical apparatus 1 may include a plurality of lock protrusions 26a and a plurality of buttons 41.

### <Connection Unit>

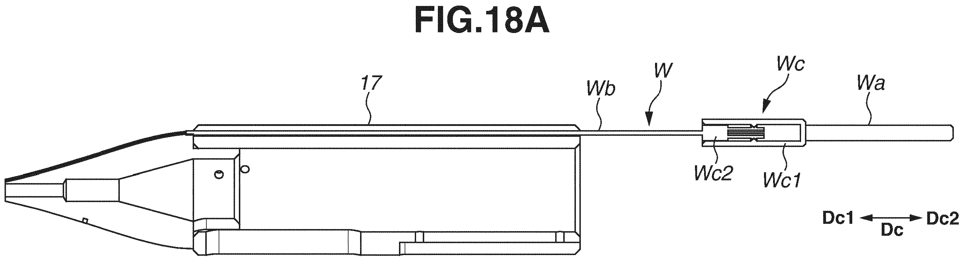

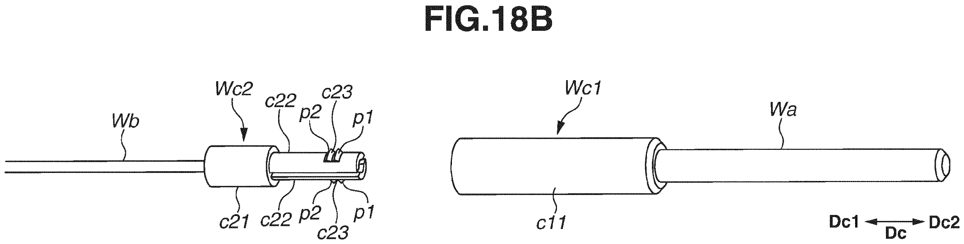

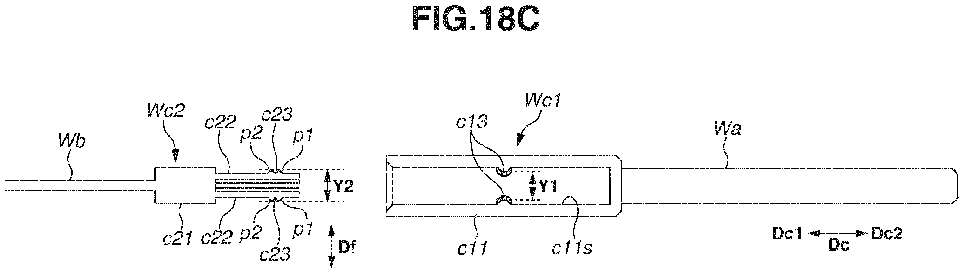

Next, the connection unit Wc according to the present exemplary embodiment will be described with reference to Figs. 18A, 18B, 18C, 19A, 19B, 19C, 20A, 20B, and 20C. Fig. 18A is a schematic diagram illustrating the drive wire W according to the present exemplary embodiment that includes the connection unit Wc. Fig. 18B is a perspective view illustrating a holder Wc1 and a rod Wc2 that are included in the connection unit Wc. Fig. 18C is a cross-sectional view illustrating the holder Wc1 and the rod Wc2 that are included in the connection unit Wc. Fig. 18A illustrates a state (connected state) in which the rod Wc2 is held in the holder Wc1, and Figs. 18B and 18C illustrate a state (cutoff state) in which the rod Wc2 is not held in the holder Wc1.

In the following description, the connection unit Wc disposed in the drive wire W, which is an arbitrary one of the first to ninth drive wires (W 11 to W33) included in the catheter unit 100 according to the present exemplary embodiment will be described. In the configuration example according to the present exemplary embodiment, the connection unit Wc having substantially the same configuration as the configuration to be described below is disposed in each of the nine drive wires (W11 to W33).

As illustrated in Fig. 18A, the connection unit Wc includes the holder Wc1 serving as a first member (engagement member, holding member) that is connected to the drive source M, and the rod Wc2 serving as a second member (engaged member, held member) that is connected to the wire member Wb of the drive wire W. Here, a state of being connected to the drive source M and a state of being connected to the wire member Wb respectively indicate a state in which a corresponding member is disposed on an upstream side (drive source side) and a state in which a corresponding member is disposed on a downstream side (wire member side) on a drive transmission path from the drive source M to the wire member Wb, and do not require the corresponding members to be physically in direct contact.

In the present exemplary embodiment, the holder Wc1 is a member formed integrally with the held portion Wa of the drive wire W. Thus, the holder Wc1 is connected to the drive source M via the held portion Wa and the coupling unit 21c (Figs. 6A, 6B, and 6C). Alternatively, the holder Wc1 and the held portion Wa may be formed as separate members. Meanwhile, the rod Wc2 is fixed to a proximal end of the wire member Wb using an arbitrary fixing method, such as pressure bonding (swaging) or adhesive bonding.

As illustrated in Fig. 18A, in the connected state in which the rod Wc2 is held in the holder Wc1, drive force of the drive source M is transmitted to the wire member Wb via the connection unit Wc. As described above, by switching the rotational direction of the drive source M, the drive wire W is driven in both the direction Dc1, which is a direction in which the proximal end of the wire member Wb is pressed toward the distal end, and the direction Dc2, which is a direction in which the proximal end of the wire member Wb is pulled in an opposite direction to the direction Dc1. The length direction of the wire member Wb in the connection unit Wc is the same as direction Dc which substantially coincides with the axis line direction of the output shaft Ma (Fig. 6A) of the drive source. The direction Dc1 is one direction of the direction Dc, and the direction Dc2 is the other direction of the direction Dc.

As illustrated in Figs. 18B and 18C, the holder Wc1 includes a cylindrical portion c11 that has a length in the direction Dc, and has a substantially bottomed cylindrical shape opened toward the direction Dc1. The cylindrical portion c11 is arranged adjacent to the held portion Wa in the direction Dc on the same axis, and the held portion Wa has a length in the direction Dc2 from a bottom portion of the cylindrical portion c11. The cylindrical portion c11 is an example of a tubular portion that has a length in the length direction of the wire member Wb and forms a space into which the second member can be inserted. For example, the cylindrical portion c11 may have a polygonal tubular shape. The cylindrical portion c11 has higher bending rigidity in a direction Df, which is a main deformation direction of a protruding piece c22 in the rod Wc2 that is to be described below, as compared with the protruding piece c22.

In addition, the holder Wc1 includes a protruding portion c13 formed inside the cylindrical portion c11. The protruding portion c13 is formed in such a manner as to protrude from an inner surface of the cylindrical portion c11 toward an axis center. In other words, the protruding portion c13 is an example of a protruding portion protruding in a direction intersecting with the length direction (direction Dc) of the wire member Wb in the connection unit Wc.

When viewed in the direction Dc, the protruding portion c13 is disposed at a position that corresponds to a recess portion c23 in the rod Wc2 to be described below. In the present exemplary embodiment, in the circumferential direction of the cylindrical portion c11, the protruding portions c13 are disposed at two positions separated from each other by 180 degrees, in such a manner as to respectively correspond to the recess portions c23 disposed at two positions separated from each other by 180 degrees. Because the protruding portion c13 is only required to be disposed at a position at which the protruding portion c13 can engage with the recess portion c23, for example, an annular protruding portion c13 may be formed over the entire circumference of the inner surface of the cylindrical portion c11.

As illustrated in Figs. 18B and 18C, the rod Wc2 includes a substantially columnar base portion c21 which has a length in the direction Dc2 from the proximal end of the wire member Wb, and two protruding pieces c22 protruding in the direction Dc2 from the base portion c21. The protruding pieces c22 each have a shape which is a part of a cylindrical shape having an outer diameter smaller than the base portion c21 and is of two parts divided from the cylindrical shape on a plane extending in the direction Dc. That is, the two protruding pieces c22 are two arcs disposed at positions opposed to each other on a common circumference, when viewed in the direction Dc2.

Thus, in a case where the protruding pieces c22 receive external force, the protruding pieces c22 mainly deform toward the direction Df which is a direction in which the two protruding pieces c22 are opposed to each other through the center of the common circumference. That is, the protruding piece c22 functions as a deformation element deformable in such a manner as to allow the protruding portion c13 to withdraw from the recess portion c23. The direction Df is a direction vertically intersecting with the length direction (direction Dc) of the wire member Wb in the connection unit Wc. The shape of the protruding pieces c22 is not limited to the above-described shape as long as the shape is a shape elastically deformable with moderate rigidity in the direction Df.

In addition, the rod Wc2 includes the recess portion c23 on an outer surface of each of the protruding pieces c22. The recess portion c23 is formed between protrusions p1 and p2 protruding outward from the outer surface of the protruding piece c22. The protrusions p1 and p2 are arranged side by side in the direction Dc.

The rod Wc2 is inserted in the direction Dc2 toward a space c11s inside the cylindrical portion c11 of the holder Wc1, and is attached to the holder Wc1 by being pressed in until the protruding portion c13 of the holder Wc1 fits into the recess portion c23 of the rod Wc2. In the present exemplary embodiment, the entire rod Wc2 (i.e., portion from the base portion c21 to the protruding piece c22) is an insertion portion that is inserted into the cylindrical portion c11. Thus, the recess portion c23 of the protruding piece c22 is disposed on the outer surface of the insertion portion to be inserted into the cylindrical portion c11.

Hereinafter, a state in which the rod Wc2 is held in the holder Wc1 in such a manner that drive force in the direction Dc1 or the direction Dc2 that has been transmitted from the drive source M can be transmitted to the wire member Wb will be referred to as a connected state (engaged state, attached state) of the connection unit Wc. A state in which the protruding portion c13 withdraws from the recess portion c23 and the connection between the drive source M and the wire member Wb is cut off will be referred to as a cutoff state (unconnected state, withdraw state, detachment state) of the connection unit Wc.

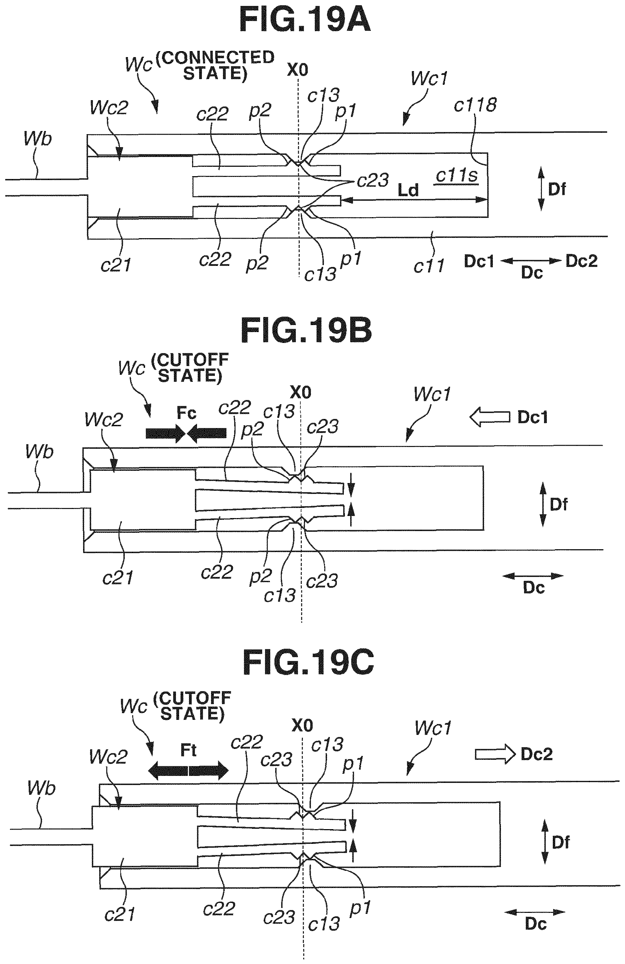

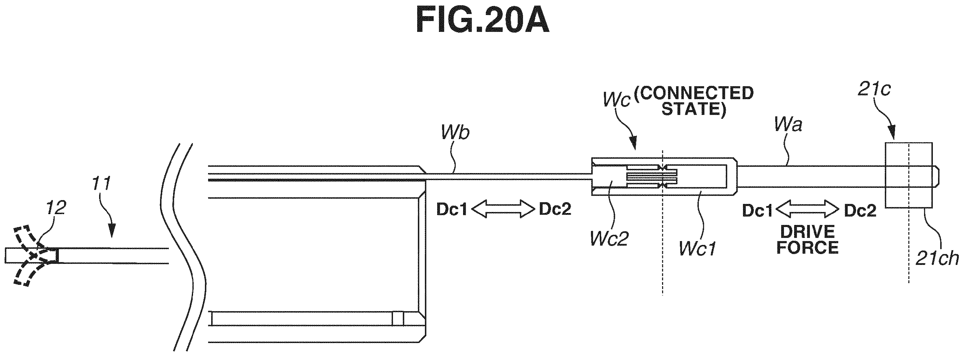

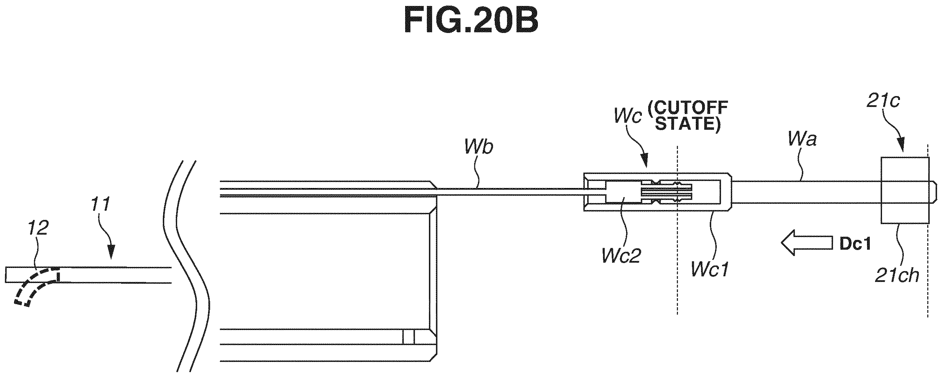

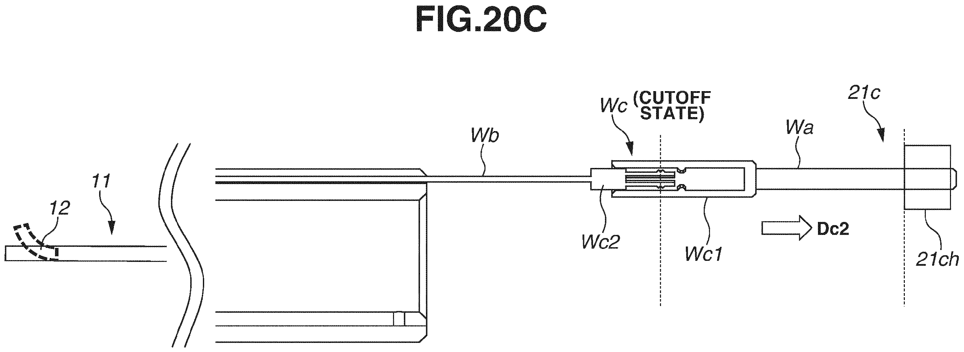

Next, movement of the connection unit Wc in the connected state and in an overload state will be described with reference to Figs. 19A, 19B, 19C, 20A, 20B, and 20C. Fig. 19A is a schematic diagram illustrating a cross-section of the connection unit Wc in the connected state. Fig. 19B is a schematic diagram illustrating a state of the connection unit Wc in a case where overload acts in a direction (direction Dc1) in which the wire member Wb is pressed. Fig. 19C is a schematic diagram illustrating a state of the connection unit Wc in a case where overload acts in a direction (direction Dc2) in which the wire member Wb is pulled. Fig. 20A is a schematic diagram illustrating movement of the catheter 11 in a case where the connection unit Wc is in the connected state. Fig. 20B is a schematic diagram illustrating movement of the catheter 11 in a case where overload acts in the direction (direction Dc1) in which the wire member Wb is pressed. Fig. 20C is a schematic diagram illustrating movement of the catheter 11 in a case where overload acts in the direction (direction Dc2) in which the wire member Wb is pulled.

As described above, in the present exemplary embodiment, the catheter 11 is operated by pressing or pulling the wire member Wb. In contrast to this, a configuration in which the catheter 11 is operated by just pulling the wire member Wb without pressing the wire member Wb is also conceivable. However, in such a configuration, the number of actuators and the number of drive wires increase, which leads to upsizing of the medical apparatus 1 and also increase in the cost. For the above-described reasons, in the present exemplary embodiment, a configuration in which the catheter 11 is operated by pressing or pulling the wire member Wb is employed.

### (Connected State)

As illustrated in Fig. 19A, in the connected state of the connection unit Wc, the protruding portion c13 of the holder Wc1 is fitted in the recess portion c23 of the rod Wc2. Specifically, the protruding portion c13 is held between the protrusions p1 and p2 in the direction Dc. Here, as illustrated in Fig. 18C, a distance in the direction Df between the tops of the protrusions p1 and a distance in the direction Df between the tops of the protrusions p2 in a state in which the rod Wc2 is not attached to the holder Wc1 correspond to substantially the same distance, which is a distance Y2. This distance Y2 is longer than a distance Y1 in the direction Df between the protruding portions c13 of the holder Wc1 in a state in which the rod Wc2 is not attached to the holder Wc1. Thus, in the connected state illustrated in Fig. 19A, the protruding portions c13 of the holder Wc1 and a part (protrusions p1 and p2) of the rod Wc2 are in a positional relationship of interfering with each other in the direction Dc. In other words, when viewed in the length direction (direction Dc) of the wire member in the connected state, at least part of the protruding portion formed on one of the first member or the second member overlaps the recess portion formed on the other one of the first member or the second member.

With such a configuration, in a case where the connection unit Wc is in the connected state, the holder Wc1 and the rod Wc2 integrally move in the direction Dc1 and the direction Dc2. In other words, in a case where the connection unit Wc is in the connected state, as illustrated in Fig. 20A, the plate spring 21ch of the coupling unit 21c moves the held portion Wa by drive force from the drive source M, which results in transmission of the drive force to the wire member Wb via the connection unit Wc. In the transmission, in response to transmitted drive force in the direction (direction Dc1) in which the wire member Wb is pressed, the bending portion 12 of the catheter 11 bends in such a manner that a side through which the wire member Wb is inserted becomes the outer side of bending. In addition, in response to transmitted drive force in the direction (direction Dc2) in which the wire member Wb is pulled, the bending portion 12 of the catheter 11 bends in such a manner that a side through which the wire member Wb is inserted becomes the outer side of bending.

In the connected state illustrated in Fig. 19A, the protruding portion c13 of the holder Wc1 is desirably configured to fit into the recess portion c23 of the rod Wc2 in a backlash-reduced state. In the present exemplary embodiment, the protruding piece c22 on which the recess portion c23 is formed is made of elastic material, and because the protruding piece c22 is in a slightly-elastically-deformed state in the direction Df in the connected state, the protruding portion c13 is pressed against the protrusions p1 and p2 on both sides by the elastic force of the protruding piece c22.

In other words, in the present exemplary embodiment, by a mechanism (snap-fit mechanism) that uses the deformation element (elastic element) made of elastic material, the holder Wc1 serving as the first member and the rod Wc2 serving as the second member are engaged with each other. In other words, in the connection unit according to the present exemplary embodiment, in a case where force at a threshold value or less (pulling force at a first threshold value or less or compression force at a second threshold value or less, which will be described below) acts between the first member and the second member, by the elastic force of the elastic element, a state in which the protruding portion is fitted in the recess portion is maintained. Employing the snap-fit mechanism as the connection unit Wc allows the connection unit Wc to be assembled by a simple operation of inserting the rod Wc2 into the holder Wc1.

Specifically, in a state in which the rod Wc2 is attached to the holder Wc1 as illustrated in Fig. 19A, the protrusions p1 and p2 of the rod Wc2 coming into contact with the protruding portion c13 of the holder Wc1 causes the protruding piece c22 to be in a slightly-bent state as compared with the state before the attachment of the rod Wc2 (Fig. 18C). That is, a distance Y2' between the tops of the protrusions p1 in a state in which the rod Wc2 has been attached to the holder Wc1 is slightly smaller than the distance Y2 between the tops of the protrusions p1 in a state in which the rod Wc2 is not attached to the holder Wc1.

In this manner, the configuration of engaging the holder Wc1 and the rod Wc2 with each other in the backlash-reduced state leads to improvement in responsivity of the bending portion 12 of the catheter 11 to the drive from the drive source M.

### (When Overload in Pressing Direction Occurs)

Next, a case where excessive force acts on the connection unit Wc in the direction (direction Dc1) in which the wire member Wb is pressed will be described. As illustrated in Fig. 19B, in transmission of drive force from the drive source M in the direction (direction Dc1) in which the wire member Wb is pressed to the holder Wc1, compression force Fc acts between the holder Wc1 and the rod Wc2.

In a case where the compression force Fc does not exceed a preset threshold value (second threshold value), the compression force Fc is received by the rod Wc2 via a contact portion between the protruding portion c13 of the holder Wc1 and the protrusion p2 of the rod Wc2. That is, the connection unit Wc according to the present exemplary embodiment is configured to maintain a state in which the protruding portion c13 is fitted in the recess portion c23, and transmit drive force from the drive source M to the wire member Wb, in a case where the compression force Fc at the second threshold value or less acts.

In a case where large compression force Fc exceeding the preset threshold value (second threshold value) acts, the protrusion p2 of the rod Wc2 is pressed by the protruding portion c13 of the holder Wc1 which causes inward elastic deformation in the protruding piece c22 of the rod Wc2. Consequently, the holder Wc1 and the rod Wc2 move relative to each other so that the protruding portion c13 moves over the protrusion p2, and the protruding portion c13 comes off from the recess portion c23. In a state (cutoff state) in which the protruding portion c13 has come off from the recess portion c23, even in a case where the holder Wc1 moves in the direction Dc1, the rod Wc2 does not move in the direction Dc1. That is, the connection unit Wc according to the present exemplary embodiment is configured to allow, in a case where the compression force Fc exceeding the second threshold value acts, the protruding portion c13 to come off from the recess portion c23, and cut off transmission of drive force from the drive source M to the wire member Wb (Fig. 20B).

The state switching of the connection unit Wc from the connected state to the cutoff state when overload occurs prevents, even in a case where the holder Wc1 is driven in the direction Dc1 by excessively-large drive force due to malfunction of the drive source M, for example, the excessively-large drive force from being transmitted to the wire member Wb. With this configuration, the bending portion 12 of the catheter 11 can be prevented from being bent by excessively-strong force.

As described above, the state of the connection unit Wc switches from the connected state to the cutoff state in accordance with the magnitude of the compression force Fc acting between the holder Wc1 and the rod Wc2. For this reason, also in a case where excessively-large external force is added to the wire member Wb by an object coming into contact with the catheter 11, the connection of the connection unit Wc is cut off. Even in a case where the proximal end of the wire member Wb is strongly pulled in the direction Dc1 by the wire member Wb receiving external force, a possibility that a member, such as the coupling unit 21c, is damaged can be reduced.

As illustrated in Fig. 19A, a clearance Ld that allows relative movements of the holder Wc1 and the rod Wc2 is left between a leading end position in the direction Dc2 of the rod Wc2 in the connected state and a wall surface c118 of a bottom portion of the space c11s of the holder Wc1. The length in the direction Dc of the clearance Ld is set in such a manner that the wall surface c118 does not come into contact with the rod Wc2 even in a case where the holder Wc1 further moves in the direction Dc1 by a predetermined distance after the protruding portion c13 has completely come off from the recess portion c23, with respect to a position X0 of the protruding portion c 13 in the connected state. With this clearance Ld, drive force in the direction Dc1 can be prevented from being transmitted due to the rod Wc2 being pressed by the wall surface c118 of the holder Wc1 even when the protruding portion c13 has come off from the recess portion c23.

The control unit 3 of the medical apparatus 1 may include a detection unit configured to detect that the connection of the connection unit Wc has been cut off, and stop the drive of each drive source M in a case where the cutoff of the connection of the connection unit Wc has been detected.

### (When Overload in Pulling Direction Occurs)

Next, a case where excessive force acts on the connection unit Wc in the direction (direction Dc2) in which the wire member Wb is pulled will be described. As illustrated in Fig. 19C, in transmission of drive force from the drive source M in the direction (direction Dc2) in which the wire member Wb is pulled to the holder Wc1, pulling force Ft acts between the holder Wc1 and the rod Wc2.

In a case where the pulling force Ft does not exceed a preset threshold value (first threshold value), the pulling force Ft is received by the rod Wc2 via a contact portion between the protruding portion c13 of the holder Wc1 and the protrusion p1 of the rod Wc2. That is, the connection unit Wc according to the present exemplary embodiment is configured to maintain, in a case where the pulling force Ft at the first threshold value or less acts, a state in which the protruding portion c13 is fitted in the recess portion c23, and transmit drive force from the drive source M to the wire member Wb.

In a case where large pulling force Ft exceeding the preset threshold value (first threshold value) acts, the protrusion p1 of the rod Wc2 is pressed by the protruding portion c13 of the holder Wc1, which causes inward elastic deformation in the protruding piece c22 of the rod Wc2. Consequently, the holder Wc1 and the rod Wc2 move relative to each other so that the protruding portion c13 moves over the protrusion p1, and the protruding portion c13 comes off from the recess portion c23. In a state (cutoff state) in which the protruding portion c13 has come off from the recess portion c23, even if the holder Wc1 moves in the direction Dc2, the rod Wc2 does not move in the direction Dc2. That is, the connection unit Wc according to the present exemplary embodiment is configured to allow, in a case where the pulling force Ft exceeding the second threshold value acts, the protruding portion c13 to come off from the recess portion c23, and cut off transmission of drive force from the drive source M to the wire member Wb (Fig. 20C).

The state switching of the connection unit Wc from the connected state to the cutoff state when overload occurs prevents, even in a case where the holder Wc1 is driven in the direction Dc2 by excessively-large drive force due to malfunction of the drive source M, for example, the excessively-large drive force from being transmitted to the wire member Wb. With this configuration, the bending portion 12 of the catheter 11 can be prevented from being bent by excessively-strong force.

As described above, the state of the connection unit Wc switches from the connected state to the cutoff state in accordance with the magnitude of the pulling force Ft acting between the holder Wc1 and the rod Wc2. For this reason, also in a case where excessively-large external force is added to the wire member Wb by an object coming into contact with the catheter 11, the connection of the connection unit Wc is cut off. With this configuration, even in a case where the proximal end of the wire member Wb is strongly pushed in the direction Dc2 due to the wire member Wb receiving external force, a possibility that a member, such as the coupling unit 21c, is damaged can be reduced.

In the configuration example according to the present exemplary embodiment, the threshold value (first threshold value) of the pulling force Ft is set to the same value as the threshold value (second threshold value) of the compression force Fc. This is a case for a configuration of cutting off, in a case where load exceeding a predetermined threshold value acts on the connection unit Wc, the connection between the drive source M and the wire member Wb irrespective the direction of the load.

### (Advantage of Present Exemplary Embodiment)

As described above, in the present exemplary embodiment, each member included in the connection unit Wc is a part of the catheter unit 100 that is a unit detachably attached to another unit (the base unit 200, etc.) of the medical apparatus 1. Thus, in a case where the catheter unit 100 is switched to the cutoff state due to load exceeding a threshold value acting on any connection unit Wc of the catheter unit 100, the catheter unit 100, the connection of which has been cut off, is detached, and a new catheter unit 100 can be attached. That is, the medical apparatus 1 is brought into a usable state again by a simple procedure of detaching the catheter unit 100 from the base unit 200 and replacing the catheter unit 100 with a new catheter unit 100.

According to the present exemplary embodiment, the connection between the drive source M and the wire member Wb can be cut off in response to both overload in the direction in which the wire member Wb is pressed and overload in the direction in which the wire member Wb is pulled.

An alternative configuration according to the present exemplary embodiment is that the connection between the drive source M and the wire member Wb is cut off by a combination of a detection element (strain gauge, etc.) and an electrically-controllable clutch (electromagnetic clutch, etc.) that releases the engagement of the clutch when overload is detected. In such a configuration, however, the apparatus is upsized and gets complicated because the detection element and the clutch are disposed. In contrast to this, in the present exemplary embodiment, a simple configuration in which the protruding portion c13 comes off from the recess portion c23 when overload occurs is implemented, which realizes a function of cutting off the connection between the drive source M and the wire member Wb.

### (Modified Example)

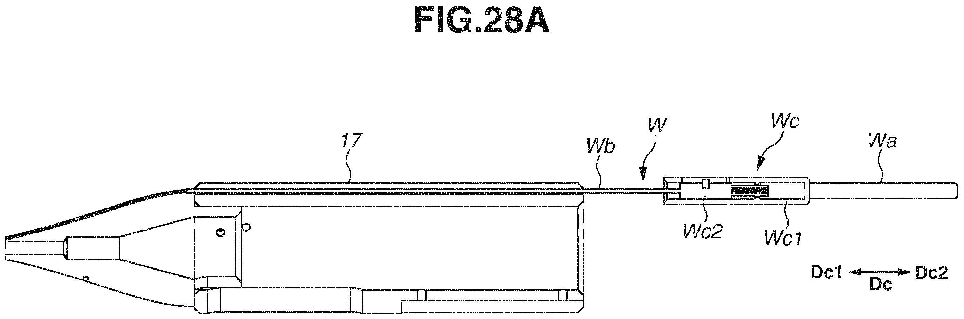

As a modified example of the first exemplary embodiment, a description will be given of a configuration in which the separation of the first member and the second member is restricted even in a case where the connection in the connection unit Wc is cut off with reference to Figs. 28A, 28B, 28C, 29, 30A, 30B, and 30C. Hereinafter, the components assigned the same reference numerals as those in the first exemplary embodiment have substantially the same configuration and function as those described in the first exemplary embodiment, and a difference from the first exemplary embodiment will be mainly described.

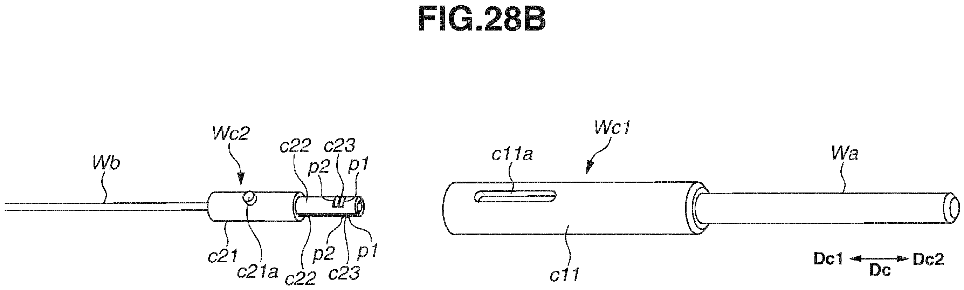

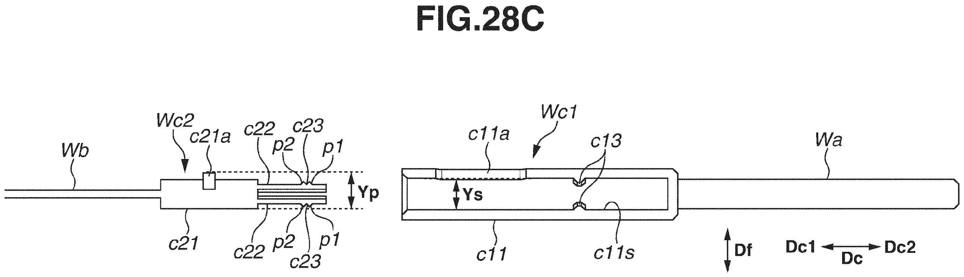

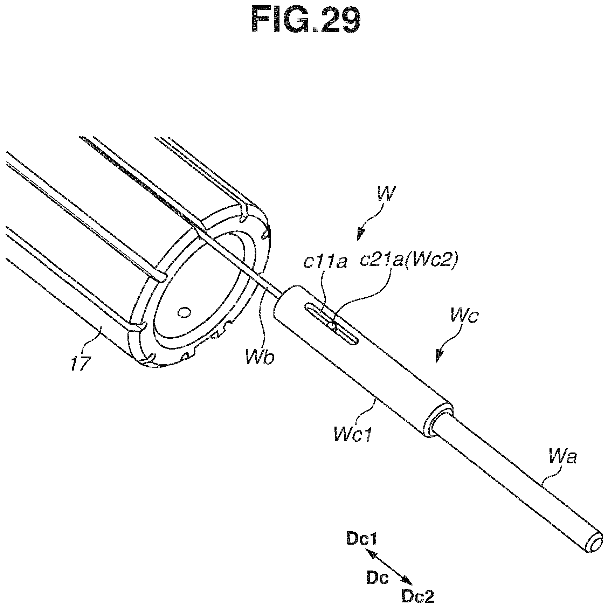

Fig. 28A is a schematic diagram illustrating the drive wire W according to this modified example that includes the connection unit Wc. Fig. 28B is a perspective view illustrating the holder Wc1 and the rod Wc2 that are included in the connection unit Wc. Fig. 28C is a cross-sectional view illustrating the holder Wc1 and the rod Wc2 that are included in the connection unit Wc. Fig. 28A illustrates a state (connected state) in which the rod Wc2 is held in the holder Wc1, and Figs. 28B and 28C illustrate a state (unattached state) in which the rod Wc2 is not attached to the holder Wc1. Fig. 29 is a perspective view illustrating the drive wire W according to this modified example.

As illustrated in Figs. 28A, 28B, 28C and 29, the rod Wc2 serving as the second member according to this modified example includes a pin c21a protruding outward from the outer circumference of the base portion c21. On the other hand, a slit c11a that accepts the pin c21a is formed in the cylindrical portion c11 of the holder Wc1 serving as the first member according to this modified example. The slit c11a is an elongate hole extending in the length direction (direction Dc) of the wire member Wb.

As illustrated in Fig. 29, the rod Wc2 is attached to the holder Wc1 in a state in which the pin c21a is fitted in the slit c11a. A configuration for attachment of the rod Wc2 including the pin c21a, to the holder Wc1 is, for example, that an elastic member, such as a coil spring, is disposed between a bottom portion of the pin 21a and the base portion c21, and the pin 21a is urged toward the leading end side (upside in Fig. 28C). In this case, in insertion of the rod Wc2 into the cylindrical portion c11, the pin c21a is pushed in while the elastic member is compressed, whereby the pin c21a can be fitted into the slit c11a through an opening portion of the cylindrical portion c11. A fitting method is not limited to this. For example, after the rod Wc2 to which the pin c21a is not attached is inserted into the cylindrical portion c11 of the holder Wc1, the pin c21a may be inserted from the outside of the cylindrical portion c11 via the slit c11a, and the pin c21a may be fixed using an adhesive agent or the like.

As illustrated in Fig. 28C, the leading end of the pin c21a protrudes outward from the inner circumference of the cylindrical portion c11 of the holder Wc1. In other words, at the position of the pin c21a, a height Yp of the rod Wc2 in a direction vertically intersecting with the length direction (direction Dc) of the wire member Wb is more than an inner diameter Ys of the cylindrical portion c11 of the holder Wc1 within a range in which the slit c11a is formed. In addition, the pin c21a is configured not to come off from the slit c11a at least at the threshold value (first threshold value and second threshold value) of load at which the protruding portion c13 comes off from the recess portion c23.

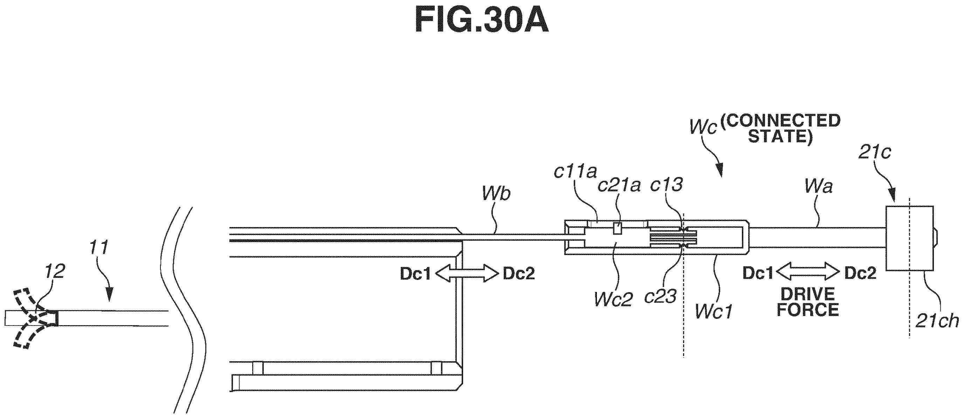

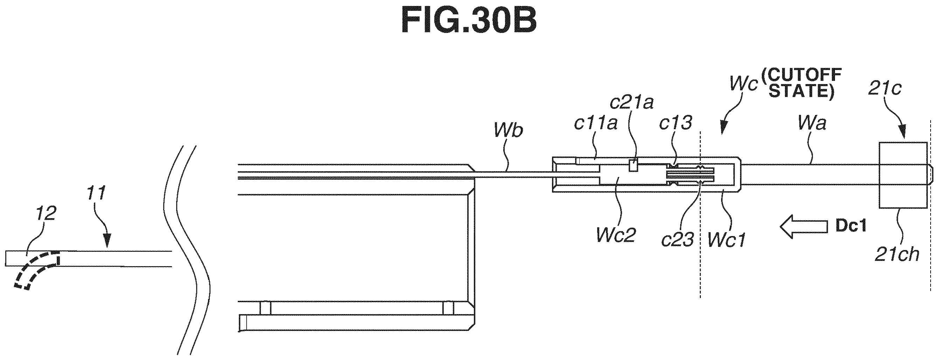

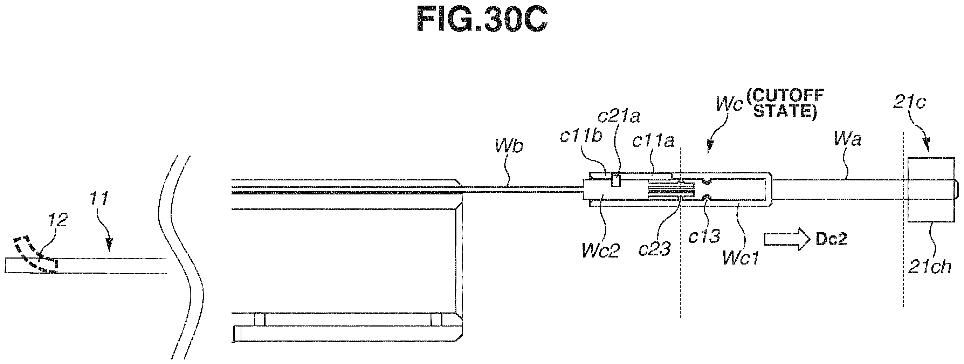

Movement of the connection unit Wc according to this modified example in the connected state and in the overload state will be described with reference to Figs. 30A, 30B, and 30C. Fig. 30A is a schematic diagram illustrating a cross-section of the connection unit Wc in the connected state. Fig. 30B is a schematic diagram illustrating a state of the connection unit Wc in a case where overload acts in a direction (direction Dc1) in which the wire member Wb is pressed. Fig. 30C is a schematic diagram illustrating a state of the connection unit Wc in a case where overload acts in a direction (direction Dc2) in which the wire member Wb is pulled.

As illustrated in Fig. 30A, similarly to the first exemplary embodiment, in the connected state of the connection unit Wc, the protruding portion c13 of the holder Wc1 is fitted in the recess portion c23 of the rod Wc2. For this reason, in the connected state, relative movements of the holder Wc1 and the rod Wc2 are restricted, and the holder Wc1 and the rod Wc2 integrally move in the direction Dc1 and the direction Dc2. In other words, drive force from the drive source M is transmitted to the wire member Wb via the connection unit Wc. In the connected state, the pin c21a of the rod Wc2 is at a position between end portions on both sides of the slit c11a of the holder Wc1, and is not in contact with the end portions.

As illustrated in Figs. 30B and 30C, in a case where overload in the direction Dc1 or the direction Dc2 acts on the connection unit Wc, similarly to the first exemplary embodiment, the protruding portion c13 comes off from the recess portion c23, and the transmission of drive force from the drive source M to the wire member Wb is cut off (cutoff state).

Even in a case where the connection unit Wc has been switched to the cutoff state, the pin c21a remains in a state of being fitted in the slit c11a. For this reason, in the cutoff state of the connection unit Wc, the holder Wc1 and the rod Wc2 are in a linked state in a state in which relative movements of the holder Wc1 and the rod Wc2 are allowed within a range in which the pin c21a slides within the slit c11a.

In a case where any connection unit Wc among the drive wires W has been switched to the cutoff state, the above-described operation unit 400is operated to detach the catheter unit 100 from the base unit 200. In the detachment, according to this modified example, even after the connection unit Wc has been switched to the cutoff state, the link between the holder Wc1 and the rod Wc2 is still maintained, and thus in the detachment of the catheter unit 100, the holder Wc1 and the held portion Wa are detached together with the rod Wc2. Specifically, the detachment of the catheter unit 100 in the direction Dc1 after the connection unit Wc has been switched to the cutoff state causes the holder Wc1 and the held portion Wa to be pulled out together with the rod Wc2 linked with the wire member Wb in a state in which the pin c21a is engaged with an end portion c11b on a side in the direction Dc1 of the slit c11a as illustrated in Fig. 30C.

That is, in a case where the connection unit Wc is formed as a part of the catheter unit 100, the first member can be prevented from being left behind within the base unit 200 when the catheter unit 100 is detached. This configuration makes a replacement operation of the catheter unit 10 easier, and the usability improves.

The pin c21a described in this modified example is an example of a stopper (retainer unit, separation restriction unit) that restricts separation between the first member and the second member in the cutoff state of the connection unit Wc. The pin c21a is not limited to this configuration. For example, a pin serving as a stopper may be disposed in the holder Wc1, and a slit that accepts the pin may be formed in the rod Wc2. In addition, the slit c11a is not limited to a through-hole and may have a groove shape. As a configuration of more reliably preventing dropout, a configuration in which a plurality of sets of pins c21a and slits c11a is disposed may be employed. The stopper is not limited to a pin and is only required to have a shape that can restrict the separation between the first member and the second member. For example, an annular protrusion portion like a snap ring may be used.

### (Another Modified Example)

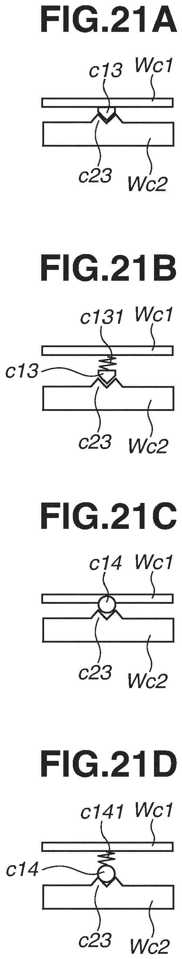

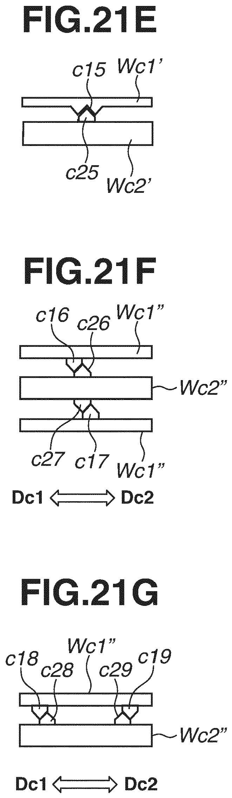

While, in the first exemplary embodiment, the connection unit Wc in which the protruding portion c13 is formed inside the holder Wc1 having a tubular shape, and the recess portions c23 are formed in the two protruding pieces c22 of the rod Wc2, another configuration that exerts a similar function may be employed. The modified example of the connection unit Wc is exemplified in Figs. 21A, 21B, 21C, 21D, 21E, 21F, and 21G.

Fig. 21A is a schematic diagram schematically illustrating a connection configuration of the holder Wc1 and the rod Wc2 exemplified in the first exemplary embodiment. As described above, the protruding portion c13 formed on the holder Wc1 fits into the recess portion c23 formed in the rod Wc2, so that the holder Wc1 and the rod Wc2 are connected to each other and integrally move in the direction Dc.

Fig. 21B illustrates a modified example in which the protruding portion c13 on the holder Wc1 is urged toward the recess portion c23 by an elastic member c131, such as a spring. In this case, change in a spring constant of the elastic member c131 and a deformation amount in the connected state can change the magnitude of load at which the protruding portion c13 comes off from the recess portion c23, i.e., a threshold value (first threshold value, second threshold value) of load at which the connection of the connection unit Wc is cut off.

Fig. 21C illustrates a modified example in which a protruding portion c14 on the holder Wc1 is a spherical or cylindrical rotational member rotatably supported by a support portion of the holder Wc1. In this case, the influence of friction on a contact surface, which is generated when the protruding portion c13 comes off from the recess portion c23, is small. Thus, the magnitude of load at which the protruding portion c13 comes off from the recess portion c23, i.e., a threshold value (first threshold value, second threshold value) of load at which the connection of the connection unit Wc is cut off can be set more accurately.

Fig. 21D illustrates a modified example in which the protruding portion c14 on the holder Wc1 is a rotational member, and the protruding portion c13 is urged toward the recess portion c23 by the elastic member c131 such as a spring. With this configuration, a threshold value (first threshold value, second threshold value) of load at which the connection of the connection unit Wc is cut off can be changed by a change in the elastic member c131, and the threshold value (first threshold value, second threshold value) of the load can be set more accurately.

Fig. 21E illustrates a modified example in which a recess portion c15 is formed in a first member Wc1', and a protruding portion c25 is formed on a second member Wc2'. This modified example can be implemented by swapping the holder Wc1 used as the first member in the first exemplary embodiment and the rod Wc2 used as the second member, for example. More specifically, it is sufficient that a member having the same shape as the holder Wc1 in the first exemplary embodiment is attached to the proximal end of the wire member Wb as the first member Wc1', and a member having the same shape as the rod Wc2 in the first exemplary embodiment is formed as the second member Wc2' integrally with the held portion Wa. The second member Wc2' and the held portion Wa may be separate members.

In addition, as another configuration of implementing the modified example illustrated in Fig. 21E, in place of the protruding portion c13, the recess portion c15 may be formed by two protrusions formed side by side in the direction Dc inside the cylindrical portion c11 of the holder Wc1 according to the first exemplary embodiment, and a protruding portion that fits into the recess portion may be formed on the protruding piece c22 of the rod Wc2. That is, in this case, similarly to the configurations illustrated in Figs. 21A to 21D, the holder Wc1 is formed integrally with the held portion Wa, and the rod Wc2 is attached to the proximal end of the wire member Wb. In this manner, with a configuration in which a protruding portion is formed on either one of the first member and the second member, and a recess portion that fits with the protruding portion is formed in the other one of the first member and the second member, a connection unit having a function similar to that in the first exemplary embodiment can be configured. Also, in second to the fourth exemplary embodiments to be described below, the arrangement of the protruding portion and the recess portion can be swapped, and it is only required to be a configuration in which a protruding portion is arranged on either one of the first member and the second member, and a recess portion that fits with the protruding portion is arranged in the other one of the first member and the second member.

Fig. 21F illustrates a modified example in which a protruding portion c16 and a protruding portion c17 are formed on a first member Wc1" at different positions, and a protruding portion c26 and a protruding portion c27 are formed on a second member Wc2" at different positions. In this modified example, a member on which the protruding portions c16 and c17 are formed in place of the protruding portion c13 in the holder Wc1 according to the first exemplary embodiment is formed integrally with the held portion Wa as the first member Wc1", for example. Then, a member on which the protruding portions c26 and c27 are formed in place of the protrusions p1 and p2 in the rod Wc2 according to the first exemplary embodiment is formed at the proximal end of the wire member Wb as the second member Wc2', whereby the configuration can be implemented. The first member Wc1' and the held portion Wa may be separate members.

The protruding portion c16 is an example of a first protruding portion, the protruding portion c17 is an example of a second protruding portion, the protruding portion c26 is an example of a third protruding portion that engages with the first protruding portion, and the protruding portion c27 is an example of a fourth protruding portion that engages with the second protruding portion. Also, in the second to the fourth exemplary embodiments to be described below, the first protruding portion and the second protruding portion in place of the "protruding portion" of the first member are formed, and the third protruding portion and the fourth protruding portion in place of the "recess portion" of the second member are formed, whereby a function similar to the combination of the protruding portion and the recess portion can be obtained.

In the above-described first exemplary embodiment and the modified examples, the connection unit Wc is formed by the protruding portion and the recess portion fitting with each other. The configuration is not limited to this configuration, and as exemplified in this modified example and the following modified example illustrated in Fig. 21F, the connection unit Wc may be formed by protruding portions and protruding portions engaging with each other at two different positions. In other words, the connection unit Wc does not depend on a specific shape of the first member and the second member, and it is sufficient that a shape formed on the first member and a shape formed on the second member are engaged in such a manner as to restrict relative movements in the direction Dc1 and the direction Dc2 of the first member and the second member. In the configuration, as long as a part at which a part of the second member comes into contact with a part of the first member in the direction Dc1, and a part at which a part of the second member comes into contact with a part of the first member in the direction Dc2 are disposed, relative movement in the direction Dc1 and the direction Dc2 of the first member and the second member is restricted. By employing a configuration in which the former contact part comes off by overload in the direction Dc1, and the latter contact part comes off by overload in the direction Dc2, the connection between the first member and the second member is cut off in accordance with the overload.

In the configuration illustrated in Fig. 21F, in a case where drive force acts in the direction Dc1, because the first member Wc1" moves in the direction Dc1, the first member Wc1" and the second member Wc2" integrally move due to the engagement between the protruding portion c17 and the protruding portion c27. In a case where overload occurs in the movement, the protruding portion c17 moves over the protruding portion c27, and the first member Wc1" further moves in the direction Dc1, which leads to cutting off the connection between the first member Wc1" and the second member Wc2". On the other hand, in a case where drive force acts in the direction Dc2, because the first member Wc1" moves in the direction Dc2, the first member Wc1" and the second member Wc2" integrally move due to the engagement between the protruding portion c16 and the protruding portion c26. In a case where overload occurs in the movement, the protruding portion c16 moves over the protruding portion c26, and the first member Wc1" further moves in the direction Dc2, which leads to cutting off the connection between the first member Wc1" and the second member Wc2".

In the configuration illustrated in Fig. 21F, the protruding portion c26 and the protruding portion c27 are formed at positions opposite to each other in the circumferential direction of the substantially columnar second member Wc2", but a positional relationship of the protruding portions is not limited to this. As illustrated in Fig. 21G, a configuration in which a protruding portion c28 and a protruding portion c29 are formed at different positions in the length direction of the second member Wc2" may be employed. Fig. 21G illustrates a modified example in which a protruding portion c18 and a protruding portion c19 are formed on the first member Wc1" at different positions, and the protruding portion c28 and the protruding portion c29 are formed on the second member Wc2" at different positions. The protruding portion c18 is an example of the first protruding portion, the protruding portion c19 is an example of the second protruding portion, the protruding portion c28 is an example of the third protruding portion that engages with the first protruding portion, and the protruding portion c29 is an example of the fourth protruding portion that engages with the second protruding portion.

In the configuration illustrated in Fig. 21G, in a case where drive force acts in the direction Dc1, because the first member Wc1" moves in the direction Dc1, the first member Wc1" and the second member Wc2" integrally move due to the engagement between the protruding portion c19 and the protruding portion c29. In a case where overload occurs in the movement, the protruding portion c19 moves over the protruding portion c29, and the first member Wc1" further moves in the direction Dc1, which leads to cutting off the connection between the first member Wc1" and the second member Wc2". On the other hand, in a case where drive force acts in the direction Dc2, because the first member Wc1" tries to move in the direction Dc2, the first member Wc1" and the second member Wc2" integrally move due to the engagement between the protruding portion c18 and the protruding portion c28. In a case where overload occurs in the movement, the protruding portion c18 moves over the protruding portion c28, and the first member Wc1" further moves in the direction Dc2, which leads to cutting off the connection between the first member Wc1" and the second member Wc2".

In this configuration, even in a case where overload occurs in the direction Dc1, and the engagement between the protruding portion c19 and the protruding portion c29 is released, the protruding portion c19 next engages with the protruding portion c28. Similarly, even in a case where overload occurs in the direction Dc2 and the engagement between the protruding portion c18 and the protruding portion c28 is released, the protruding portion c18 next engages with the protruding portion c29. That is, even in a case where breakaway functions and the transmission of drive force is temporarily cut off, the integrity of the first member Wc1" and the second member Wc2" is maintained, and is not switched to a state in which the first member Wc1" and the second member Wc2" are completely separated. With this configuration, for example, in a case where the catheter unit 100 is detached from the base unit 200 to extract the catheter 11 from the inside of the body of patient and replace the catheter 11 with new one, the following effect is obtained. Specifically, a possibility that the first member Wc1" linked with the held portion Wa remains on the base unit 200 side can be reduced. Thus, the usability improves.

Figs. 21F and 21G illustrate the configuration in which the first member Wc1" and the second member Wc2" are engaged with each other at two positions. Needless to say, the first member Wc1" and the second member Wc2" may be engaged with each other at three or more positions. The shape of the protruding portions is also not limited. Furthermore, an elastic member, such as a spring, as illustrated in Figs. 21B and 21D may be attached to a protruding portion.

Furthermore, as another modified example, a configuration in which, in a case where compression force or pulling force at a threshold value or more acts between the first member and the second member, a protruding portion or a recess portion plastically deforms (yields), and the protruding portion comes off from the recess portion, may be employed. With such irreversible deformation, it is also possible to implement a function of cutting off the connection between the drive source M and the wire member Wb when overload occurs.

### [Second Exemplary Embodiment]

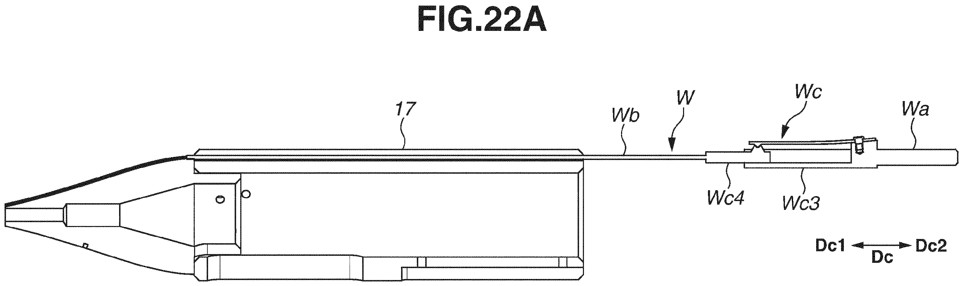

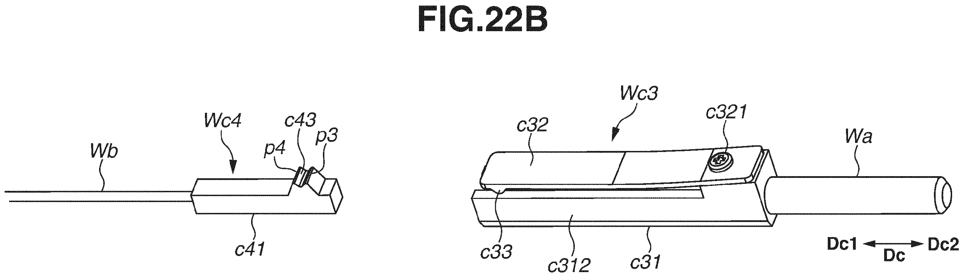

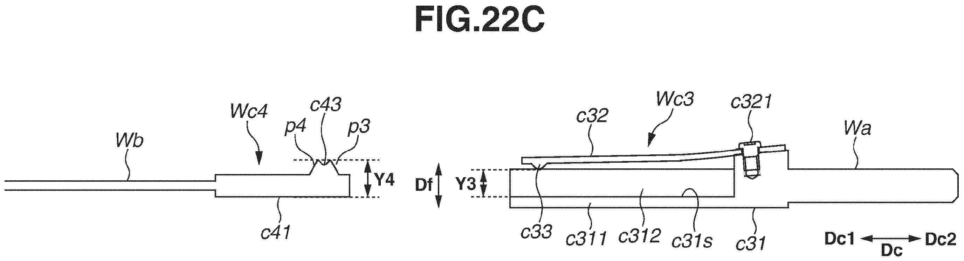

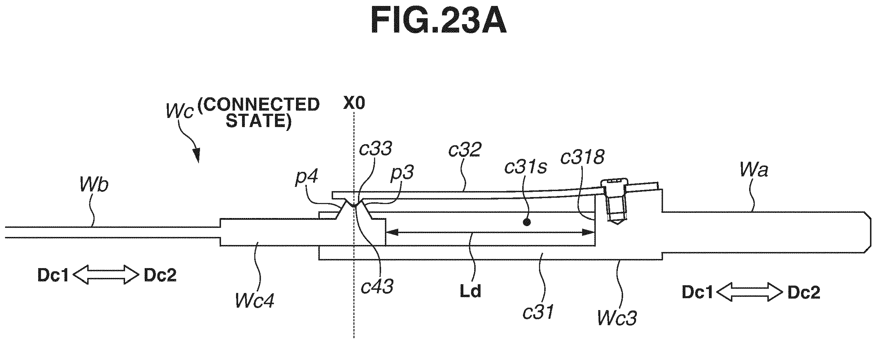

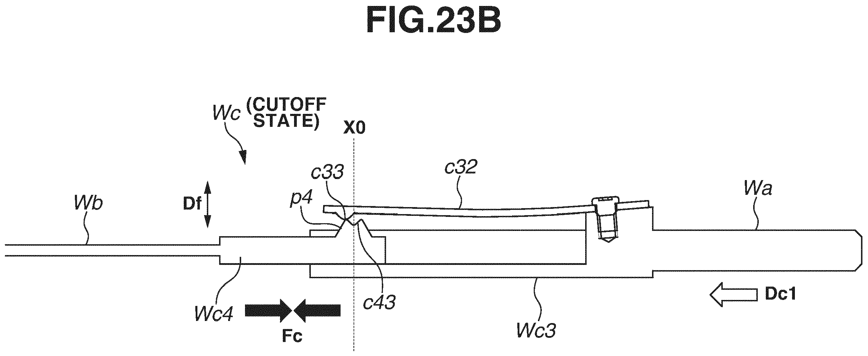

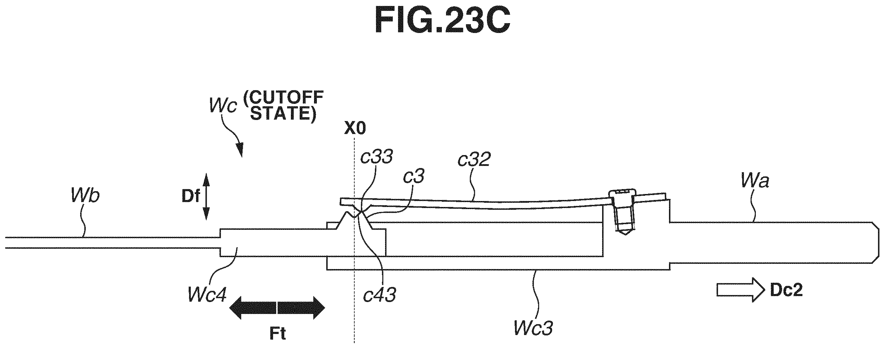

A medical apparatus according to the second exemplary embodiment will be described with reference to Figs. 22A, 22B, 22C, 23A, 23B, and 23C. The present exemplary embodiment differs from the first exemplary embodiment in a configuration of a connection unit Wc that connects the drive source M and the wire member Wb. The components assigned the same reference numerals as those in the first exemplary embodiment have substantially the same configuration and function as those described in the first exemplary embodiment, and a difference from the first exemplary embodiment will be mainly described.

Fig. 22A is a schematic diagram illustrating the drive wire W according to the present exemplary embodiment that includes the connection unit Wc. Fig. 22B is a perspective view illustrating a holder Wc3 and a rod Wc4 that are included in the connection unit Wc. Fig. 22C is a cross-sectional view illustrating the holder Wc3 and the rod Wc4 that are included in the connection unit Wc. Fig. 22A illustrates a state (connected state) in which the rod Wc4 is held in the holder Wc3, and Figs. 22B and 22C illustrate a state (cutoff state) in which the rod Wc4 is not held in the holder Wc3.

In the following description, the connection unit Wc disposed in the drive wire W, which is an arbitrary one of the first to ninth drive wires (W 11 to W33) included in the catheter unit 100 according to the present exemplary embodiment, will be described. In the configuration example according to the present exemplary embodiment, the connection unit Wc having substantially the same configuration as the configuration to be described below is disposed in each of the nine drive wires (W11 to W33).

As illustrated in Fig. 22A, the connection unit Wc includes the holder Wc3 serving as a first member (engagement member, holding member) that is connected to the drive source M, and the rod Wc4 serving as a second member (engaged member, held member) that is connected to the wire member Wb of the drive wire W. In the present exemplary embodiment, at least part of the holder Wc3 is a member formed integrally with the held portion Wa of the drive wire W. Accordingly, the holder Wc3 is connected to the drive source M via the held portion Wa and the coupling unit 21c (Figs. 6A, 6B, and 6C). The holder Wc3 and the held portion Wa may be formed as separate members. Meanwhile, the rod Wc4 is fixed to the proximal end of the wire member Wb using an arbitrary fixing method, such as pressure bonding (swaging) or adhesive bonding.

As illustrated in Figs. 22B and 22C, the holder Wc3 includes a main body portion (base portion) c31 formed integrally with the held portion Wa, and a plate spring c32 attached to the main body portion c31 using a fixing unit, such as a screw c321. The main body portion c31 has a groove shape formed with a bottom portion c311 and side wall portions c312 and has a cross-sectional shape resembling a U-shape with right-angled corners (rectangular shape with one side opened) when viewed in the direction Dc. The plate spring c32 is attached in such a manner as to cover an opening portion of the main body portion c31 when viewed in the direction Df, and has a length in the direction Dc1 from the screw c321. Thus, a space c31s that accepts the rod Wc4, which will be described below, is formed between the bottom portion c311 and the side wall portion c312 of the main body portion c31, and the plate spring c32. The main body portion c31 is disposed next to the held portion Wa in the direction Dc, and the held portion Wa has a length in the Dc2 direction from the end portion in the direction Dc2 of the main body portion c31.

The holder Wc3 also includes a protruding portion c33 at the leading end of the plate spring c32. The protruding portion c33 is disposed in such a manner as to protrude from a leading end portion in the direction Dc2 of the plate spring c32 toward the space c31s between the main body portion c31 and the plate spring c32. In other words, the protruding portion c33 is an example of a protruding portion protruding in a direction intersecting with the length direction (direction Dc) of the wire member Wb in the connection unit Wc.

In a case where the plate spring c32 receives external force, the plate spring c32 mainly deforms in the direction Df. That is, the plate spring c32 functions as a deformation element deformable in such a manner as to allow the protruding portion c33 to come off from a recess portion c43 to be described below.

When viewed in the direction Dc, the protruding portion c33 is formed at a position corresponding to the recess portion c43 in the rod Wc4 to be described below. In the present exemplary embodiment, the protruding portion c33 is formed at one part in such a manner as to correspond to the recess portion c43 formed at one part. The protruding portion c33 is only required to be formed at a position at which the protruding portion c33 can engage with the recess portion c43, and thus, for example, a plurality of plate springs c32 each including the protruding portion c33 may be formed, and a plurality of recess portions c43 corresponding thereto may be formed in the rod Wc4.

As illustrated in Figs. 22B and 22C, the rod Wc4 includes a base portion c41 that has a substantially quadrangular prism shape and has a length in the direction Dc2 from the proximal end of the wire member Wb, and the recess portion c43 formed on a side surface (surface facing the plate spring c32) in the direction Df of the base portion c41. The recess portion c43 is formed between protrusions p3 and p4 protruding in the direction Df from the base portion c41. The protrusions p3 and p4 are formed side by side in the direction Dc.

The rod Wc4 is inserted in the direction Dc2 toward the space c31s between the main body portion c31 and the plate spring c32 of the holder Wc3 and is attached to the holder Wc3 by being pressed in until the protruding portion c33 of the holder Wc3 fits into the recess portion c43 of the rod Wc4. In the present exemplary embodiment, a part of the rod Wc4 in the direction Dc2 is an insertion portion that is inserted into the space c31s in the holder Wc3.

Next, movement of the connection unit Wc in the connected state and in an overload state will be described with reference to Figs. 23A, 23B, and 23C. Fig. 23A is a schematic diagram illustrating a cross-section of the connection unit Wc in the connected state. Fig. 23B is a schematic diagram illustrating a state of the connection unit Wc in a case where overload acts in a direction (direction Dc1) in which the wire member Wb is pressed. Fig. 23C is a schematic diagram illustrating a state of the connection unit Wc in a case where overload acts in a direction (direction Dc2) in which the wire member Wb is pulled.

As illustrated in Fig. 23A, in the connected state of the connection unit Wc, the protruding portion c33 of the holder Wc3 is fitted in the recess portion c43 of the rod Wc4. Specifically, the protruding portion c33 is held between the protrusions p3 and p4 in the direction Dc. Here, as illustrated in Fig. 22C, a distance in the direction Df from the bottom surface of the base portion c41 to the top of the protrusion p3 or p4 in a state in which the rod Wc4 is not attached to the holder Wc3 is a distance Y4. The distance Y4 is longer than a distance Y3 in the direction Df from the protruding portion c33 of the plate spring c32 of the holder Wc3 to the bottom portion c311 of the main body portion c31. Thus, in the connected state illustrated in Fig. 23A, the protruding portion c33 of the holder Wc3 and a part (protrusion p3 or p4) of the rod Wc4 are in a positional relationship of interfering with each other in the direction Dc. In other words, when viewed in the length direction (direction Dc) of the wire member in the connected state, at least part of the protruding portion formed on one of the first member or the second member overlaps the recess portion formed on the other one of the first member or the second member.

With such a configuration, in a case where the connection unit Wc is in the connected state, relative movements of the holder Wc3 and the rod Wc4 are restricted, and the holder Wc3 and the rod Wc4 integrally move in the direction Dc1 and the direction Dc2. In other words, in a case where the connection unit Wc is in the connected state, drive force from the drive source M is transmitted to the wire member Wb via the connection unit Wc.

In the connected state illustrated in Fig. 23A, the protruding portion c33 of the holder Wc3 is desirably configured to fit into the recess portion c43 of the rod Wc4 in a backlash-reduced state. In the present exemplary embodiment, the protruding portion 33 is formed at the leading end portion of the plate spring c32 having elasticity, and by the elastic force of the plate spring c32 due to the plate spring c32 slightly-elastically-deformed in the direction Df (upward in Fig. 23A) in the connected state, the protruding portion c33 is pressed against the protrusions p3 and p4 on both sides. In other words, with a mechanism (snap-fit mechanism) that uses a deformation element made of elastic material, the connection unit Wc according to the present exemplary embodiment connects the holder Wc3 serving as the first member and the rod Wc4 serving as the second member.

In this manner, with the configuration in which the holder Wc3 and the rod Wc4 are engaged with each other in the backlash-reduced state, responsivity of the bending portion 12 of the catheter 11 to the drive from the drive source M can be improved.

### (When Overload in Pressing Direction Occurs)

Next, a description will be given on a case where excessive force acts on the connection unit Wc in the direction (direction Dc1) in which the wire member Wb is pressed. As illustrated in Fig. 23B, in a case where drive force in the direction (direction Dc1) in which the wire member Wb is pressed is transmitted from the drive source M to the holder Wc3, compression force Fc acts between the holder Wc3 and the rod Wc4.

In a case where the compression force Fc does not exceed a preset threshold value (second threshold value), the compression force Fc is received by the rod Wc4 via a contact portion between the protruding portion c33 of the holder Wc3 and the protrusion p4 of the rod Wc4. That is, the connection unit Wc according to the present exemplary embodiment is configured to maintain, in a case where the compression force Fc at the second threshold value or less acts, a state in which the protruding portion c33 is fitted in the recess portion c43, and transmit drive force from the drive source M to the wire member Wb.

In a case where large compression force Fc exceeding the preset threshold value (second threshold value) acts, the plate spring c32 elastically deforms in such a manner that the protruding portion c33 moves over the protrusion p4 of the rod Wc4. Consequently, the holder Wc3 and the rod Wc4 move relative to each other while the protruding portion c33 moves over the protrusion p4, and the protruding portion c33 comes off from the recess portion c43. In a state (cutoff state) in which the protruding portion c33 has come off from the recess portion c43, even in a case where the holder Wc3 moves in the direction Dc1, the rod Wc4 does not move in the direction Dc1. That is, the connection unit Wc according to the present exemplary embodiment is configured to allow, in a case where the compression force Fc exceeding the second threshold value acts, the protruding portion c33 to come off from the recess portion c43, and cut off the transmission of drive force from the drive source M to the wire member Wb (Fig. 23B).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As illustrated in Fig. 23A, a clearance Ld that allows relative movements of the holder Wc3 and the rod Wc4 is left between a leading end position in the direction Dc2 of the rod Wc4 in the connected state and a wall surface c318 serving as a bottom portion of the space c31s of the holder Wc3. The length in the direction Dc of the clearance Ld is set in such a manner that the wall surface c318 does not come into contact with the rod Wc4 even in a case where the holder Wc3 further moves in the direction Dc1 by a predetermined distance after the protruding portion c33 has completely come off from the recess portion c43, with respect to a position X0 of the protruding portion c33 in the connected state. With a clearance Ld, drive force in the direction Dc1 is prevented from being transmitted by the rod Wc4 being pressed by the wall surface c318 of the holder Wc3 although the protruding portion c33 has come off from the recess portion c43.

### (When Overload in Pulling Direction Occurs)

Next, a case where excessive force acts on the connection unit Wc in the direction (direction Dc2) in which the wire member Wb is pulled will be described. As illustrated in Fig. 23C, in transmission of drive force from the drive source M to the holder Wc3 in the direction (direction Dc2) in which the wire member Wb is pulled, pulling force Ft acts between the holder Wc3 and the rod Wc4.

In a case where the pulling force Ft does not exceed a preset threshold value (first threshold value), the pulling force Ft is received by the rod Wc4 via a contact portion between the protruding portion c33 of the holder Wc3 and the protrusion p3 of the rod Wc4. That is, the connection unit Wc according to the present exemplary embodiment is configured to maintain, in a case where the pulling force Ft at the first threshold value or less acts, a state in which the protruding portion c33 is fitted in the recess portion c43, and drive force from the drive source M is transmitted to the wire member Wb.

In a case where large pulling force Ft exceeding the preset threshold value (first threshold value) acts, the plate spring c32 elastically deforms in such a manner that the protruding portion c33 moves over the protrusion p3 of the rod Wc4. Consequently, the holder Wc3 and the rod Wc4 move relative to each other while the protruding portion c33 moves over the protrusion p3, and the protruding portion c33 comes off from the recess portion c43. In a state (cutoff state) in which the protruding portion c33 has come off from the recess portion c43, even in a case where the holder Wc3 moves in the direction Dc2, the rod Wc4 does not move in the direction Dc2. That is, the connection unit Wc according to the present exemplary embodiment is configured to allow, in a case where the pulling force Ft exceeding the second threshold value acts, the protruding portion c33 to come off from the recess portion c43, and cut off transmission of drive force from the drive source M to the wire member Wb (Fig. 23C).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As described above, also according to the present exemplary embodiment, each member included in the connection unit Wc is a part of the catheter unit 100 that is a unit detachably attached to another unit (the base unit 200, etc.) of the medical apparatus 1. Thus, the medical apparatus 1 can be brought into a usable state again by a simple method of detaching the catheter unit 100 from the base unit 200 after cutting off the connection between the drive source M and the wire member Wb, and replacing the catheter unit 100 with a new catheter unit 100.

### [Third Exemplary Embodiment]

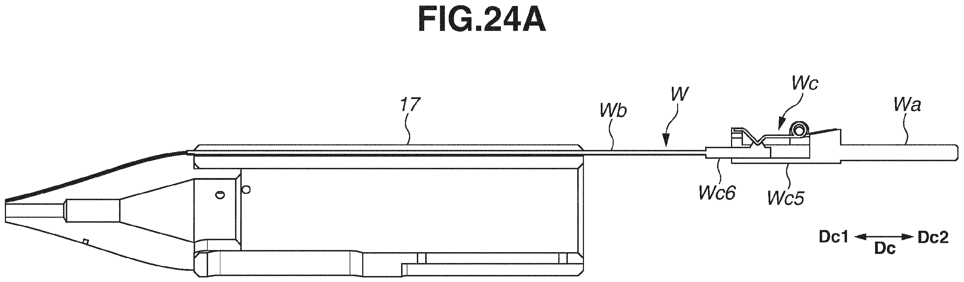

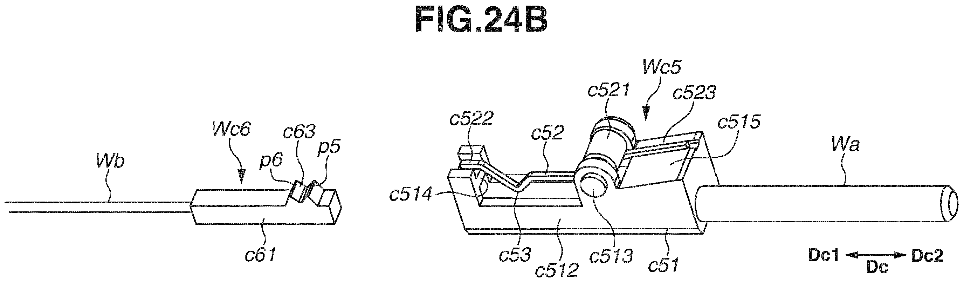

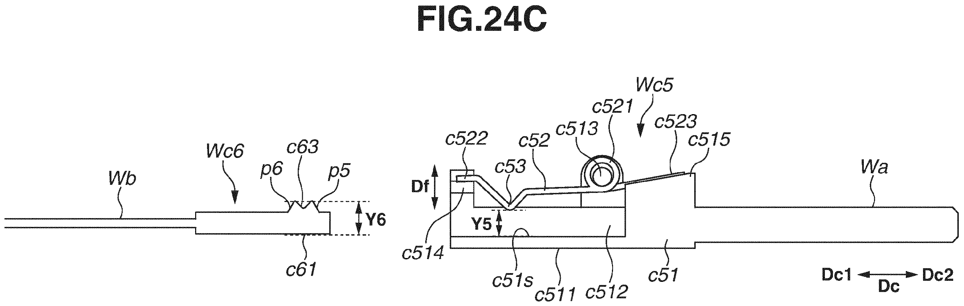

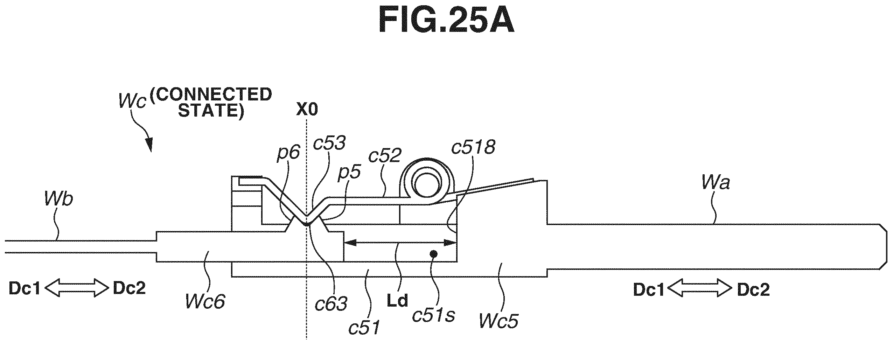

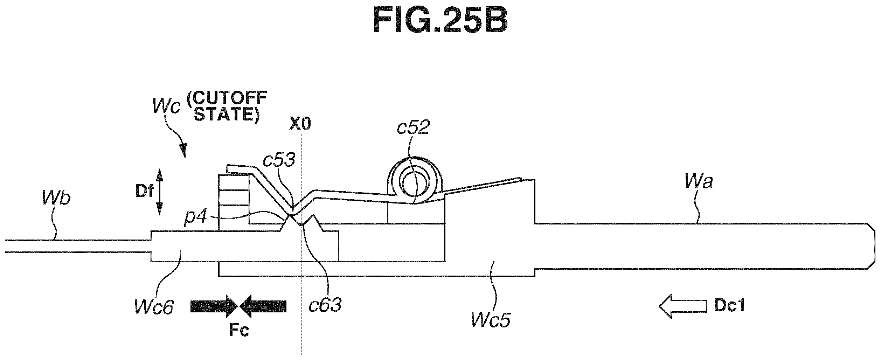

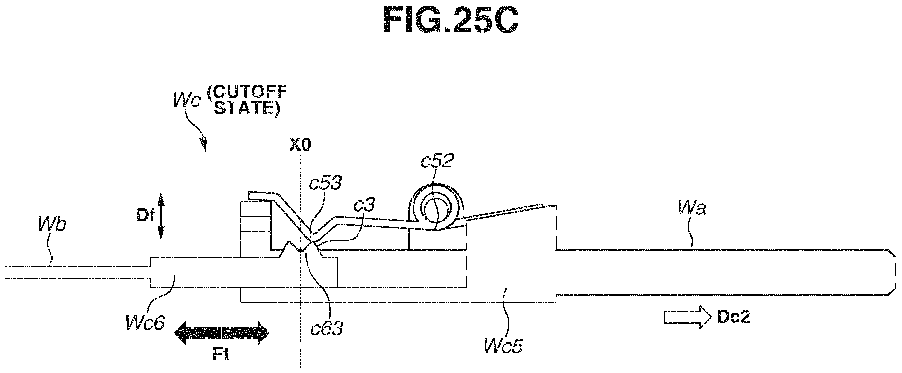

A medical apparatus according to the third exemplary embodiment will be described with reference to Figs. 24A, 24B, 24C, 25A, 25B, and 25C. The present exemplary embodiment differs from the first exemplary embodiment in a configuration of a connection unit Wc that connects the drive source M and the wire member Wb. Hereinafter, the components assigned the same reference numerals as those in the first exemplary embodiment have substantially the same configuration and function as those described in the first exemplary embodiment, and a difference from the first exemplary embodiment will be mainly described.

Fig. 24A is a schematic diagram illustrating the drive wire W according to the present exemplary embodiment that includes the connection unit Wc. Fig. 24B is a perspective view illustrating a holder Wc5 and a rod Wc6 that are included in the connection unit Wc. Fig. 24C is a cross-sectional view illustrating the holder Wc5 and the rod Wc6 that are included in the connection unit Wc. Fig. 24A illustrates a state (connected state) in which the rod Wc6 is held in the holder Wc5, and Figs. 24B and 24C illustrate a state (cutoff state) in which the rod Wc6 is not held in the holder Wc5.

In the following description, the connection unit Wc disposed in the drive wire W, which is an arbitrary one of the first to ninth drive wires (W 11 to W33) included in the catheter unit 100 according to the present exemplary embodiment, will be described. In the configuration example according to the present exemplary embodiment, the connection unit Wc having substantially the same configuration as the configuration to be described below is arranged in each of the nine drive wires (W11 to W33).

As illustrated in Fig. 24A, the connection unit Wc includes the holder Wc5 serving as a first member (engagement member, holding member) that is connected to the drive source M, and the rod Wc6 serving as a second member (engaged member, held member) that is connected to the wire member Wb of the drive wire W. In the present exemplary embodiment, at least part of the holder Wc5 is a member formed integrally with the held portion Wa of the drive wire W. Thus, the holder Wc5 is connected to the drive source M via the held portion Wa and the coupling unit 21c (Figs. 6A, 6B, and 6C). The holder Wc5 and the held portion Wa may be formed as separate members. Meanwhile, the rod Wc6 is fixed to the proximal end of the wire member Wb using an arbitrary fixing method, such as pressure bonding (swaging) or adhesive bonding.

As illustrated in Figs. 24B and 24C, the holder Wc5 includes a main body portion (base portion) c51 formed integrally with the held portion Wa, and a torsion spring (torsion coil spring) c52 supported by the main body portion c31. The main body portion c51 has a groove shape formed with a bottom portion c511 and side wall portions c512 and has a cross-sectional shape resembling a U-shape with right-angled corners (rectangular shape with one side opened) when viewed in the direction Dc. The torsion spring c52 is attached to an opened portion in the Df direction of the main body portion c51. Thus, a space c51s that accepts the rod Wc6, which will be described below, is formed between the bottom portion c511 and the side wall portions c512 of the main body portion c51, and the torsion spring c52. The main body portion c51 is disposed next to the held portion Wa in the direction Dc, and the held portion Wa has a length in the direction Dc2 from the end portion in the direction Dc2 of the main body portion c51.

The torsion spring c52 includes a coil portion c521 supported by a shaft portion c513 attached to the main body portion c51, an arm portion c522 lengthened from the coil portion c521 in the direction Dc1, and an arm portion c523 extending from the coil portion c521 in the direction Dc2. The arm portion c522 on a side in the direction Dc1 is a free end that is supported by a support portion 514 of the main body portion c51, and is elastically-deformable in such a manner as to move away from the support portion 514 by being pressed by the rod Wc6. The arm portion c523 on a side in the direction Dc2 is a fixed end that is supported by a support portion c515 of the main body portion c51 and remains in contact with the support portion c515 even in a case where the other arm portion c522 is pressed by the rod Wc6.

The holder Wc5 includes a protruding portion c53 having a bending shape formed on the arm portion c523 of the torsion spring c52 on the free end side. The protruding portion c53 protrudes, between the leading end of the arm portion c523 and the coil portion c521, toward the space c51s which is between the torsion spring c52 and the main body portion c51. In other words, the protruding portion c53 is an example of a protruding portion protruding in a direction intersecting with the length direction (direction Dc) of the wire member Wb in the connection unit Wc.

In a case where the torsion spring c52 receives external force, the torsion spring c52 mainly deforms in the direction Df. That is, the torsion spring c52 functions as a deformation element deformable in such a manner as to allow the protruding portion c53 to come off from a recess portion c63 to be described below.

When viewed in the direction Dc, the protruding portion c53 is arranged at a position corresponding to the recess portion c63 to be described below in the rod Wc6. In the present exemplary embodiment, the protruding portion c53 is formed at one part in such a manner as to correspond to the recess portion c63 formed at one part. The protruding portion c53 is only required to be formed at a position at which the protruding portion c53 can engage with the recess portion c63, and thus, for example, a plurality of torsion springs c52 each including the protruding portion c53 may be formed, and a plurality of recess portions c63 corresponding thereto may be formed in the rod Wc6.

As illustrated in Figs. 25B and 25C, the rod Wc6 includes a base portion c61 that has a substantially quadrangular prism shape and has a length in the direction Dc2 from the proximal end of the wire member Wb, and the recess portion c63 formed on a side surface (surface facing the torsion spring c52) in the direction Df of the base portion c61. The recess portion c63 is formed between protrusions p5 and p6 protruding in the direction Df from the base portion c61. The protrusions p5 and p6 are formed side by side in the direction Dc.

The rod Wc6 is inserted in the direction Dc2 toward the space c51s between the main body portion c51 and the torsion spring c52 of the holder Wc5 and is attached to the holder Wc5 by being pressed in until the protruding portion c53 of the holder Wc5 fits into the recess portion c63 of the rod Wc6. In the present exemplary embodiment, a part of the rod Wc6 on a side in the direction Dc2 is an insertion portion to be inserted into the space c51s in the holder Wc5.

Next, movement of the connection unit Wc in the connected state and in an overload state will be described with reference to Figs. 25A, 25B, and 25C. Fig. 25A is a schematic diagram illustrating a cross-section of the connection unit Wc in the connected state. Fig. 25C is a schematic diagram illustrating a state of the connection unit Wc in a case where overload acts in a direction (direction Dc1) in which the wire member Wb is pressed. Fig. 25C is a schematic diagram illustrating a state of the connection unit Wc in a case where overload acts in a direction (direction Dc2) in which the wire member Wb is pulled.

As illustrated in Fig. 25A, in the connected state of the connection unit Wc, the protruding portion c53 of the holder Wc5 is fitted in the recess portion c63 of the rod Wc6. Specifically, the protruding portion c53 is held between the protrusions p5 and p6 in the direction Dc. As illustrated in Fig. 24C, a distance in the direction Df from the bottom surface of the base portion c61 to the top of the protrusion p5 or p6 in a state in which the rod Wc6 is not attached to the holder Wc5 is a distance Y6. The distance Y6 is longer than a distance Y5 in the direction Df from the protruding portion c53 of the torsion spring c52 of the holder Wc5 to the bottom portion c511 of the main body portion c51. Thus, in the connected state illustrated in Fig. 25A, the protruding portion c53 of the holder Wc5 and a part (protrusion p5 or p6) of the rod Wc6 are in a positional relationship of interfering with each other in the direction Dc. In other words, when viewed in the length direction (direction Dc) of the wire member in the connected state, at least part of the protruding portion formed on one of the first member or the second member overlaps the recess portion formed on the other one of the first member or the second member.

With such a configuration, in a case where the connection unit Wc is in the connected state, relative movement of the holder Wc5 and the rod Wc6 are restricted, and the holder Wc5 and the rod Wc6 integrally move in the direction Dc1 and the direction Dc2. In other words, in a case where the connection unit Wc is in the connected state, drive force from the drive source M is transmitted to the wire member Wb via the connection unit Wc.

In the connected state illustrated in Fig. 25A, the protruding portion c53 of the holder Wc5 is desirably configured to fit into the recess portion c63 of the rod Wc6 in a backlash-reduced state. In the present exemplary embodiment, the protruding portion c53 is formed in the arm portion c522 of the torsion spring c52 having elasticity, and the torsion spring c52 is in a slightly-elastically-deformed state in such a manner that the arm portion c522 is lifted up in the direction Df (upward in Fig. 25A) in the connected state. For this reason, by the elastic force of the torsion spring c52, the protruding portion c53 is pressed against the protrusions p5 and p6 on both sides. In other words, the connection unit Wc according to the present exemplary embodiment connects the holder Wc5 serving as the first member and the rod Wc6 serving as the second member to each other, by a mechanism (snap-fit mechanism) that uses a deformation element made of elastic material.

In this manner, with the configuration of engaging the holder Wc5 and the rod Wc6 with each other in the backlash-reduced state, responsivity of the bending portion 12 of the catheter 11 to the drive caused by the drive source M can be improved.

### (When Overload in Pressing Direction Occurs)

Next, a case where excessive force acts on the connection unit Wc in the direction (direction Dc1) in which the wire member Wb is pressed will be described. As illustrated in Fig. 25B, in transmission of drive force from the drive source M to the holder Wc5 in the direction (direction Dc1) in which the wire member Wb is pressed, compression force Fc acts between the holder Wc5 and the rod Wc6.

In a case where the compression force Fc does not exceed a preset threshold value (second threshold value), the compression force Fc is received by the rod Wc6 via a contact portion between the protruding portion c53 of the holder Wc5 and the protrusion p6 of the rod Wc6. That is, the connection unit Wc according to the present exemplary embodiment is configured to maintain, in a case where the compression force Fc at the second threshold value or less acts, a state in which the protruding portion c53 is fitted in the recess portion c63, and drive force from the drive source M is transmitted to the wire member Wb.

In a case where large compression force Fc exceeding the preset threshold value (second threshold value) acts, the torsion spring c52 elastically deforms in such a manner that the protruding portion c53 moves over the protrusion p6 of the rod Wc6. Consequently, the holder Wc5 and the rod Wc6 move relative to each other while the protruding portion c53 moves over the protrusion p6, and the protruding portion c53 comes off from the recess portion c63. In a state (cutoff state) in which the protruding portion c53 has come off from the recess portion c63, even in a case where the holder Wc5 moves in the direction Dc1, the rod Wc6 does not move in the direction Dc1. That is, the connection unit Wc according to the present exemplary embodiment is configured to allow, in a case where the compression force Fc exceeding the second threshold value acts, the protruding portion c53 to come off from the recess portion c63 and cut off transmission of drive force from the drive source M to the wire member Wb (Fig. 25B).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As illustrated in Fig. 25A, a clearance Ld that allows relative movements of the holder Wc5 and the rod Wc6 is left between a leading end position in the direction Dc2 of the rod Wc6 in the connected state and a wall surface c518 serving as a bottom portion of the space c51s of the holder Wc5. The length in the direction Dc of the clearance Ld is set in such a manner that the wall surface c518 does not come into contact with the rod Wc6 even in a case where the holder Wc5 further moves in the direction Dc1 by a predetermined distance after the protruding portion c53 has completely come off from the recess portion c63, with respect to a position X0 of the protruding portion c53 in the connected state. With such a clearance Ld, drive force in the direction Dc1 is prevented from being transmitted by the rod Wc6 being pressed by the wall surface c518 of the holder Wc5 even though the protruding portion c53 has come off from the recess portion c63.

### (When Overload in Pulling Direction Occurs)

Next, a case where excessive force acts on the connection unit Wc in the direction (direction Dc2) in which the wire member Wb is pulled will be described. As illustrated in Fig. 25C, in transmission of drive force from the drive source M to the holder Wc5 in the direction (direction Dc2) in which the wire member Wb is pulled, pulling force Ft acts between the holder Wc5 and the rod Wc6.

In a case where the pulling force Ft does not exceed a preset threshold value (first threshold value), the pulling force Ft is received by the rod Wc6 via a contact portion between the protruding portion c53 of the holder Wc5 and the protrusion p5 of the rod Wc6. That is, the connection unit Wc according to the present exemplary embodiment is configured to maintain, in a case where the pulling force Ft at the first threshold value or less acts, a state in which the protruding portion c53 is fitted in the recess portion c63, and transmit drive force from the drive source M to the wire member Wb.

In a case where large pulling force Ft exceeding the preset threshold value (first threshold value) acts, the torsion spring c52 elastically deforms in such a manner that the protruding portion c53 moves over the protrusion p5 of the rod Wc6. Consequently, the holder Wc5 and the rod Wc6 move relative to each other while the protruding portion c53 moves over the protrusion p5, and the protruding portion c53 comes off from the recess portion c63. In a state (cutoff state) in which the protruding portion c53 has come off from the recess portion c63, even in a case where the holder Wc5 moves in the direction Dc2, the rod Wc6 does not move in the direction Dc2. That is, the connection unit Wc according to the present exemplary embodiment is configured to allow, in a case where the pulling force Ft exceeding the second threshold value acts, the protruding portion c53 to come off from the recess portion c63, and cut off transmission of drive force from the drive source M to the wire member Wb (Fig. 25C).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As described above, also according to the present exemplary embodiment, each member included in the connection unit Wc is a part of the catheter unit 100 that is a unit detachably attached to another unit (the base unit 200, etc.) of the medical apparatus 1. Thus, the medical apparatus 1 can be brought into a usable state again by a simple method of detaching the catheter unit 100 from the base unit 200 after cutting off the connection between the drive source M and the wire member Wb, and replacing the catheter unit 100 with a new catheter unit 100.

### [Fourth Exemplary Embodiment]

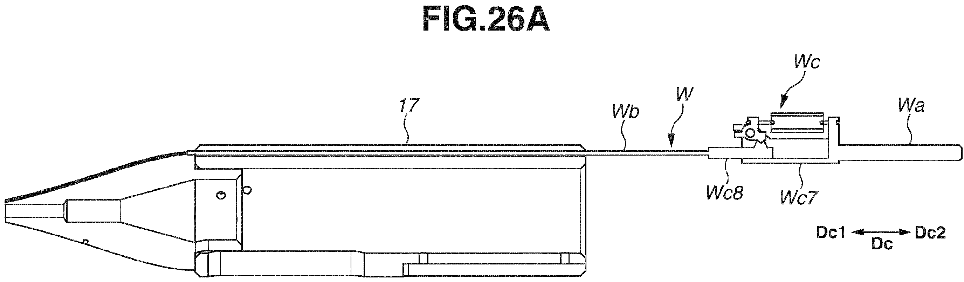

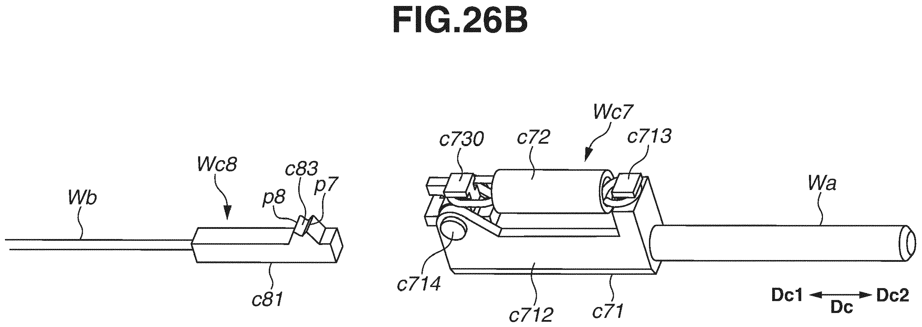

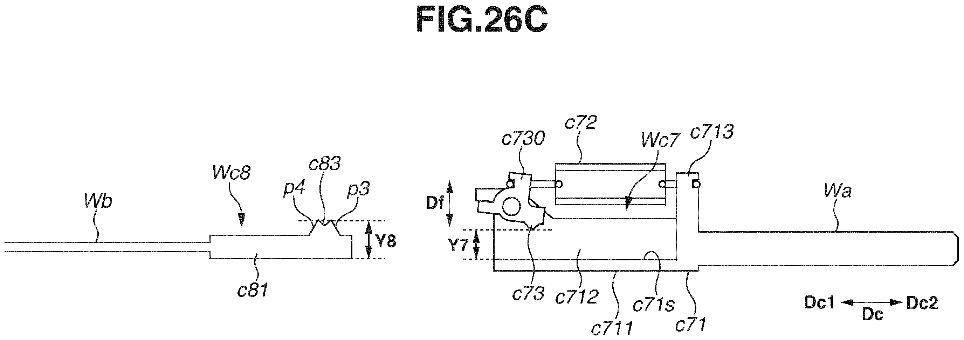

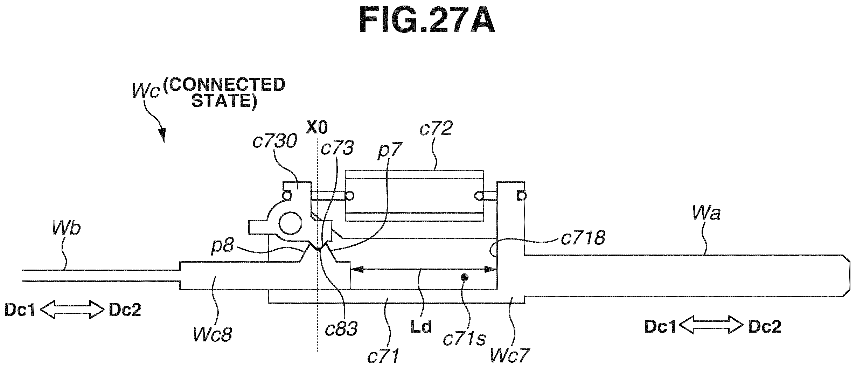

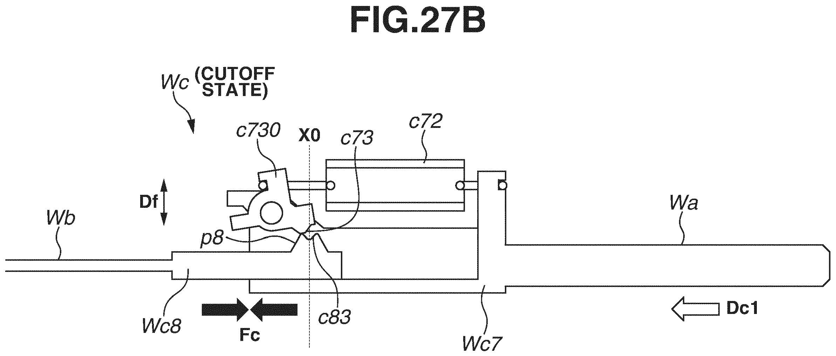

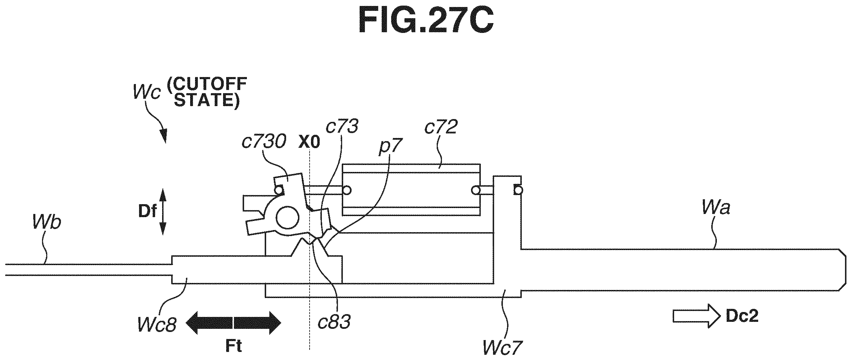

A medical apparatus according to the fourth exemplary embodiment will be described with reference to Figs. 26A, 26B, 26C, 27A, 27B, and 27C. The present exemplary embodiment differs from the first exemplary embodiment in a configuration of a connection unit Wc that connects the drive source M and the wire member Wb. Hereinafter, the components assigned the same reference numerals as those in the first exemplary embodiment have substantially the same configuration and function as those described in the first exemplary embodiment, and a difference from the first exemplary embodiment will be mainly described.

Fig. 26A is a schematic diagram illustrating the drive wire W according to the present exemplary embodiment that includes the connection unit Wc. Fig. 26B is a perspective view illustrating a holder Wc7 and a rod Wc8 that are included in the connection unit Wc. Fig. 26C is a cross-sectional view illustrating the holder Wc7 and the rod Wc8 that are included in the connection unit Wc. Fig. 26A illustrates a state (connected state) in which the rod Wc8 is held in the holder Wc7, and Figs. 26B and 26C illustrate a state (cutoff state) in which the rod Wc8 is not held in the holder Wc7.

In the following description, the connection unit Wc disposed in the drive wire W, which is an arbitrary one of the first to ninth drive wires (W 11 to W33) included in the catheter unit 100 according to the present exemplary embodiment, will be described. In the configuration example according to the present exemplary embodiment, the connection unit Wc having substantially the same configuration as the configuration to be described below is disposed in each of the nine drive wires (W11 to W33).

As illustrated in Fig. 26A, the connection unit Wc includes the holder Wc7 serving as a first member (engagement member, holding member) that is connected to the drive source M, and the rod Wc8 serving as a second member (engaged member, held member) that is connected to the wire member Wb of the drive wire W. In the present exemplary embodiment, at least part of the holder Wc7 is a member formed integrally with the held portion Wa of the drive wire W. Thus, the holder Wc7 is connected to the drive source M via the held portion Wa and the coupling unit 21c (Figs. 6A, 6B, and 6C). The holder Wc7 and the held portion Wa may be formed as separate members. Meanwhile, the rod Wc8 is fixed to the proximal end of the wire member Wb using an arbitrary fixing method, such as pressure bonding (swaging) or adhesive bonding.

As illustrated in Figs. 26B and 26C, the holder Wc7 includes a main body portion (base portion) c71 formed integrally with the held portion Wa, a torsion coil spring c72 supported by the main body portion c31, and a rotational member c730. The main body portion c71 has a groove shape formed with a bottom portion c711 and side wall portions c712 and has a cross-sectional shape resembling a U-shape with right-angled corners (rectangular shape with one side opened) when viewed in the direction Dc. The torsion coil spring c72 and the rotational member c730 are attached to an opened portion in the direction Df of the main body portion c71. Thus, a space c71s that accepts the rod Wc8, which will be described below, is formed between the bottom portion c711 and the side wall portions c712 of the main body portion c71, the torsion coil spring c72, and the rotational member c730. The main body portion c71 is disposed next to the held portion Wa in the direction Dc, and the held portion Wa has a length in the direction Dc2 from the end portion in the direction Dc2 of the main body portion c71.

The torsion coil spring c72 is disposed in such a manner that an axis line direction (expansion/contraction direction) coincides with the direction Dc, an end portion in the direction Dc2 is supported by a support portion c713 of the main body portion c71, and an end portion in the direction Dc1 is attached to the rotational member c730. The rotational member c730 is rotatable around a support shaft c714 disposed in the main body portion c71. The rotational member c730 is urged in a clockwise direction in Figs. 26A to 26C by elastic force of the torsion coil spring c72 and positioned by being locked by a locking unit c715 disposed in the main body portion c71.

The holder Wc7 includes a protruding portion c73 formed on the rotational member c730. In a state in which the rotational member c730 is locked by the locking unit c715, the protruding portion c73 protrudes toward the bottom portion c711 of the main body portion c71 from the support shaft c714. In other words, the protruding portion c73 is an example of a protruding portion protruding in a direction intersecting with the length direction (direction Dc) of the wire member Wb in the connection unit Wc.

In a case where the protruding portion c73 is rotated counterclockwise in such a manner as to move away upward in Figures by the protruding portion c73 of the rotational member c730 being pressed by the rod Wc8, the torsion coil spring c72 deforms in accordance with the rotation of the rotational member c730. That is, the torsion coil spring c72 functions as a deformation element deformable in such a manner as to allow the protruding portion c73 to come off from a recess portion c83 to be described below.

When viewed in the direction Dc, the protruding portion c73 is formed at a position corresponding to the recess portion c83 in the rod Wc8 to be described below. In the present exemplary embodiment, the protruding portion c73 is formed at one part in such a manner as to correspond to the recess portion c83 formed at one part. The protruding portion c73 is only required to be formed at a position at which the protruding portion c73 can engage with the recess portion c83.

As illustrated in Figs. 27B and 27C, the rod Wc8 includes a base portion c81 that has a substantially quadrangular prism shape and has a length in the direction Dc2 from the proximal end of the wire member Wb, and the recess portion c83 formed on a side surface (surface facing the rotational member c730) in the direction Df of the base portion c81. The recess portion c83 is formed between protrusions p7 and p8 protruding in the direction Df from the base portion c81. The protrusions p7 and p8 are arranged side by side in the direction Dc.

The rod Wc8 is inserted in the direction Dc2 toward the space c71s between the main body portion c71, the rotational member c730, and the torsion coil spring c72 of the holder Wc7. Then, the rod Wc8 is attached to the holder Wc7 by being pressed in until the protruding portion c73 of the holder Wc7 fits into the recess portion c83 of the rod Wc8. In the present exemplary embodiment, a part of the rod Wc8 on a side in the direction Dc2 is an insertion portion that is inserted into the space c71s in the holder Wc7.

Next, movement of the connection unit Wc in the connected state and in an overload state will be described with reference to Figs. 27A, 27B, and 27C. Fig. 27A is a schematic diagram illustrating a cross-section of the connection unit Wc in the connected state. Fig. 27C is a schematic diagram illustrating a state of the connection unit Wc in a case where overload acts in a direction (direction Dc1) in which the wire member Wb is pressed. Fig. 27C is a schematic diagram illustrating a state of the connection unit Wc in a case where overload acts in a direction (direction Dc2) in which the wire member Wb is pulled.

As illustrated in Fig. 27A, in the connected state of the connection unit Wc, the protruding portion c73 of the holder Wc7 is fitted in the recess portion c83 of the rod Wc8. Specifically, the protruding portion c73 is held between the protrusions p7 and p8 in the direction Dc. As illustrated in Fig. 26C, a distance in a direction Df from the bottom surface of the base portion c81 to the top of the protrusion p7 or p8 in a state in which the rod Wc8 is not attached to the holder Wc7 is a distance Y8. The distance Y8 is longer than a distance Y7 in the direction Df from the protruding portion c73 of the holder Wc7 to the bottom portion c711 of the main body portion c71. Thus, in the connected state illustrated in Fig. 27A, the protruding portion c73 of the holder Wc7 and a part (protrusion p7 or p8) of the rod Wc8 are in a positional relationship of interfering with each other in the direction Dc. In other words, when viewed in the length direction (direction Dc) of the wire member in the connected state, at least part of the protruding portion formed on one of the first member or the second member overlaps the recess portion formed on the other one of the first member or the second member.

With such a configuration, in a case where the connection unit Wc is in the connected state, relative movements of the holder Wc7 and the rod Wc8 are restricted, and the holder Wc7 and the rod Wc8 integrally move in the direction Dc1 and the direction Dc2. In other words, in a case where the connection unit Wc is in the connected state, drive force from the drive source M is transmitted to the wire member Wb via the connection unit Wc.

In the connected state illustrated in Fig. 27A, the protruding portion c73 of the holder Wc7 is desirably configured to fit into the recess portion c83 of the rod Wc8 in a backlash-reduced state. In the present exemplary embodiment, the protruding portion c73 is connected to the torsion coil spring c72 having elasticity, and the torsion coil spring c72 is in a slightly-elastically-deformed state in such a manner that the protruding portion c73 is lifted up in the direction Df (upward in Fig. 27A) in the connected state. For this reason, by the elastic force of the torsion coil spring c72, the protruding portion c73 is pressed against the protrusions p7 and p8 on both sides. In other words, the connection unit Wc according to the present exemplary embodiment connects the holder Wc7 serving as the first member and the rod Wc8 serving as the second member, by a mechanism (snap-fit mechanism) that uses a deformation element made of elastic material.

In this manner, with the configuration of engaging the holder Wc7 and the rod Wc8 with each other in the backlash-reduced state, the responsivity of the bending portion 12 of the catheter 11 to the drive from the drive source M can be improved.

### (When Overload in Pressing Direction Occurs)

Next, a case where excessive force acts on the connection unit Wc in the direction (direction Dc1) in which the wire member Wb is pressed will be described. As illustrated in Fig. 27B, in transmission of drive force from the drive source M to the holder Wc7 in the direction (direction Dc1) in which the wire member Wb is pressed, compression force Fc acts between the holder Wc7 and the rod Wc8.

In a case where the compression force Fc does not exceed a preset threshold value (second threshold value), the compression force Fc is received by the rod Wc8 via a contact portion between the protruding portion c73 of the holder Wc7 and the protrusion p8 of the rod Wc8. That is, the connection unit Wc according to the present exemplary embodiment is configured to maintain, in a case where the compression force Fc at the second threshold value or less acts, a state in which the protruding portion c73 is fitted in the recess portion c83, and transmit drive force from the drive source M to the wire member Wb.

In a case where large compression force Fc exceeding the preset threshold value (second threshold value) acts, the rotational member c730 rotates in such a manner that the protruding portion c73 moves over the protrusion p8 of the rod Wc8, and consequently, the torsion coil spring c72 elastically deforms. Consequently, the holder Wc7 and the rod Wc8 move relative to each other while the protruding portion c73 moves over the protrusion p8, and the protruding portion c73 comes off from the recess portion c83. In a state (cutoff state) in which the protruding portion c73 has come off from the recess portion c83, even in a case where the holder Wc7 moves in the direction Dc1, the rod Wc8 does not move in the direction Dc1. That is, the connection unit Wc according to the present exemplary embodiment is configured to allow, in a case where the compression force Fc exceeding the second threshold value acts, the protruding portion c73 to come off from the recess portion c83, and cut off transmission of drive force from the drive source M to the wire member Wb (Fig. 27B).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As illustrated in Fig. 27A, a clearance Ld that allows relative movements of the holder Wc7 and the rod Wc8 is left between a leading end position in the direction Dc2 of the rod Wc8 in the connected state and a wall surface c718 serving as a bottom portion of the space c71s of the holder Wc7. The length in the direction Dc of the clearance Ld is set in such a manner that the wall surface c718 does not come into contact with the rod Wc8 even in a case where the holder Wc7 further moves in the direction Dc1 by a predetermined distance after the protruding portion c73 has completely come off from the recess portion c83, with respect to a position X0 of the protruding portion c73 in the connected state. With such a clearance Ld, drive force in the direction Dc1 is prevented from being transmitted by the rod Wc8 being pressed by the wall surface c718 of the holder Wc7 even though the protruding portion c73 has come off from the recess portion c83.

### (When Overload in Pulling Direction Occurs)

Next, a case where excessive force acts on the connection unit Wc in the direction (direction Dc2) in which the wire member Wb is pulled will be described. As illustrated in Fig. 27C, in transmission of drive force from the drive source M to the holder Wc7 in the direction (direction Dc2) in which the wire member Wb is pulled, pulling force Ft acts between the holder Wc7 and the rod Wc8.

In a case where the pulling force Ft does not exceed a preset threshold value (first threshold value), the pulling force Ft is received by the rod Wc8 via a contact portion between the protruding portion c73 of the holder Wc7 and the protrusion p7 of the rod Wc8. That is, the connection unit Wc according to the present exemplary embodiment is configured to maintain, in a case where the pulling force Ft at the first threshold value or less acts, a state in which the protruding portion c73 is fitted in the recess portion c83, and transmit drive force from the drive source M to the wire member Wb.

In a case where large pulling force Ft exceeding the preset threshold value (first threshold value) acts, the rotational member c730 rotates in such a manner that the protruding portion c73 moves over the protrusion p7 of the rod Wc8, and consequently, the torsion coil spring c72 elastically deforms. Consequently, the holder Wc7 and the rod Wc8 move relative to each other while the protruding portion c73 moves over the protrusion p7, and the protruding portion c73 comes off from the recess portion c83. In a state (cutoff state) in which the protruding portion c73 has come off from the recess portion c83, even in a case where the holder Wc7 moves in the direction Dc2, the rod Wc8 does not move in the direction Dc2. That is, the connection unit Wc according to the present exemplary embodiment is configured to allow, in a case where the pulling force Ft exceeding the second threshold value acts, the protruding portion c73 to come off from the recess portion c83, and cut off transmission of drive force from the drive source M to the wire member Wb (Fig. 27C).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As described above, also according to the present exemplary embodiment, each member included in the connection unit Wc is a part of the catheter unit 100 that is a unit detachably attached to another unit (the base unit 200, etc.) of the medical apparatus 1. Thus, the medical apparatus 1 can be brought into a usable state again by a simple method of detaching the catheter unit 100 from the base unit 200 after cutting off the connection between the drive source M and the wire member Wb, and replacing the catheter unit 100 with a new catheter unit 100.

### [Fifth Exemplary Embodiment]

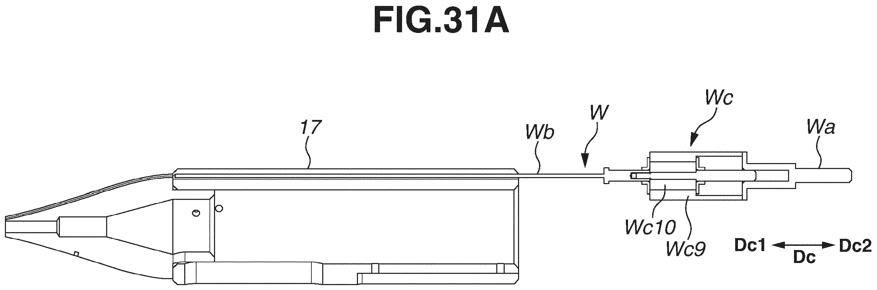

A medical apparatus according to the fifth exemplary embodiment will be described with reference to Figs. 31A, 31B, 31C, 32A, 32B, 32C, 32D, 32E, 32F, and 33A to 33C. In the present exemplary embodiment, a configuration of a connection unit Wc that connects the drive source M and the wire member Wb differs from the configuration in which a protruding portion formed on either one of the first member and the second member and a recess portion formed in the other one are engaged with each other as in the exemplary embodiments described so far. In the present exemplary embodiment, the connection unit Wc includes a magnet. Hereinafter, the components assigned the same reference numerals as those in the first exemplary embodiment have substantially the same configuration and action as those described in the first exemplary embodiment, and a difference from the first exemplary embodiment will be mainly described.

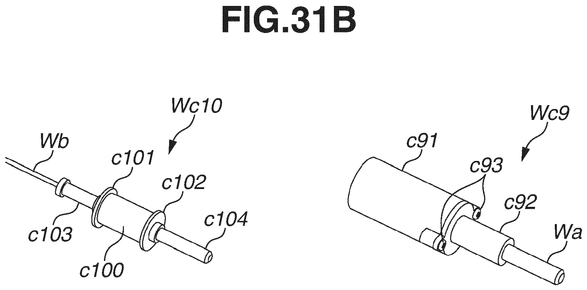

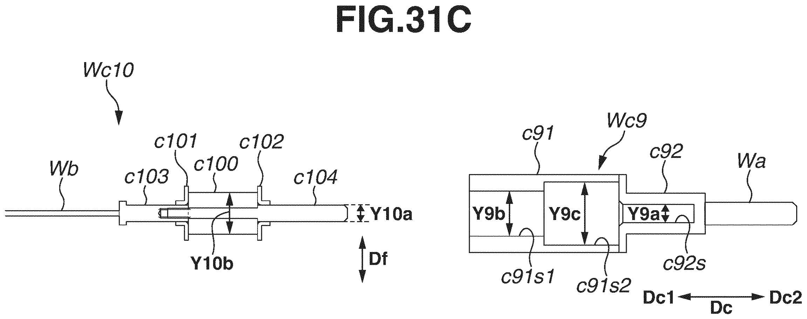

Fig. 31A is a schematic diagram illustrating the drive wire W according to the present exemplary embodiment that includes the connection unit Wc. Fig. 31B is a perspective view illustrating a holder Wc9 and a rod Wc10 that are included in the connection unit Wc. Fig. 31C is a cross-sectional view illustrating the holder Wc9 and the rod Wc10 that are included in the connection unit Wc. Fig. 31A illustrates a state (connected state) in which the rod Wc10 is held in the holder Wc9, and Figs. 31B and 31C are perspective views separately illustrating configurations of the holder Wc9 and the rod Wc10.

In the following description, the connection unit Wc disposed in the drive wire W, which is an arbitrary one of the first to ninth drive wires (W 11 to W33) included in the catheter unit 100 according to the present exemplary embodiment, will be described. In the configuration example according to the present exemplary embodiment, the connection unit Wc having substantially the same configuration as the configuration to be described below is disposed in each of the nine drive wires (W11 to W33).

As illustrated in Fig. 31A, the connection unit Wc includes the holder Wc9 serving as a first member (engagement member, holding member) that is connected to the drive source M, and the rod Wc10 serving as a second member (engaged member, held member) that is connected to the wire member Wb of the drive wire W. In the present exemplary embodiment, at least part of the holder Wc9 is a member formed integrally with the held portion Wa of the drive wire W. Thus, the holder Wc9 is connected to the drive source M via the held portion Wa and the coupling unit 21c (Figs. 6A, 6B, and 6C). The holder Wc9 and the held portion Wa may be formed as separate members. Meanwhile, the rod Wc10 is fixed to the proximal end of the wire member Wb by using an arbitrary fixing method, such as pressure bonding (swaging) or adhesive bonding.

As illustrated in Figs. 31B and 31C, the holder Wc9 includes a cylindrical portion c92 formed integrally with the held portion Wa, and a main body cylindrical portion c91 (engagement member) fixed to the cylindrical portion c92 by a fixing member c93 such as a screw. The main body cylindrical portion c91 is a tubular member that has a length in the direction Dc, has no bottom, and is opened at both ends in the direction Dc. The cylindrical portion c92 is a member that has a length in the direction Dc, and has a substantially bottomed cylindrical shape opened toward the direction Dc1. The main body cylindrical portion c91 and the cylindrical portion c92 are an example of a tubular portion that has a length in the length direction of the wire member Wb and defines a space into which the second member can be inserted, and as an alternative, a polygonal tubular shape may be employed, for example. The cylindrical portion c92 needs not be formed integrally with the held portion Wa, and may be a separate member.

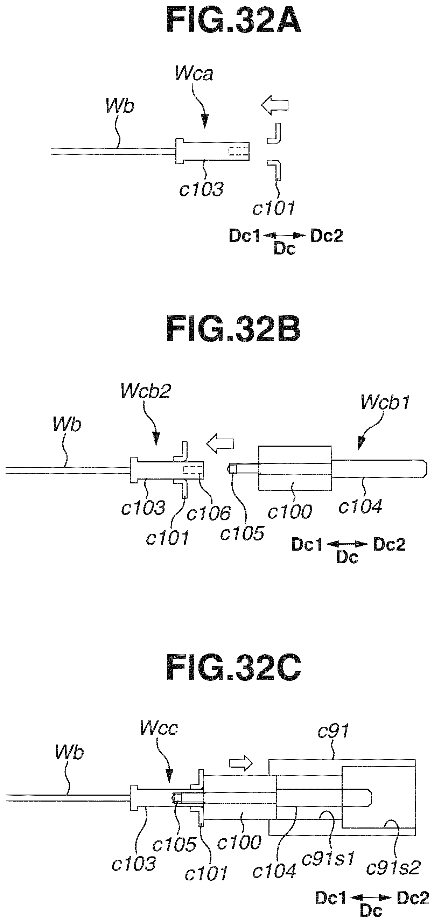

As illustrated in Figs. 31B and 31C, the rod Wc10 includes a substantially columnar first shaft portion c103 lengthened in the direction Dc2 from the proximal end of the wire member Wb, and a second shaft portion c104 that is fixed to the first shaft portion c103 by being inserted into the first shaft portion c103. The rod Wc10 further includes a magnet c100 fixed to the second shaft portion c104, and iron plates c101 and c102 pivotally supported in such a manner as to slidable with respect to the first shaft portion c103 and the second shaft portion c104. The iron plates c101 and c102 are disk-shaped members each having a hole at its center. The iron plates c101 and c102 are attracted to the magnet c100 by magnetic force, and in close contact in such a manner as to be side by side in the direction Dc as illustrated in Figs. 32B and 32C.

In the present exemplary embodiment, a neodymium magnet with strong magnetic force is used as the magnet c100. The magnet c100 is not limited to this, and a permanent magnet of another type, such as a ferrite magnet, may be used. The material of the iron plates c101 and c102 is not limited to iron. The iron plates c101 and c102 are only required to be plates of another type of metal being a ferromagnetic member (magnetic member). The orientation of a magnetic pole is not limited. It is sufficient that the magnet c100 and the iron plate c101 and c102 are in a relationship of attracting each other.

Here, as illustrated in Fig. 31C, two spaces c91s1 and c91s2 are formed inside the main body cylindrical portion c91 of the holder Wc9. In the direction Df orthogonal to the direction Dc, a distance Y9b of the space c91s1 is shorter than a distance Y9c of the space c91s2. A space c92s is formed inside the cylindrical portion c92. A distance in the direction Df of the space c92s is a distance Y9a. Meanwhile, a distance in the direction Df of the second shaft portion c104 of the rod Wc10 is a distance Y10a. A distance in the direction Df of the magnet c100 is a distance Y10b.

As illustrated in Fig. 31A, because the second shaft portion c104 of the rod Wc10 is inserted into the space c92s in the cylindrical portion c92 of the holder Wc9, the distance Y9a and the distance Y10a are substantially the same, or the distance Y9a is longer. Furthermore, because the magnet c100 of the rod Wc10 is inserted into the space c91s1 in the main body cylindrical portion c91 of the holder Wc9, the distance Y9b and the distance Y10b are substantially the same, or the distance Y9b is slightly longer.

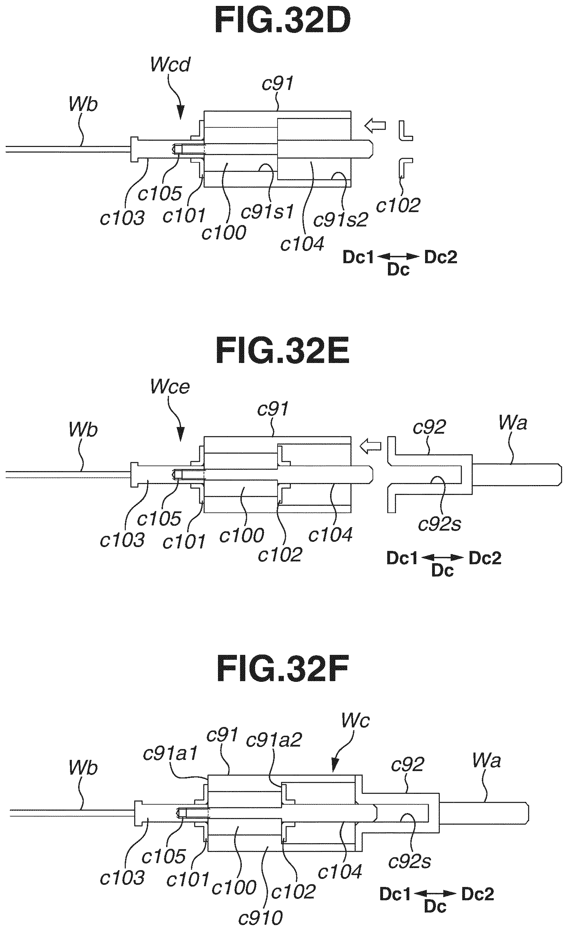

A procedure of forming the connection unit Wc by fitting together of the holder Wc9 and the rod Wc10 will be described with reference to Figs. 32A, 32B, 32C, 32D, 32E, and 32F. First of all, Fig. 32A illustrates a component Wca including the wire member Wb and the first shaft portion c103 fixed to the proximal end of the wire member Wb. Then, the iron plate c101 is put around the component Wca in the direction Dc1 in such a manner that the first shaft portion c103 passes through the hole of the disk-shaped iron plate c101. The iron plate c101 is configured to be slidable with respect to the first shaft portion c103 in the direction Dc.

Next, as illustrated in Fig. 32B, a component Wcb 1 including the magnet c100 and the second shaft portion c104 is prepared. A hole is formed at the center of the magnet c100, and the component Wcb1 can be obtained by inserting the second shaft portion c104 in such a manner that the second shaft portion c104 passes through the hole formed in the magnet c100. Instead of a configuration in which the second shaft portion c104 passes through the magnet c100, a configuration in which different axis members are bonded to both ends in the direction Dc of the magnet c100 may be employed. After the insertion of the second shaft portion c104, the magnet c100 is fixed to the second shaft portion c104 using an adhesive agent or the like. That is, the magnet c100 is not configured to be slidable with respect to the second shaft portion c104 in the direction Dc.

A screw portion c105 is disposed at the end portion in the direction Dc1 of the second shaft portion c104. A screw receiver portion c106 is disposed at the end portion in the direction Dc2 of the first shaft portion c103. The screw receiver portion c106 is a hole in which a screw groove is formed on the inner surface, and has a configuration into which the screw portion 105 can be inserted. A component including the first shaft portion c103 obtained through the process illustrated in Fig. 32A will be referred to as a component Wcb2, and the component Wcb1 can be fixed to the component Wcb2 by inserting and screwing the screw portion c105 into the screw receiver portion c106.

Fig. 32C illustrates an operation in which a component Wcc obtained by fitting the first shaft portion c103 and the second shaft portion c104 together through the process illustrated in Fig. 32B is inserted into the inside of the main body cylindrical portion c91 that is a part of the holder Wc9. As described above, the two spaces c91s1 and c91s2 are formed in the main body cylindrical portion c91, and the second shaft portion c104 is inserted into the main body cylindrical portion c91 in the direction Dc2 until the magnet c100 is completely fitted in the space c91s1.

Fig. 32D illustrates a state in which the magnet c100 has been completely fitted in the space c91s1. A component obtained through the process illustrated in Fig. 32C will be referred to as a component Wcd. In this state, the second shaft portion c104 is inside the space c92s2, and a part of the second shaft portion c104 is exposed to the outside from the main body cylindrical portion c91 in the direction Dc2. Then, the iron plate c102 is fitted around the component Wcd in the direction Dc2 in such a manner that the second shaft portion c104 passes through the hole of the disk-shaped iron plate c102. The iron plate c102 is configured to be slidable with respect to the second shaft portion c104 in the direction Dc.

Fig. 32E illustrates an operation of attaching the cylindrical portion c92 formed integrally with the held portion Wa, to a component Wce obtained through the process illustrated in Fig. 32D. The space c92s is formed inside the cylindrical portion c92, and the cylindrical portion c92 is attached to the component Wce in such a manner that the second shaft portion c104 is inserted into the space c92s. After the attachment of the cylindrical portion c92, the cylindrical portion c92 is fixed to the main body cylindrical portion c91 using the fixing member c93 (illustrated in Fig. 31B). The connection unit Wc according to the present exemplary embodiment is thereby prepared as illustrated in Fig. 32F.

In Fig. 32F, the iron plate c101 is pivotably supported by the first shaft portion c103 and pulled by the magnet c100 in the direction Dc2. Thus, the iron plate c101 is in close contact with an end surface in the direction Dc1 of the magnet c100, and an end surface c91a1 in the direction Dc1 of the main body cylindrical portion c91. The iron plate c102 is pivotably supported by the second shaft portion c104 and pulled by the magnet c100 in the direction Dc1. Thus, the iron plate c102 is in close contact with an end surface in the direction Dc2 of the magnet c100, and an end surface c91a2 in the direction Dc2 of the main body cylindrical portion c91.

In Fig. 32F, a portion that is formed at a position corresponding to the space c91s1 (illustrated in Fig. 32D) in the main body cylindrical portion c91 and partially protrudes toward the inside of the main body cylindrical portion c91 is referred to as a protruding portion c910. The above-described end surfaces c91a1 and c91a2 can also be represented as end surfaces in the direction Dc of the protruding portion c910. In the present exemplary embodiment, a state in which the protruding portion c910 and the iron plate c101 or c102 are in close contact with each other is maintained, whereby the connected state of the holder Wc9 and the rod Wc10 is maintained.

Hereinafter, a state in which the rod Wc10 is held in the holder Wc9 in such a manner that drive force in the direction Dc1 or the direction Dc2 that has been transmitted from the drive source M can be transmitted to the wire member Wb will be referred to as a connected state (engaged state, attached state) of the connection unit Wc. A state in which either the iron plate c101 or c102 comes off from the magnet c100 and the connection between the drive source M and the wire member Wb is cut off will be referred to as a cutoff state (unconnected state, withdraw state, detachment state) of the connection unit Wc.

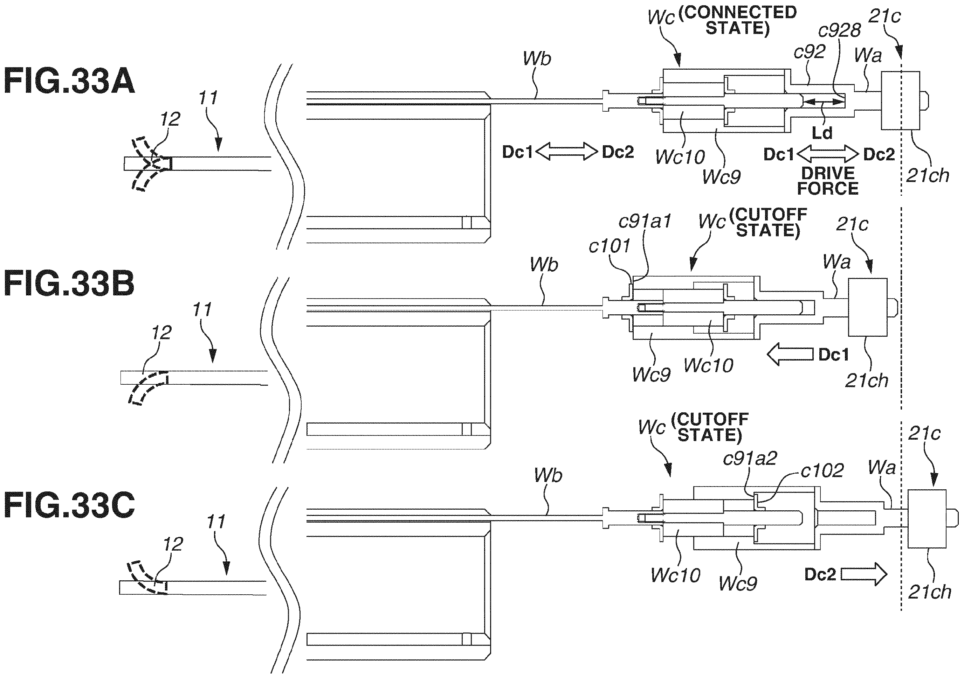

Next, movement of the connection unit Wc in the connected state and in an overload state will be described with reference to Figs. 33A to 33C. Fig. 33A is a schematic diagram illustrating a cross-section of the connection unit Wc in the connected state. Fig. 33B is a schematic diagram illustrating a state of the connection unit Wc in a case where overload acts in a direction (direction Dc1) in which the wire member Wb is pressed. Fig. 33C is a schematic diagram illustrating a state of the connection unit Wc in a case where overload acts in a direction (direction Dc2) in which the wire member Wb is pulled.

As illustrated in Fig. 33A, in the connected state of the connection unit Wc, the holder Wc9 and the rod Wc10 engage with each other by the action of the magnet c100 and integrally move. Specifically, as described with reference to Fig. 32F, the end surface c91a1 in the direction Dc1 of the main body cylindrical portion c91 that is a part of the holder Wc9, and the iron plate c101 are in close contact with each other, and the end surface c91a2 in the direction Dc2 of the main body cylindrical portion c91 and the iron plate c102 are in close contact with each other. Furthermore, the iron plates c101 and c102 are attracted by the magnet c100. That is, these members continue to maintain the state of being in close contact with each other, unless load larger than magnetic force acting between the iron plates c101 and c102 and the magnet c100 is generated, and the iron plates c101 and c102 come off from the magnet c100.

With such a configuration, in a case where the connection unit Wc is in the connected state, relative movements of the holder Wc9 and the rod Wc10 are restricted, and the holder Wc9 and the rod Wc10 integrally move in the direction Dc1 and the direction Dc2. In other words, in a case where the connection unit Wc is in the connected state, drive force from the drive source M is transmitted to the wire member Wb via the connection unit Wc.

### (When Overload in Pressing Direction Occurs)

Next, a case where excessive force acts on the connection unit Wc in the direction (direction Dc1) in which the wire member Wb is pressed will be described. As illustrated in Fig. 33B, in transmission of drive force in the direction (direction Dc1) in which the wire member Wb is pressed is transmitted from the drive source M to the holder Wc9, compression force Fc acts between the holder Wc9 and the rod Wc10.

In a case where the compression force Fc does not exceed a preset threshold value (second threshold value), the compression force Fc is received by the rod Wc10 via the end surface c91a1 of the main body cylindrical portion c91 of the holder Wc9 and the iron plate c101 of the rod Wc10. That is, the connection unit Wc according to the present exemplary embodiment is configured to maintain, in a case where the compression force Fc at the second threshold value or less acts, a state in which the end surface c91a1 of the main body cylindrical portion c91 and the iron plate c101 are in close contact with each other, and transmit drive force from the drive source M to the wire member Wb.

In a case where large compression force Fc exceeding the preset threshold value (second threshold value) acts, force is added in a direction in which the magnet c100 and iron plate c101 are separated. In this case, while the iron plate c101 moves in the direction Dc1 together with the main body cylindrical portion c91, the iron plate c101 moves away from the magnet c100. In a state (cutoff state) in which the iron plate c101 and the magnet c100 have been separated in this manner, even in a case where the holder Wc9 moves in the direction Dc1, the rod Wc10 does not move in the direction Dc1. That is, the connection unit Wc according to the present exemplary embodiment is configured to separate, in a case where the compression force Fc exceeding the second threshold value acts, the iron plate c101 and the magnet c100, and cut off transmission of drive force from the drive source M to the wire member Wb (Fig. 33B).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As illustrated in Fig. 33A, a clearance Ld that allows relative movements of the holder Wc9 and the rod Wc10 is left between a leading end position in the direction Dc2 of the rod Wc10 in the connected state and a wall surface c928 serving as a bottom portion of the space c92s of the cylindrical portion c92 of the holder Wc9. The length in the direction Dc of the clearance Ld is set as follows. Specifically, the length in the direction Dc of the clearance Ld is set in such a manner that the wall surface c928 does not come into contact with the rod Wc10 even in a case where the holder Wc9 further moves in the direction Dc1 by a predetermined distance after the iron plate c101 has been completely separated from the magnet c100, with respect to a position X0 of the iron plate c101 in the connected state. With such a clearance Ld, drive force in the direction Dc1 is prevented from being transmitted by the rod Wc10 being pressed by the wall surface c928 of the holder Wc9 even though the iron plate c101 has come off from the magnet c100.

### (When Overload in Pulling Direction Occurs)

Next, a case where excessive force acts on the connection unit Wc in the direction (direction Dc2) in which the wire member Wb is pulled will be described. As illustrated in Fig. 33C, in transmission of drive force from the drive source M to the holder Wc9 in the direction (direction Dc2) in which the wire member Wb is pulled, pulling force Ft acts between the holder Wc9 and the rod Wc10.

In a case where the pulling force Ft does not exceed a preset threshold value (first threshold value), the pulling force Ft is received by the rod Wc10 via the end surface c91a2 of the main body cylindrical portion c91 of the holder Wc9 and the iron plate c102 of the rod Wc10. That is, the connection unit Wc according to the present exemplary embodiment is configured to maintain, in a case where the pulling force Ft at the first threshold value or less acts, a state in which the end surface c91a2 of the main body cylindrical portion c91 and the iron plate c102 are in close contact with each other, and transmit drive force from the drive source M to the wire member Wb.

In a case where large pulling force Ft exceeding the preset threshold value (first threshold value) acts, force is added in a direction in which the magnet c100 and the iron plate c102 are separated. In this case, while the iron plate c102 moves in the direction Dc2 together with the main body cylindrical portion c91, the iron plate c102 moves away from the magnet c100. In a state (cutoff state) in which the iron plate c102 and the magnet c100 have been separated from each other in this manner, even in a case where the holder Wc9 moves in the direction Dc2, the rod Wc10 does not move in the direction Dc2. That is, the connection unit Wc according to the present exemplary embodiment is configured to separate, in a case where the pulling force Ft exceeding the first threshold value acts, the iron plate c102 and the magnet c100 from each other, and cut off transmission of drive force from the drive source M to the wire member Wb (Fig. 33C).

The switching of the state of the connection unit Wc from the connected state to the cutoff state when overload occurs reduces a possibility that a member, such as the coupling unit 21c, is damaged when the bending portion 12 of the catheter 11 is bent by excessively-strong force due to the malfunction of the drive source M, or the wire member Wb receives external force.

As described above, also according to the present exemplary embodiment, each member included in the connection unit Wc is a part of the catheter unit 100 that is a unit detachably attached to another unit (the base unit 200, etc.) of the medical apparatus 1. Thus, the medical apparatus 1 can be brought into a usable state again by a simple method of detaching the catheter unit 100 from the base unit 200 after cutting off the connection between the drive source M and the wire member Wb, and replacing the catheter unit 100 with a new catheter unit 100.

### (Other Exemplary Embodiments)

In each of the above-described exemplary embodiments, the description has been given of a case in which, as a threshold value of load at which the connection of the connection unit Wc is cut off, the first threshold value for a case where the pulling force acts between the first member and the second member, and the second threshold value for a case where the compression force acts between the first member and the second member are the same values. Alternatively, the first threshold value and the second threshold value may be set to different values. For example, in a case where load expected in a normal use condition varies depending on the direction in which the wire member Wb is driven, the first threshold value and the second threshold value are set in accordance with the range of the expected load.

In each of the above-described exemplary embodiments, common values are set for the first threshold value and the second threshold value in the connection units among sets of the plurality of drive sources (M11 to M33) and the plurality of wire members (Wb11 to Wb33). Alternatively, different values of the first threshold value and the second threshold value may be set in the connection units among sets of drive sources and wire members. For example, in a case where expected load varies between a wire member connected to a guide ring closer to the leading end and a wire member connected to a guide ring closer to the proximal end in the bending portion 12 of the catheter 11, the first threshold value and the second threshold value are set in accordance with the range of the expected load.

In each of the above-described exemplary embodiments, drive sources and wire members are connected via connection units in the sets of the plurality of drive sources (M11 to M33) and the plurality of wire members (Wb11 to Wb33). However, a connection unit may be disposed in only a part of all sets including the drive sources and the wire members.

In each of the above-described exemplary embodiments, the description has been given using the bendable catheter 11 as an example of a target to be operated. However, the target is not limited to this, and the target to be operated may include a multi-joint robot. Examples of the multi-joint robot include a medical robot arm including a surgical appliance (forceps, sharp pointed scalpel, etc.) at its leading end, for example. In a case where a wire is used to bend a joint of this robot, the breakaway mechanism described in each of the above-described exemplary embodiments may be applied.

The present invention is not limited to the above-described exemplary embodiments, and various modifications and variations can be made without departing from the spirit and scope of the present invention. Accordingly, the following claims are appended to disclose the scope of the present invention.

This application claims the benefit of Japanese Patent Application No. 2021-141376, filed August 31, 2021, which is hereby incorporated by reference herein in its entirety.

## Claims

1. A medical apparatus comprising:
a base unit including a drive source therein; and
a bendable unit that is to be detachably attached to the base unit and includes a bending portion and a bending drive unit configured to receive drive force from the drive source and bend the bending portion, the bending drive unit including a linear member connected to the bending portion,
wherein the bendable unit includes a connection unit including a first member connected to the drive source, and a second member connected to the linear member,
wherein, in a case where pulling force at a first threshold value or less or compression force at a second threshold value or less acts between the first member and the second member, the connection unit maintains a state in which the first member and the second member are connected, and transmits the drive force from the drive source to the linear member, and
wherein, in a case where pulling force exceeding the first threshold value or compression force exceeding the second threshold value acts between the first member and the second member, the connection unit separates the first member and the second member from each other, and cuts off transmission of the drive force from the drive source to the linear member.

2. The medical apparatus according to Claim 1,
wherein the connection unit includes a stopper disposed on at least one of the first member and the second member, and
wherein, in a state in which transmission of the drive force from the drive source to the linear member is cut off in the connection unit, the stopper restricts separation of the first member and the second member by engaging with the other one of the first member and the second member when the bendable unit is detached.

3. The medical apparatus according to Claim 1 or 2,
wherein the first member and the second member are connected by a protruding portion that is disposed on one of the first member and the second member and protrudes in a direction intersecting with a length direction of the linear member, being fitted in a recess portion disposed on the other one of the first member and the second member, and
wherein, in a case where pulling force at a first threshold value or less or compression force at a second threshold value or less acts between the first member and the second member, a state in which the protruding portion is fitted in the recess portion is maintained, and the drive force is transmitted from the drive source to the linear member, and
wherein, in a case where pulling force exceeding the first threshold value or compression force exceeding the second threshold value acts between the first member and the second member, the protruding portion comes off from the recess portion, and transmission of the drive force from the drive source to the linear member is cut off.

4. The medical apparatus according to Claim 3,
wherein the connection unit includes an elastic element which is elastically-deformable,
wherein, in a case where pulling force at the first threshold value or less or compression force at the second threshold value or less acts between the first member and the second member, a state in which the protruding portion is fitted in the recess portion is maintained with elastic force of the elastic element, and
wherein, in a case where pulling force exceeding the first threshold value or compression force exceeding the second threshold value acts between the first member and the second member, the protruding portion is allowed to come off from the recess portion with elastic deformation of the elastic element.

5. The medical apparatus according to Claim 3 or 4,
wherein the first member has a space into which the second member is insertable from one side in the length direction of the linear member, and
wherein, in a state in which the second member is inserted in the space of the first member, the protruding portion is fitted in the recess portion.

6. The medical apparatus according to Claim 5,
wherein the first member includes a tubular portion that has a length in the length direction of the linear member and forms the space,
wherein the second member includes an insertion portion to be inserted into the space in the tubular portion,
wherein the protruding portion is disposed on one of an inner surface of the tubular portion and an outer surface of the insertion portion, and
wherein the recess portion is disposed in the other one of the inner surface the tubular portion and the outer surface of the insertion portion.

7. The medical apparatus according to Claim 1 or 2,
wherein a first protruding portion and a second protruding portion protruding in a direction intersecting with a length direction of the linear member are disposed on the first member, a third protruding portion and a fourth protruding portion are disposed on the second member, and the first member and the second member are connected by the first protruding portion engaging with the third protruding portion and the second protruding portion engaging with the fourth protruding portion,
wherein, in a case where pulling force at a first threshold value or less acts between the first member and the second member, a state in which the first protruding portion is engaged with the third protruding portion is maintained, and the drive force is transmitted from the drive source to the linear member,
wherein, in a case where pulling force exceeding the first threshold value acts between the first member and the second member, the first protruding portion comes off from the third protruding portion, and transmission of the drive force from the drive source to the linear member is cut off,
wherein, in a case where compression force at a second threshold value or less acts between the first member and the second member, a state in which the second protruding portion is engaged with the fourth protruding portion is maintained, and the drive force is transmitted from the drive source to the linear member, and
wherein, in a case where compression force exceeding the second threshold value acts between the first member and the second member, the second protruding portion comes off from the fourth protruding portion, and transmission of the drive force from the drive source to the linear member is cut off.

8. The medical apparatus according to Claim 7,
wherein the connection unit includes an elastic element which is elastically-deformable,
wherein, in a case where pulling force at the first threshold value or less or compression force at the second threshold value or less acts between the first member and the second member, a state in which the first protruding portion is engaged with the third protruding portion and the second protruding portion is engaged with the fourth protruding portion is maintained with elastic force of the elastic element, and
wherein, in a case where pulling force exceeding the first threshold value or compression force exceeding the second threshold value acts between the first member and the second member, the first protruding portion is allowed to come off from the third protruding portion, and the second protruding portion is allowed to come from the fourth protruding portion, with elastic deformation of the elastic element.

9. The medical apparatus according to Claim 7 or 8,
wherein the first member has a space into which the second member is insertable from one side in the length direction of the linear member, and
wherein, in a state in which the second member is inserted in the space of the first member, the first protruding portion is engaged with the third protruding portion, and the second protruding portion is engaged with the fourth protruding portion.

10. The medical apparatus according to Claim 9,
wherein the first member includes a tubular portion that has a length in the length direction of the linear member and forms the space,
wherein the second member includes an insertion portion to be inserted into the space in the tubular portion,
wherein the first protruding portion and the second protruding portion are disposed on an inner surface of the tubular portion, and
wherein the third protruding portion and the fourth protruding portion are disposed on an outer surface of the insertion portion.

11. The medical apparatus according to Claim 1 or 2,
wherein a magnet, a first magnetic member, and a second magnetic member are disposed on the second member, an engagement member engaging with the first magnetic member and the second magnetic member is disposed on the first member, the first magnetic member and the second magnetic member are arranged in such a manner as to hold the magnet therebetween in a length direction of the linear member, and the first member and the second member are connected to each other with the first magnetic member and the second magnetic member engaging with the engagement member by the first magnetic member and the second magnetic member being in close contact with the magnet,
wherein, in a case where pulling force at a first threshold value or less acts between the first member and the second member, a state in which the first magnetic member in close contact with the magnet is engaged with the engagement member is maintained, and the drive force is transmitted from the drive source to the linear member,
wherein, in a case where pulling force exceeding the first threshold value acts between the first member and the second member, the first magnetic member moves away from the magnet, and transmission of the drive force from the drive source to the linear member is cut off,
wherein, in a case where compression force at a second threshold value or less acts between the first member and the second member, a state in which the second magnetic member in close contact with the magnet is engaged with the engagement member is maintained, and the drive force is transmitted from the drive source to the linear member, and
wherein, in a case where compression force exceeding the second threshold value acts between the first member and the second member, the second magnetic member moves away from the magnet, and transmission of the drive force from the drive source to the linear member is cut off.

12. The medical apparatus according to Claim 11,
wherein the engagement member has a space into which the magnet is insertable, and the engagement member includes a protruding portion partially protruding inward at a position corresponding to the space, and
wherein, in a state in which the first member and the second member are connected, the first magnetic member is in close contact with the magnet and is in close contact with an end surface of the protruding portion on a proximal end side in a length direction of the linear member, and the second magnetic member is in close contact with the magnet and is in close contact with an end surface of the protruding portion on a distal end side in the length direction of the linear member.

13. The medical apparatus according to Claim 11 or 12,
wherein the second member includes a shaft portion that pivotally supports the magnet, the first magnetic member, and the second magnetic member, the shaft portion is fixed to the linear member, the magnet is fixed to the shaft portion, and the first magnetic member and the second magnetic member are slidable with respect to the shaft portion.

14. The medical apparatus according to any one of Claims 1 to 13, further comprising:
a plurality of drive sources; and
a plurality of linear members configured to bend the bending portion by being respectively driven by the plurality of drive sources,
wherein the connection unit is disposed for each set of a drive source and a linear member that includes one drive source of the plurality of drive sources, and one linear member of the plurality of linear members that is to be driven by the corresponding drive source.
